(19)

Europäisches
Patentamt
European
Patent Office
Office européen
des brevets

(11) **EP 4 545 141 A2**

(12) **EUROPEAN PATENT APPLICATION**

(43) Date of publication:
**30.04.2025  Bulletin 2025/18**

(21) Application number: **25150459.3**

(22) Date of filing: **19.12.2018**

(51) International Patent Classification (IPC):
***A61P 25/28*** *(2006.01)*

(52) Cooperative Patent Classification (CPC):
**A61K 38/465; A61K 9/0019; A61K 47/26;**
**C12N 9/16; C12Y 301/06001**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **19.12.2017  US 201762607831 P**
**20.12.2017  US 201762608569 P**
**02.02.2018  US 201862625814 P**
**13.11.2018  US 201862760597 P**

(62) Document number(s) of the earlier application(s) in
accordance with Art. 76 EPC:
**18834191.1 / 3 728 572**

(71) Applicant: **Takeda Pharmaceutical Company**
**Limited**
**Osaka-shi, Osaka (JP)**

(72) Inventors:
• **KEEFE, Andrew**
**Lexington, 02421 (US)**
• **ENRIQUEZ, George**
**Lexington, 02421 (US)**

(74) Representative: **Carpmaels & Ransford LLP**
**One Southampton Row**
**London WC1B 5HA (GB)**

Remarks:
•This application was filed on 07-01-2025 as a
divisional application to the application mentioned
under INID code 62.
•Claims filed after the date of filing of the divisional
application (Rule 68(4) EPC).

(54) **PURIFIED ARYLSULFATASE A AND COMPOSITIONS THEREOF**

(57)     The present invention provides, among other
things, methods of treatment of Metachromatic Leuko-
dystrophy Disease (MLD) and compositions comprising
recombinant arylsulfatase A (ASA) protein using enzyme
replacement therapy.

EP 4 545 141 A2

**Description**

**CROSS REFERENCE TO RELATED APPLICATIONS**

This application claims priority to United States Provisional Patent Application serial numbers 62/607,831 filed December 19, 2017; 62/608,569 filed December 20, 2017; 62/625,814 filed February 2, 2018; and 62/760,597 filed November 13, 2018; the entirety of each of which is hereby incorporated by reference.

**BACKGROUND**

Metachromatic Leukodystrophy Disease (MLD) is an autosomal recessive disorder resulting from a deficiency of the enzyme Arylsulfatase A (ASA). ASA, which is encoded by the ARSA gene in humans, is an enzyme that breaks down cerebroside 3-sulfate or sphingolipid 3-O-sulfogalactosylceramide (sulfatide) into cerebroside and sulfate. In the absence of the enzyme, sulfatides accumulate in the nervous system (e.g., myelin sheaths, neurons and glial cells) and to a lesser extent in visceral organs. The consequence of these molecular and cellular events is progressive demyelination and axonal loss within the CNS and PNS, which is accompanied clinically by severe motor and cognitive dysfunction.
A defining clinical feature of this disorder is central nervous system (CNS) degeneration, which results in cognitive impairment (e.g., mental retardation, nervous disorders, and blindness, among others).
MLD can manifest itself in young children (Late-infantile form), where affected children typically begin showing symptoms just after the first year of life (e.g., at about 15-24 months), and generally do not survive past the age of 5 years. MLD can manifest itself in children (Juvenile form), where affected children typically show cognitive impairment by about the age of 3-10 years, and life-span can vary (e.g., in the range of 10-15 years after onset of symptoms). MLD can manifest itself in adults (Adult-onset form) and can appear in individuals of any age (e.g., typically at age 16 and later) and the progression of the disease can vary greatly.
Enzyme replacement therapy (ERT) is an approved therapy for treating MLD, which involves administering exogenous replacement ASA enzyme, particularly recombinant Arylsulfatase A (rhASA) (e.g., recombinant human Arylsulfatase A (rhASA)) to patients with MLD.

**SUMMARY OF THE INVENTION**

The present invention provides, among other things, compositions comprising purified recombinant ASA protein for enzyme replacement therapy and methods of preparation thereof.
As described in the Examples section, exemplary recombinant ASA proteins purified using certain processes described herein conform to the marketing purity requirements in the US and many other countries.
In addition, a recombinant ASA protein purified according to the present invention features distinct characteristics such as a glycan map (e.g., threshold amounts of mannose-6-phosphated recombinant ASA protein (M6P ASA protein)) that can facilitate bioavailability, improved targeting, and/or improved efficacy of the recombinant ASA protein.
In one aspect, the invention provides a method of treating metachromatic leukodystrophy (MLD), the method comprising administering intrathecally to a subject in need thereof a therapeutically effective dose of a formulation comprising purified recombinant human arylsulfatase A (rhASA) protein, wherein the purified rhASA protein is characterized by a proteoglycan map comprising one or more peaks (i.e., species) corresponding to neutral recombinant ASA protein (neutral ASA protein, sialylated recombinant ASA protein (sialic acid ASA protein), mannose-6-phosphated recombinant ASA protein (M6P ASA protein), N-acetyl-glucosamine mannose-6-phosphated recombinant ASA protein (capped M6P ASA protein), and hybrid recombinant ASA protein (hybrid ASA protein), and wherein administering the formulation results in stabilizing or decreasing the progression of at least one symptom of MLD.
In one embodiment, the therapeutically effective dose of purified rhASA is 100 mg.
In one embodiment, the therapeutically effective dose of purified rhASA is 150 mg.
In one embodiment, the formulation is administered once weekly.
In one embodiment, the subject has late infantile MLD.
In one embodiment stabilizing or decreasing the progression of MLD is measured using a change in Gross Motor Function Classification (GMFC-MLD).
In one embodiment, administering the formulation results in a change in GMFC-MLD level by $\leq 4$, $\leq 3$ or $\leq 2$ levels from baseline at 2 years of treatment in the subject having late infantile MLD.
In one embodiment, administering the formulation results in maintenance of GMFC-MLD score at two years of treatment.
In one embodiment, maintenance of GMFC-MLD score is a change in GMFC-MLD of no greater than 2 levels from baseline at two years of treatment.
In one embodiment, the baseline is an assessment score of GMFC-MLD prior to the first administration of the formulation.

In one embodiment, stabilizing or decreasing the progression of MLD is measured using a change in Gross Motor Function Measure (GMFM-MLD).

In one embodiment, administering the formulation results in maintenance of GMFM-MLD score at two years of treatment.

In one embodiment, the GMFM-MLD score is maintained at > 40.

In one embodiment, the stabilizing or decreasing the progression of MLD is measured by the sulfatide levels in the cerebrospinal fluid.

In one embodiment, the stabilizing or decreasing the progression of MLD is measured by the brain N-acetylaspartate/creatine ratio (NAA/cr).

In one embodiment, the stabilizing or decreasing the progression of MLD is measured by the Expressive Language Function Classification-MLD levels (ELFC-MLD).

In one embodiment, the subject has baseline GMFC-MLD level 1-2.

In one embodiment, the subject has baseline GMFC-MLD level 3.

In one embodiment, the subject has baseline GMFC-MLD level 4.

In one embodiment, the subject is pre-symptomatic. In one embodiment, the pre-symptomatic subject is less than 18 months old. In one embodiment, the pre-symptomatic subject is assessed with Alberta Infant Motor Scale.

In some embodiments, the rhASA protein has an amino acid sequence at least 70% identical to SEQ ID NO: 1.

In one aspect, the invention provides a method of treating late infantile MLD, the method comprising: administering to the subject a formulation comprising a purified recombinant arylsulfatase A (rhASA) protein having an amino acid sequence at least 70% identical to SEQ ID NO:1, where the purified rhASA protein is characterized by a proteoglycan map comprising one or more peaks (i.e., species) corresponding to neutral recombinant ASA protein (neutral ASA protein, sialylated recombinant ASA protein (sialic acid ASA protein), mannose-6-phosphated recombinant ASA protein (M6P ASA protein), N-acetyl-glucosamine mannose-6-phosphated recombinant ASA protein (capped M6P ASA protein), and hybrid recombinant ASA protein (hybrid ASA protein), and the formulation is administered intrathecally at a dose of 150 mg at an interval of once every week.

In one embodiment, the formulation contains less than about 150 ng/mg Host Cell Protein (HCP).

In some embodiments, the purified recombinant ASA protein comprises at least about 23% of the total purified recombinant ASA protein corresponds to mannose-6-phosphated recombinant ASA protein (M6P ASA protein).

In one embodiment, the proteoglycan map of the purified recombinant ASA protein comprises: about 15% to about 25% neutral recombinant ASA protein (neutral ASA protein), about 35% to about 45% sialylated recombinant ASA protein (sialic acid ASA protein), about 23% to about 33% mannose-6-phosphated recombinant ASA protein (M6P ASA protein), about 1% to about 10% N-acetyl-glucosamine mannose-6-phosphated recombinant ASA protein (capped M6P ASA protein), and about 5% to about 15% hybrid recombinant ASA protein (hybrid ASA protein).

In one embodiment, the proteoglycan map of the purified recombinant ASA protein comprises: about 18% to about 22% neutral ASA protein, about 37% to about 41% sialic acid ASA protein, about 26% to about 29% M6P ASA protein, about 4% to about 6% capped M6P ASA protein, and about 7% to about 9% hybrid ASA protein.

In one embodiment, administration of the formulation results in minimal adverse effects (AE).

In one aspect, the present invention provides new processes for purifying recombinant ASA protein that can provide advantages such as cost and time reductions by improvements in process efficiencies (e.g., reducing total numbers of steps for the preparation of a drug substance (DS) or a drug product (DP)) or by permitting the preparation of compositions having beneficial features (e.g., a composition comprising purified recombinant ASA protein having a threshold population of mannose-6-phosphated recombinant ASA protein (M6P ASA protein) that can have improved bioavailability, targeting, or efficacy) or DS compositions comprising a surfactant (e.g., polysorbate-20 (P20)) that can have improved stability to storage (e.g., cold storage) or result in improved process efficiency for preparation of the DS or the DP.

In one aspect, the invention provides a composition comprising purified recombinant arylsulfatase A (rhASA) protein having an amino acid sequence at least 70% identical to SEQ ID NO: 1, wherein the purified rhASA protein is characterized by one or more proteoglycan species selected from neutral recombinant ASA protein (neutral ASA protein, sialylated recombinant ASA protein (sialic acid ASA protein), mannose-6-phosphated recombinant ASA protein (M6P ASA protein), N-acetyl-glucosamine mannose-6-phosphated recombinant ASA protein (capped M6P ASA protein), or hybrid recombinant ASA protein (hybrid ASA protein). In some embodiments, the mannose-6-phosphated recombinant ASA protein (M6P ASA protein) is present in an amount that is at least about 23% of the total purified rhASA protein content.

In one embodiment, the invention provides a composition comprising purified recombinant arylsulfatase A (rhASA) protein having an amino acid sequence at least 70% identical to SEQ ID NO: 1, wherein the purified rhASA protein is present as mannose-6-phosphated recombinant ASA protein (M6P ASA protein) in an amount that is at least about 23% of the total purified rhASA protein content; and the purified recombinant ASA protein contains less than about 150 ng/mg Host Cell Protein (HCP).

In one aspect, the present invention provides a composition comprising purified recombinant human arylsulfatase A (rhASA) protein having an amino acid sequence at least 70% identical to SEQ ID NO:1, wherein at least about 23% of the total purified recombinant ASA protein corresponds to mannose-6-phosphated recombinant ASA protein (M6P ASA protein) characterized by a proteoglycan map; and the composition contains less than about 150 ng/mg Host Cell Protein (HCP).

In one embodient, the M6P ASA protein is present in the composition in an amount that is at least about 26% of the total purified rhASA protein content. In one embodiment, the M6P ASA protein is present in an amount that is at least about 28% of the total purified rhASA protein content. In one embodiment, the M6P ASA protein is present in the composition in an amount that is about 20% to about 33% of the total purified rhASA protein content.

In one embodiment, the total purified rhASA protein comprises neutral recombinant ASA protein (neutral ASA protein), sialylated recombinant ASA protein (sialic acid ASA protein), N-acetyl-glucosamine mannose-6-phosphated recombinant ASA protein (capped M6P ASA protein), or hybrid recombinant ASA protein (hybrid ASA protein), or any combination thereof.

In one embodiment, the total purified rhASA protein comprises: about 23% to about 33% mannose-6-phosphated recombinant ASA protein (M6P ASA protein), about 15% to about 25% neutral recombinant ASA protein (neutral ASA protein), about 35% to about 45% sialylated recombinant ASA protein (sialic acid ASA protein), about 1% to about 10% N-acetyl-glucosamine mannose-6-phosphated recombinant ASA protein (capped M6P ASA protein), and about 5% to about 15% hybrid recombinant ASA protein (hybrid ASA protein).

In one embodiment, the neutral ASA protein is present in an amount that is about 16% to about 22% of the total purified rhASA protein.

In one embodiment, the neutral ASA protein is present in an amount that is about 20% to about 25% of the total purified rhASA protein.

In one embodiment, the sialic acid ASA protein is present in an amount that is about 35% to about 40% of the total purified rhASA protein.

In one embodiment, the sialic acid ASA protein is present in an amount that is about 37% to about 42% of the total purified rhASA protein.

In one embodiment, the capped M6P ASA protein is present in an amount that is about 3% to about 5% of the total purified rhASA protein.

In one embodiment, the capped M6P ASA protein is present in an amount that is about 4% to about 6% of the total purified rhASA protein.

In one embodiment, the capped M6P ASA protein is present in an amount that is about 4.5% to about 5.5% of the total purified rhASA protein.

In one embodiment, the hybrid ASA protein is present in an amount that is about 7% to about 10% of the total purified rhASA protein.

In one embodiment, the hybrid ASA protein is present in an amount that is about 7.5% to about 8.5% of the total purified rhASA protein.

In one embodiment, the total purified rhASA protein comprises: about 16% to about 23% neutral ASA protein, about 37% to about 42% sialic acid ASA protein, about 23% to about 27% M6P ASA protein, about 4% to about 8% capped M6P ASA protein, and about 7% to about 10% hybrid ASA protein. In one embodiment, the total purified rhASA protein comprises: about 20% to about 23% neutral ASA protein, about 35% to about 39% sialic acid ASA protein, about 26% to about 32% M6P ASA protein, about 3% to about 5% capped M6P ASA protein, and about 7% to about 9% hybrid ASA protein.

In one embodiment, the total purified rhASA protein comprises: about 18% to about 22% neutral ASA protein, about 37% to about 41% sialic acid ASA protein, about 26% to about 29% M6P ASA protein, about 4% to about 6% capped M6P ASA protein, and about 7% to about 9% hybrid ASA protein.

In one embodiment, the total purified rhASA protein is present in a concentration of about 20 mg/mL to about 45 mg/mL. In one embodiment, the total purified rhASA protein is present in a concentration of about 25 mg/mL to about 34 mg/mL or about 28 mg/mL to about 32 mg/mL. In one embodiment, the total purified rhASA protein is present in a concentration of about 25 mg/mL, about 26 mg/mL, about 27 mg/mL, about 28 mg/mL, about 29 mg/mL, about 30 mg/mL, about 31 mg/mL, about 32 mg/mL, about 33 mg/mL, or about 34 mg/mL.

In one embodiment, the purified rhASA protein has a specific activity of about 50 to about 130 U/mL. In one embodiment, the purified rhASA protein has a specific activity of about 70 to about 100 U/mg. In one embodiment, the purified rhASA protein has a specific activity of about 80 to about 90 U/mg. In one embodiment, the purified rhASA protein has a specific activity of about 75 to about 95 U/mg.

In one embodiment, the purified rhASA protein contains less than about 140 ng/mg Host Cell Protein (HCP). In one embodiment, the purified rhASA protein contains less than about 100 ng/mg HCP. In one embodiment, the purified rhASA protein contains less than about 80 ng/mg HCP. In one embodiment, the purified rhASA protein contains less than about 60 ng/mg HCP. In one embodiment, the purified rhASA protein contains less than about 100 pg/mg Host Cell DNA (HCD). In one embodiment, the purified rhASA protein contains less than about 50 pg/mg HCD. In one embodiment, the purified rhASA protein contains less than about 10 pg/mg HCD. In one embodiment, the purified rhASA protein contains less than about 5 pg/mg HCD, less than about 4 pg/mg HCD, less than about 3 pg/mg HCD, less than about 2 pg/mg HCD, or less than about 1 pg/mg HCD.

In one embodiment, the purified rhASA protein has an amino acid sequence at least 80% identical to SEQ ID NO:1. In one embodiment, the purified rhASA protein has an amino acid sequence at least 90% identical to SEQ ID NO:1. In one embodiment, the purified rhASA protein has an amino acid sequence at least 95% identical to SEQ ID NO:1.

In one embodiment, the purified rhASA protein has an amino acid sequence that is identical to SEQ ID NO:1.

In one embodiment, the composition comprises about 0.001% to about 0.01% polysorbate-20 (P20). In one aspect, the invention provides a formulation comprising the composition as detailed above and a physiologically acceptable carrier. In one embodiment, the formulation is suitable for intravenous administration.

In one embodiment, the formulation is suitable for intrathecal administration. In one embodiment, the formulation is suitable for subcutaneous administration.

In one aspect, the invention provides a method of purifying recombinant arylsulfatase A (ASA) protein, the method comprising: purifying recombinant arylsulfatase A (ASA) protein from an impure preparation by conducting one or more chromatography steps; pooling eluate from the one or more chromatography steps; optionally adjusting the pH of the pooled eluate to pH that is about 6.0 to about 8.0; optionally subjecting the pooled eluate or the pH-adjusted eluate to ultrafiltration and/or diafiltration; obtaining an eluate comprising purified recombinant arylsulfatase A (ASA) protein; and adding of a surfactant to the eluate comprising purified recombinant arylsulfatase A (ASA) protein.

In one embodiment, the method further comprises the step of adjusting the pH of the pooled eluate to pH that is about 6.0 to about 8.0. In one embodiment, the step of subjecting the pH-adjusted eluate to ultrafiltration and/or diafiltration.

In one embodiment, said adding of a surfactant occurs prior to cold storage of the eluate comprising purified recombinant ASA protein.

In one embodiment, said surfactant is present in a concentration that is about 0.0001 % (v/v) to about 0.01 % (v/v).In one embodiment, said surfactant is present in a concentration that is about 0.001% (v/v) to about 0.01% (v/v). In one embodiment, said surfactant is polysorbate-20 (P20).

In one embodiment, the conducting one or more chromatography steps comprises conducting anion-exchange chromatography, mixed-mode chromatography, hydrophobic interaction chromatography that is phenyl chromatography, and cation-exchange chromatography, in that order. In some embodiments, the anion-exchange chromatography uses a column with TMAE resin.

## BRIEF DESCRIPTION OF THE DRAWING

The Figures described below, that together make up the Drawing are for illustration purposes only, not for limitation.

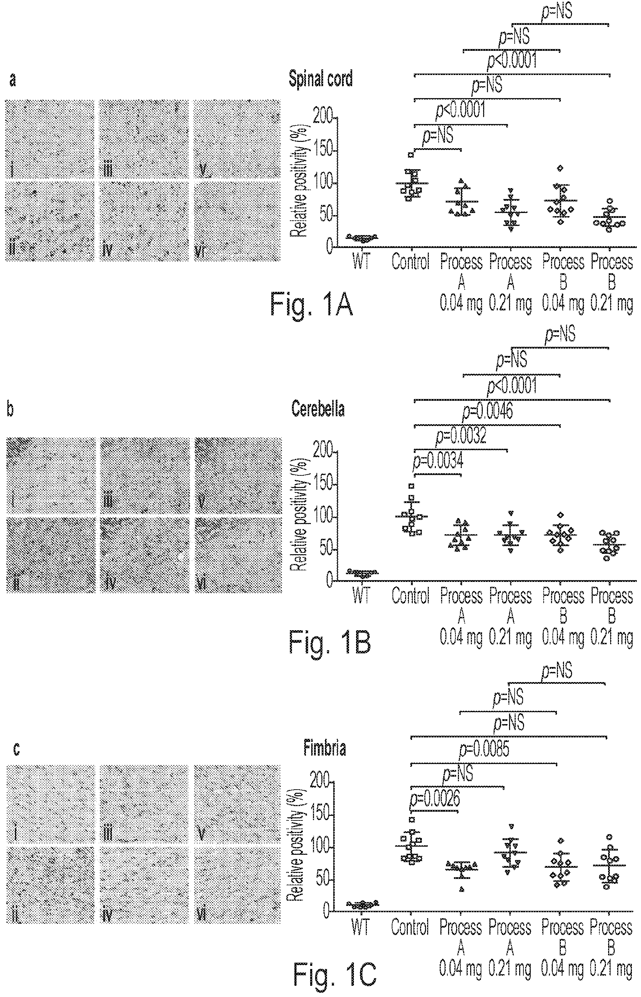

Figure 1A-1F show representative images of immunohistochemical staining of LAMP-1 in MLD mice treated with process A or process B rhASA with corresponding morphometric analysis in the white matter of (A) spinal cord, (B) cerebella, (C) fimbria, (D) cerebral peduncle, (E) cerebral cortex and (F) striatum of immunotolerant MLD mice treated with rhASA 0.04 mg or 0.21 mg from process A (iii, v) or process B (iv, vi), or control (ii). Untreated C57/B16 mice served as WT controls (i). LAMP-1, lysosomal-associated membrane protein-1; MLD, metachromatic leukodystrophy; NS, not significant; rhASA, recombinant human arylsulfatase A; WT, wild type.

Figure 2A and 2B show concentration-time curves of process A and process B rhASA in (Fig. 2A) serum and (Fig. 2B) CSF after a single 6.0 mg intrathecal dose in juvenile cynomolgus monkeys.

Figure 3A and 3B show biodistribution of process A and process B rhASA in rats. Representative whole body autoradioluminograms showing tissue distribution of radioactivity 4 hours after a single intrathecal dose of (Fig. 3A) process A and (Fig. 3B) process B [$^{125}$I]-rhASA 0.62 mg in male Sprague Dawley rats.

Figure 4 shows urinary, fecal and total excretion profiles of process A and process B [$^{125}$I]-rhASA 0.62 mg after a single intrathecal dose in male Sprague Dawley rats (n = 2 and n = 3, respectively).

Figure 5 shows the dose-escalation and extension study design of an open label, dose-escalation safety evaluation of rhASA. CSF, cerebrospinal fluid; GMFM-88, Gross Motor Function Measure-88; IDDD, intrathecal drug delivery device; IT, intrathecal; MLD, metachromatic leukodystrophy; rhASA, recombinant human arylsulfatase A.

Figure 6 shows a clinical PK/PD model for rhASA concentrations in CSF, CNS, and serum and the effect on CSF sulfatide concentrations. CL, drug clearance; CMT, compartment; CNS, central nervous system; CSF, cerebrospinal fluid; $k_{in}$, formation rate of sulfatides; $k_{out}$, depletion rate constant of sulfatides; $K_{trans}$, transit rate constant; PK/PD, pharmacokinetics/pharmacodynamics; $Q_{CSF}$, intercompartmental clearance; rhASA, recombinant human arylsulfatase A.

Figure 7A, 7B and 7C, show goodness-of-fit plots of population- and individual-predicted rhASA concentrations in CSF, sulfatide concentration in CSF and GMFM-88 total scores, respectively.

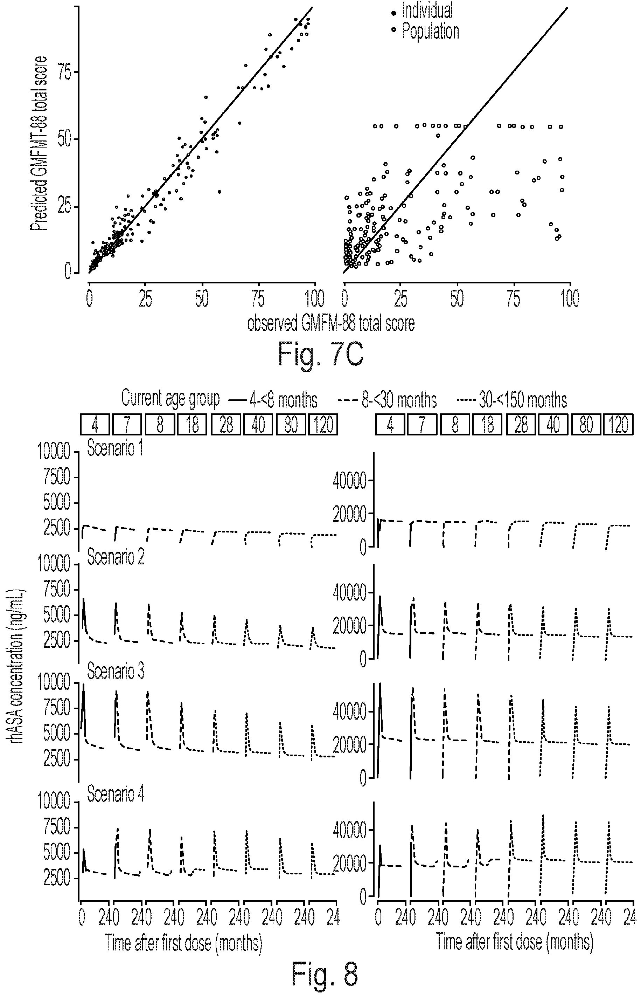

Figure 8 shows representative simulated rhASA trough concentrations in the cerebrospinal fluid (CSF) (left panel) and central nervous system (CNS) (right panel) by dose regimen and baseline age. Scenario 1: 100 mg every other week; Scenario 2: 100 mg every week for 12 weeks (initial weekly dosing), then 100 mg every other week; Scenario 3: 150 mg every week for 12 weeks (initial weekly dosing), then 150 mg every other week; Scenario 4: age-adjusted dosing weekly for 12 weeks (initial weekly dosing), then every other week (scenario 4: 80 mg, < 8 months; 120 mg, 8-< 30 months; 150 mg, ≥ 30 months).

Figure 9 shows simulated individual-level predicted GMFM-88 total score by dosing scenario. The four dosing scenarios included: scenario 1, 100 mg every other week (EOW); scenario 2, 100 mg every week (EW) for 12 weeks, then 100 mg EOW; scenario 3, 150 mg every week for 12 weeks, then 150 mg EOW; scenario 4, age-adjusted dosing weekly for 12 weeks, then EOW (80 mg, < 8 months; 120 mg, 8-< 30 months; 150 mg, ≥ 30 months).

Figure 10 shows the dose-escalation and extension study design of an open label, dose-escalation safety evaluation of rhASA. The decision to proceed with escalation to a higher dose was based on a DSMB review after all patients in the cohort had received at least two doses of the previous dose. DSMB, Data Safety Monitoring Board; EOS, end of study; EOW, every other week; GMFM-88, Gross Motor Function Measure-88; IDDD, intrathecal drug delivery device; MLD, metachromatic leukodystrophy; MRI, magnetic resonance imaging; rhASA, recombinant human arylsulfatase A.

Figure 11 shows individual GMFM-88 total score by patient age over study period for patients identified as responders and non-responders to treatment.

Figure 12 shows individual MLD severity scores at baseline and week 40 for patients with data available who responded to treatment

Figure 13 shows individual N-acetylaspartate (NAA)/creatine ratios in frontal parietal white matter at baseline and week 40 for patients with data available who responded to treatment.

Figure 14 shows mean (± SD) concentration of rhASA in cerebrospinal fluid over time.

Figure 15 shows observed mean GMFM-88 total scores over time of Cohort 1 (process A: 10 mg), Cohort 2 (process A: 30 mg), Cohort 3 (process A: 100 mg) Cohort 4 (process B: 100 mg). Error bars represent standard error of the mean.

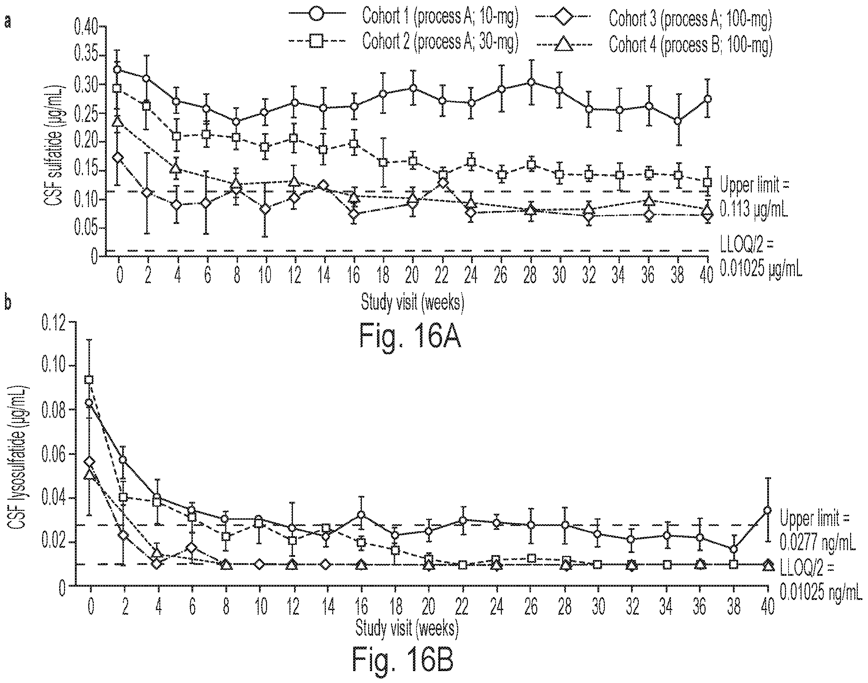

Figure 16A and 16B show mean CSF sulfatide and CSF lysosulfatide levels respectively, for Cohort 1 (process A: 10 mg), Cohort 2 (process A: 30 mg), Cohort 3 (process A: 100 mg) Cohort 4 (process B: 100 mg). Error bars represent standard error of the mean. The upper limit of normal for CSF sulfatide (0.113 $\mu$g/mL) and for CSF lysosulfatide (0.0277 ng/mL) is the highest concentration observed from CSF samples from 60 children who did not have leukodystrophy but who had undergone a spinal tap (and therefore may have other neurological conditions). CSF, cerebrospinal fluid; LLOQ/2, lower limit of quantification.

Figure 17A and 17B show mean change from baseline over time in (Figure 17A) NAA/creatine ratio and (Figure 17B) choline/creatine ratio in right frontal white matter. Baseline data were available for 16 patients and end of study data were available for 12 patients. Error bars represent standard deviation from the mean.

Figure 18A and 18B show mean change from baseline over time in (Figure 18A) NAA/creatine ratio and (Figure 18B) choline/creatine ratio in right frontal-parietal white matter. Error bars represent standard deviation from the mean.

Figure 19A and 19B show mean change from baseline over time in (Figure 19A) NAA/creatine ratio and (Figure 19B) choline/creatine ratio in right parieto-occipital white matter. Error bars represent standard deviation from the mean.

Figure 20A and 20B show mean change from baseline over time in (Figure 20A) NAA/creatine ratio and (Figure 20B) choline/creatine ratio in midline occipital grey matter. Error bars represent standard deviation from the mean.

Figure 21 shows mean total MLD severity score over time. Error bars represent standard error of the mean.

Figure 22 shows individual GMFM-88 total score by age in months of sibling pair patients from a clinical study of rhASA.

## DEFINITIONS

In order for the present invention to be more readily understood, certain terms are first defined below. Additional definitions for the following terms and other terms are set forth throughout the specification.

Approximately or about: As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

Biologically active: As used herein, the phrase "biologically active" refers to a characteristic of any agent that has activity in a biological system, and particularly in an organism. For instance, an agent that, when administered to an organism, has a biological effect on that organism, is considered to be biologically active. In particular embodiments, where a protein or polypeptide is biologically active, a portion of that protein or polypeptide that shares at least one biological activity of the protein or polypeptide is typically referred to as a "biologically active" portion.

Cation-independent mannose-6-phosphate receptor (CI-MPR): As used herein, the term "cation-independent mannose-6-phosphate receptor (CI-MPR)" refers to a cellular receptor that binds mannose-6-phosphate (M6P) tags on acid hydrolase precursors in the Golgi apparatus that are destined for transport to the lysosome. In addition to mannose-6-phosphates, the CI-MPR also binds other proteins including IGF-II. The CI-MPR is also known as "M6P/IGF-II receptor," "CI-MPR/IGF-II receptor," "IGF-II receptor" or "IGF2 Receptor." These terms and abbreviations thereof are used interchangeably herein.

Chromatography: As used herein, the term "chromatography" refers to a technique for separation of mixtures. Typically, the mixture is dissolved in a fluid called the "mobile phase," which carries it through a structure holding another material called the "stationary phase." Column chromatography is a separation technique in which the stationary bed is within a tube, i.e., column.

Diluent: As used herein, the term "diluent" refers to a pharmaceutically acceptable (e.g., safe and non-toxic for administration to a human) diluting substance useful for the preparation of a reconstituted formulation. Exemplary diluents include sterile water, bacteriostatic water for injection (BWFI), a pH buffered solution (e.g. phosphate-buffered saline), sterile saline solution, Ringer's solution or dextrose solution.

Elution: As used herein, the term "elution" refers to the process of extracting one material from another by washing with a solvent. For example, in ion-exchange chromatography, elution is a process to wash loaded resins to remove captured ions.

Eluate: As used herein, the term "eluate" refers to a combination of mobile phase "carrier" and the analyte material that emerge from the chromatography, typically as a result of eluting.

Enzyme replacement therapy (ERT): As used herein, the term "enzyme replacement therapy (ERT)" refers to any therapeutic strategy that corrects an enzyme deficiency by providing the missing enzyme. Once administered, enzyme is taken up by cells and transported to the lysosome, where the enzyme acts to eliminate material that has accumulated in the lysosomes due to the enzyme deficiency. Typically, for lysosomal enzyme replacement therapy to be effective, the therapeutic enzyme is delivered to lysosomes in the appropriate cells in target tissues where the storage defect is manifest. The purification processes described herein may be used to purify and formulate recombinant Arylsulfatase A as a drug substance for ERT of MLD.

Equilibrate or Equilibration: As used herein, the terms "equilibrate" or "equilibration" in relation to chromatography refer to the process of bringing a first liquid (e.g., buffer) into balance with another, generally to achieve a stable and equal distribution of components of the liquid (e.g., buffer). For example, in some embodiments, a chromatographic column may be equilibrated by passing one or more column volumes of a desired liquid (e.g., buffer) through the column.

Improve, increase, or reduce: As used herein, the terms "improve," "increase" or "reduce," or grammatical equivalents, indicate values that are relative to a baseline measurement, such as a measurement in the same individual prior to initiation of the treatment described herein, or a measurement in a control individual (or multiple control individuals) in the absence of the treatment described herein. A "control individual" is an individual afflicted with the same form of lysosomal storage disease as the individual being treated, who is about the same age as the individual being treated (to ensure that the stages of the disease in the treated individual and the control individual(s) are comparable).

Impurities: As used herein, the term "impurities" refers to substances inside a confined amount of liquid, gas, or solid, which differ from the chemical composition of the target material or compound. Impurities are also referred to as contaminants.

Load: As used herein, the term "load" refers to, in chromatography, adding a sample-containing liquid or solid to a column. In some embodiments, particular components of the sample loaded onto the column are then captured as the loaded sample passes through the column. In some embodiments, particular components of the sample loaded onto the column are not captured by, or "flow through", the column as the loaded sample passes through the column.

Polypeptide: As used herein, a "polypeptide", generally speaking, is a string of at least two amino acids attached to one another by a peptide bond. In some embodiments, a polypeptide may include at least 3-5 amino acids, each of which is attached to others by way of at least one peptide bond. Those of ordinary skill in the art will appreciate that polypeptides sometimes include "non-natural" amino acids or other entities that nonetheless are capable of integrating into a polypeptide chain, optionally.

Pool: As used herein, the term "pool" in relation to chromatography refers to combining one or more fractions of fluid that has passed through a column together. For example, in some embodiments, one or more fractions which contain a desired component of a sample that has been separated by chromatography (e.g., "peak fractions") can be "pooled" together generate a single "pooled" fraction.

Replacement enzyme: As used herein, the term "replacement enzyme" refers to any enzyme that can act to replace at least in part the deficient or missing enzyme in a disease to be treated. In some embodiments, the term "replacement enzyme" refers to any enzyme that can act to replace at least in part the deficient or missing lysosomal enzyme in a lysosomal storage disease to be treated. In some embodiments, a replacement enzyme (e.g., rhASA) is capable of reducing accumulated materials in mammalian lysosomes or that can rescue or ameliorate one or more lysosomal storage disease (e.g., MLD) symptoms. Replacement enzymes suitable for the invention include both wild-type or modified lysosomal enzymes and can be produced using recombinant and synthetic methods or purified from nature sources. A replacement enzyme can be a recombinant, synthetic, gene-activated or natural enzyme.

*Soluble:* As used herein, the term "soluble" refers to the ability of a therapeutic agent to form a homogenous solution. In some embodiments, the solubility of the therapeutic agent in the solution into which it is administered and by which it is transported to the target site of action is sufficient to permit the delivery of a therapeutically effective amount of the therapeutic agent to the targeted site of action. Several factors can impact the solubility of the therapeutic agents. For example, relevant factors which may impact protein solubility include ionic strength, amino acid sequence and the presence of other co-solubilizing agents or salts (e.g., calcium salts). In some embodiments, therapeutic agents in accordance with the present invention are soluble in its corresponding pharmaceutical composition.

*Stability:* As used herein, the term "stable" refers to the ability of the therapeutic agent (e.g., a recombinant enzyme) to maintain its therapeutic efficacy (e.g., all or the majority of its intended biological activity and/or physiochemical integrity) over extended periods of time. The stability of a therapeutic agent, and the capability of the pharmaceutical composition to maintain stability of such therapeutic agent, may be assessed over extended periods of time (e.g., for at least 1, 3, 6, 12, 18, 24, 30, 36 months or more). In the context of a formulation a stable formulation is one in which the therapeutic agent therein essentially retains its physical and/or chemical integrity and biological activity upon storage and during processes (such as freeze/thaw, mechanical mixing and lyophilization). For protein stability, it can be measure by formation of high molecular weight (HMW) aggregates, loss of enzyme activity, generation of peptide fragments and shift of charge profiles.

*Viral Processing:* As used herein, the term "viral processing" refers to "viral removal," in which viruses are simply removed from the sample (e.g. viral filtration), or "viral inactivation," in which the viruses remain in a sample but in a non-infective form. In some embodiments, viral removal may utilize nanofiltration and/or chromatographic techniques, among others. In some embodiments, viral inactivation may utilize solvent inactivation, detergent inactivation, pasteurization, acidic pH inactivation, and/or ultraviolet inactivation, among others.

## DETAILED DESCRIPTION

The present invention provides, among other things, methods and compositions comprising purified recombinant ASA (rhASA) protein for enzyme replacement therapy. The invention is based at least in part on the finding that rhASA is safe and well tolerated by infants and children; so as to support a dose escalation for higher therapeutic efficacy. The instant invention is directed in part to an improved recombinant rhASA therapeutic dose and an administration interval for treating MLD for improving motor function, or at least decreasing or halting a progressive decline of motor function in subjects with ASA deficiency.

In one aspect, the invention relates to a method of treating MLD, comprising the steps of administering a therapeutically effective dose of rhASA at a suitable dose interval to decrease or halt the progression of MLD.

In some embodiments, the therapeutically effective dose is 100 mg or higher. In some embodiments the suitable dose interval is once every other week. In some embodiments, the suitable dose interval is once every week. In some embodiments the method comprises administration of a formulation comprising rhASA at a dose of 100 mg or higher once every other week (EOW). In some embodiments, the formulation comprising rhASA is administered to a subject at a dose is 100 mg or higher once every week (EW). In some embodiments, the formulation comprising rhASA is administered to a subject at a dose of 150 mg once EOW. In some embodiments, the formulation comprising rhASA is administered to a subject at a dose of 150 mg once EW.

In some embodiments, the subject had received a dose of at least 10 mg for a duration of time prior to being administered a dose of 100 mg. In some embodiments, the subject had received a dose of at least 30 mg for a duration of time prior to being administered a dose of 100 mg. In some embodiments, the subject had received a dose of 100 mg for a duration of time and then continued to receive 100 mg rhASA. In some embodiments, the duration of time is 20 weeks, 30 weeks, 35 weeks or 40 weeks. In some embodiments, the duration of time is 40 weeks.

In some embodiments, subjects receiving the at least 100 mg dose were responders to the prior treatment with 10 mg. In some embodiments, subjects receiving the at least 100 mg dose were responders to the prior treatment with 30 mg. In some embodiments, subjects receiving the at least 100 mg dose were non-responders to the prior treatment with 10 mg. In some embodiments, subjects receiving the at least 100 mg dose were non-responders to the prior treatment with 30 mg. In some embodiments, subjects receiving the at least 100 mg dose did not exhibit any adverse effect (AE) to the prior treatment with 10 mg. In some embodiments, a subject receiving the at least 100 mg dose did not exhibit any AE to the prior treatment with 30 mg. In some embodiments, a subject receiving the at least 100 mg dose did not exhibit any AE to the prior treatment with 100 mg.

In some embodiments, the formulation comprising rhASA is administered at a dose of 100 mg or higher once EW for 12 weeks, followed by administration EOW. In some embodiments, the formulation comprising rhASA is administered at a dose of 150 mg once EW for 12 weeks, followed by administration EOW. In some embodiments, subject exhibits no adverse effect with the administration.

The instant invention is based in part on the finding that the therapeutic application of rhASA is effective in treating a wider range of MLDs, especially, a late infantile MLD. In one aspect, the invention provides a method of treating late infantile MLD. In some embodiments, the method comprises administering a formulation comprising rhASA to a subject having late infantile MLD. In some embodiments, the method comprises administering an effective therapeutic dose of the formulation comprising rhASA at a suitable dosage interval to a subject having late infantile MLD to decrease or halt the progression of late infantile MLD. In some embodiments, the method comprises intrathecal administration. In some embodiments, the effective therapeutic dose for treating late infantile MLD is 150 mg. In some embodiments a suitable dosage interval for treating late infantile MLD is once every week.

In some embodiments, the subject having late infantile MLD is 72 months old or younger. In some embodiments, the subject having late infantile MLD is 48 months old or younger. In some embodiments, the subject having late infantile MLD is 24 months old or younger. In some embodiments, the subject having late infantile MLD is 18 months old or younger. Subjects are selected and categorized on the basis of motor function, as determined by Gross Motor Function Classification (GMFC).

In some embodiments, the method comprises administration of a formulation comprising rhASA intrathecally to a subject having late infantile MLD at a dose of 150 mg once EW. In some embodiments, the administration was continued for at least two years.

Subject selection for treating late infantile MLD with a formulation comprising rhASA comprises several factors based on the age, disease state and motor function characterization. In some embodiments, the treatment is administered to an early symptomatic subject. In some embodiments, the subject with early symptomatic late infantile MLD is 18-48 months of age. In some embodiments, the subject with early symptomatic late infantile MLD has a Gross Motor Function Classification in MLD [GMFC-MLD] level 1-2.

In some embodiments, the treatment for late infantile MLD is administered to an intermediate symptomatic subject. In some embodiments, the subject with advanced symptomatic subject is 18-72 months of age. In some embodiments, the subject with advanced symptomatic subject has a GMFC MLD level 4.

In some embodiments, the treatment for late infantile MLD is administered to pre-symptomatic or minimally symptomatic subject. In some embodiments, the subject with pre-symptomatic or minimally symptomatic is 18 months of age or younger, or pre-symptomatic siblings of participants, with the same ASA allelic constitution. In one embodiment, the pre-symptomatic subject is assessed with Alberta Infant Motor Scale (Piper M and Darrah J, "Motor Assessment of a Developing Infant," Published February 9, 1994, pages 222; ISBN: 9780721643076, Saunders).

In some embodiments, the invention present invention provides purified rhASA composition for administration to subject having MLD.

## Treatment of Metachromatic Leukodystrophy Disease (MLD)

Compositions and methods of the present invention may be used to effectively treat individuals suffering from or susceptible to MLD by delivery to the CNS. Certain methods of the invention for treating MLD generally comprise a step of administering intrathecally to a subject in need of treatment a recombinant arylsulfatase A (ASA) enzyme at a therapeutically effective dose and an administration interval for a treatment period sufficient to stabilize or slow progression of motor dysfunction as measured by GMFC-MLD level (indicating motor function decline). According to many methods provided in the present disclosure, no serious adverse effects associated with administration of the recombinant arylsulfatase A are observed in the subject.

## Motor functions

In some embodiments, neurological impairment in an MLD patient is characterized by decline in motor function, e.g., gross motor function. In some embodiments, treatment efficacy comprises a measure that relates to one or more motor functions.

In some embodiments, provided are methods of treating metachromatic leukodystrophy (MLD) Syndrome comprising a step of administering intrathecally to a subject in need of treatment a recombinant arylsulfatase A (ASA) enzyme at a therapeutically effective dose and an administration interval for a treatment period sufficient to improve, stabilize or reduce decline of one or more motor functions relative to baseline. In some embodiments, administering of the recombinant ASA enzyme further results in improvement, stabilization or reduction decline of one or more cognitive, adaptive, and/or executive functions.

The one or more motor functions may comprise, for example, gross motor function. It will be appreciated that gross motor function may be assessed by any appropriate method. For example, in some embodiments, gross motor function is measured as a change from a baseline in motor function using a Gross Motor Function Measure, such as the Gross Motor Function Measure-88 (GMFM-88) total raw score or percentage or the or Gross Motor Function Classification (GMFC).

### Gross Motor Function Measure-88 (GMFM-88)

In some embodiments, neurological impairment in an MLD patient is characterized by decline in gross motor function. It will be appreciated that gross motor function may be assessed by any appropriate method. For example, in some embodiments, gross motor function is measured as the change from a baseline in motor function using the Gross Motor Function Measure-88 (GMFM-88) total raw score. In some embodiments, the subject being treated has a baseline GMFM-88 score of greater than 40%. In some embodiments, the subject being treated has a baseline GMFM-88 score of less than 40%.

In some embodiments, administering the recombinant ASA enzyme in accordance with methods disclosed herein results in a smaller decline in motor functions than would be typically observed without the administration. For example, in some embodiments, administering of the recombinant ASA enzyme in accordance with methods of the invention results in decline of the GMFM-88 score by less than 10%, 20%, 30%, 40%, or 50%.

In some embodiments, the subject being treated has a baseline GMFM-88 score of 35. In some embodiments, the subject being treated has a baseline GMFM-88 score of greater than 35. In some embodiments, the subject being treated has a baseline GMFM-88 score of 40. In some embodiments, the subject being treated has a baseline GMFM-88 score of greater than 40. In some embodiments, administering the recombinant ASA enzyme in accordance with methods disclosed herein results in maintenance of motor functions relative to baseline. In some embodiments, administering the recombinant ASA enzyme in accordance with methods disclosed herein results in a maintenance of motor functions measured by GMFM-88 score.

In some embodiments, administering the recombinant ASA enzyme in accordance with methods disclosed herein results in substantial stabilization of motor function, e.g., substantial stabilization of a score such as the GMFM-88 score. By "substantial stabilization" it is meant that there is a lack of decline or worsening over a period of time, e.g., over the treatment period and/or over a period during which decline or worsening would normally be expected in the absence of treatment.

In some embodiments, administering the recombinant ASA enzyme in accordance with methods disclosed herein results in improvement of motor function, e.g., improvement of a score such as the GMFM-88 score.

It is to be further understood that for any particular subject, specific dosage regimens should be adjusted over time according to the individual need and the professional judgment of the person administering or supervising the administration of the enzyme replacement therapy and that dosage ranges set forth herein are exemplary only and are not intended to limit the scope or practice of the claimed invention.

### Gross Motor Function Classification (GMFC-MLD)

The Gross Motor Function Classification (GMFC-MLD) can be used as a tool for standardized assessment of gross motor function in MLD. The GMFC-MLD consists of seven levels representing all clinically related stages from normal (level 0) to loss of all gross motor function (level 6). In some embodiments, gross motor function is measured using the GMFC-MLD.

In some embodiments, the subject being treated has a baseline GMFC level less than or equal to 2 (ability to stand and walk assisted). In some embodiments, administering the recombinant ASA enzyme in accordance with methods disclosed herein results in maintenance of motor functions relative to baseline as assessed by GMFC level. In some embodiments, administering the recombinant ASA enzyme in accordance with methods disclosed herein results in a maintenance of GMFC level equal to or less than 2.

In some embodiments, administering the recombinant ASA enzyme in accordance with methods of the invention results in an increase in their GMFC-MLD level (indicating motor function decline) of no more than 2 from baseline to 2 years. In some embodiments, administering the recombinant ASA enzyme in accordance with methods of the invention results in an increase in their GMFC-MLD level (indicating motor function decline) of no more than 3 from baseline to 2 years. In some embodiments, administering the recombinant ASA enzyme in accordance with methods of the invention results in maintenance of gross motor function, evaluated as a change from baseline of no greater than 2 levels of GMFC-MLD.

### Therapeutic Administration

Purified recombinant ASA protein may be administered to a MLD patient in accordance with known methods. For example, purified recombinant ASA protein may be delivered intravenously, subcutaneously, intramuscularly, parenterally, transdermally, or transmucosally (e.g., orally or nasally)).

In some embodiments, a recombinant ASA or a pharmaceutical composition containing the same is administered to a subject by intravenous administration.

In some embodiments, a recombinant ASA or a pharmaceutical composition containing the same is administered to a subject by intrathecal administration. As used herein, the term "intrathecal administration" or "intrathecal injection" refers to an injection into the spinal canal (intrathecal space surrounding the spinal cord). Various techniques may be used including, without limitation, lateral cerebroventricular injection through a burrhole or cisternal or lumbar puncture or the like. In some embodiments, "intrathecal administration" or "intrathecal delivery" according to the present invention refers to IT administration or delivery via the lumbar area or region, i.e., lumbar IT administration or delivery. As used herein, the term "lumbar region" or "lumbar area" refers to the area between the third and fourth lumbar (lower back) vertebrae and, more inclusively, the L2-S1 region of the spine. In some embodiments, a recombinant ASA or a pharmaceutical composition containing the same is administered to a subject by intrathecal administration as described in PCT international publications WO2011/163648 and WO2011/163650, incorporated herein by reference in their entirety.

In some embodiments, a recombinant ASA or a pharmaceutical composition containing the same is administered to the subject by subcutaneous (i.e., beneath the skin) administration. For such purposes, the formulation may be injected using a syringe. However, other devices for administration of the formulation are available such as injection devices (e.g., the Inject-ease and Genject devices); injector pens (such as the GenPen); needleless devices (e.g., MediJector and BioJector); and subcutaneous patch delivery systems.

In some embodiments, intrathecal administration may be used in conjunction with other routes of administration (e.g., intravenous, subcutaneously, intramuscularly, parenterally, transdermally, or transmucosally (e.g., orally or nasally)).

The present invention contemplates single as well as multiple administrations of a therapeutically effective amount of a recombinant ASA or a pharmaceutical composition containing the same described herein. A recombinant ASA or a pharmaceutical composition containing the same can be administered at regular intervals, depending on the nature, severity and extent of the subject's condition (e.g., a lysosomal storage disease). In some embodiments, a therapeutically effective amount of a recombinant ASA or a pharmaceutical composition containing the same may be administered periodically at regular intervals (e.g., once every year, once every six months, once every five months, once every three months, bimonthly (once every two months), monthly (once every month), biweekly (once every two weeks), weekly, daily or continuously).

## CNS Delivery

It is contemplated that various stable formulations described herein are generally suitable for CNS delivery of therapeutic agents. Stable formulations according to the present invention can be used for CNS delivery via various techniques and routes including, but not limited to, intraparenchymal, intracerebral, intravetricular cerebral (ICV), intrathecal (e.g., IT-Lumbar, IT-cisterna magna) administrations and any other techniques and routes for injection directly or indirectly to the CNS and/or CSF. The term "cisterna magna" refers to the space around and below the cerebellum via the opening between the skull and the top of the spine. Typically, injection via cisterna magna is also referred to as "cisterna magna delivery."

## Intrathecal Delivery

In some embodiments, a replacement enzyme is delivered to the CNS in a formulation described herein. In some embodiments, a replacement enzyme is delivered to the CNS by administering into the cerebrospinal fluid (CSF) of a subject in need of treatment. In some embodiments, intrathecal administration is used to deliver a desired replacement enzyme (e.g., an ASA protein) into the CSF. As used herein, intrathecal administration (also referred to as intrathecal injection) refers to an injection into the spinal canal (intrathecal space surrounding the spinal cord). Various techniques may be used including, without limitation, lumbar puncture. Exemplary methods are described in Lazorthes et al. Advances in Drug Delivery Systems and Applications in Neurosurgery, 143-192, the contents of which are incorporated herein by reference.

According to the present invention, an enzyme may be injected at any region surrounding the spinal canal. In some embodiments, an enzyme is injected into the lumbar area. As used herein, the term "lumbar region" or "lumbar area" refers to the area between the third and fourth lumbar (lower back) vertebrae and, more inclusively, the L2-S1 region of the spine. Typically, intrathecal injection via the lumbar region or lumber area is also referred to as "lumbar intrathecal delivery" or "lumbar intrathecal administration."

In some embodiments, therapeutic proteins, e.g., recombinant arylsulfatase A is delivered by lumbar intrathecal administration, for example, delivered between the third and fourth lumbar (lower back) vertebrae and, more inclusively, the L2-S1 region of the spine. It is contemplated that lumbar intrathecal administration or delivery distinguishes over cisterna magna delivery in that lumbar intrathecal administration or delivery according to the present invention provides better and more effective delivery to the distal spinal canal, while cisterna magna delivery, among other things, typically does not deliver well to the distal spinal canal.

### Device for Intrathecal Delivery

Various devices may be used for intrathecal delivery according to the present invention. In some embodiments, a device for intrathecal administration contains a fluid access port (e.g., injectable port); a hollow body (e.g., catheter) having a first flow orifice in fluid communication with the fluid access port and a second flow orifice configured for insertion into spinal cord; and a securing mechanism for securing the insertion of the hollow body in the spinal cord. As a non-limiting example, a suitable securing mechanism contains one or more nobs mounted on the surface of a hollow body and a sutured ring adjustable over the one or more nobs to prevent the hollow body (e.g., catheter) from slipping out of the spinal cord. In various embodiments, the fluid access port comprises a reservoir. In some embodiments, the fluid access port comprises a mechanical pump (e.g., an infusion pump). In some embodiments, an implanted catheter is connected to either a reservoir (e.g., for bolus delivery), or an infusion pump. The fluid access port may be implanted or external.

In some embodiments, intrathecal administration may be performed by either lumbar puncture (i.e., slow bolus) or via a port-catheter delivery system (i.e., infusion or bolus). In some embodiments, the catheter is inserted between the laminae of the lumbar vertebrae and the tip is threaded up the thecal space to the desired level (generally L3-L4).

In some embodiments, intrathecal administration is through intermittent or continuous access to an implanted intrathecal drug delivery device (IDDD).

Relative to intravenous administration, a single dose volume suitable for intrathecal administration is typically small. Typically, intrathecal delivery according to the present invention maintains the balance of the composition of the CSF as well as the intracranial pressure of the subject. In some embodiments, intrathecal delivery is performed absent the corresponding removal of CSF from a subject. In some embodiments, a suitable single dose volume may be e.g., less than about 10 ml, 8 ml, 6 ml, 5 ml, 4 ml, 3 ml, 2 ml, 1.5 ml, 1 ml, or 0.5 ml. In some embodiments, a suitable single dose volume may be about 0.5-5 ml, 0.5-4 ml, 0.5-3 ml, 0.5-2 ml, 0.5-1 ml, 1-3 ml, 1-5 ml, 1.5-3 ml, 1-4 ml, or 0.5-1.5 ml. In some embodiments, intrathecal delivery according to the present invention involves a step of removing a desired amount of CSF first. In some embodiments, less than about 10 ml (e.g., less than about 9 ml, 8 ml, 7 ml, 6 ml, 5 ml, 4 ml, 3 ml, 2 ml, 1 ml) of CSF is first removed before intrathecal administration. In those cases, a suitable single dose volume may be e.g., more than about 3 ml, 4 ml, 5 ml, 6 ml, 7 ml, 8 ml, 9 ml, 10 ml, 15 ml, or 20 ml.

Other devices for intrathecal administration of therapeutic compositions or formulations to an individual are described in U.S. Pat. No. 6,217,552, incorporated herein by reference. Alternatively, the drug may be intrathecally given, for example, by a single injection, or continuous infusion. It should be understood that the dosage treatment may be in the form of a single dose administration or multiple doses.

For injection, formulations of the invention can be formulated in liquid solutions. In addition, the enzyme may be formulated in solid form and re-dissolved or suspended immediately prior to use. Lyophilized forms are also included. The injection can be, for example, in the form of a bolus injection or continuous infusion (e.g., using infusion pumps) of the enzyme.

### Non-intrathecal delivery methods

Therapeutic enzymes, e.g., recombinant arylsulfatase A, can be administered by non-intrathecal means, for example, by lateral cerebroventricular injection into the brain of a subject. The injection can be made, for example, through a burr hole made in the subject's skull. In another embodiment, the enzyme and/or other pharmaceutical formulation is administered through a surgically inserted shunt into the cerebral ventricle of a subject. For example, the injection can be made into the lateral ventricles, which are larger. In some embodiments, injection into the third and fourth smaller ventricles can also be made.

Alternatively, pharmaceutical compositions can be administered by injection into the cisterna magna, or lumbar area of a subject.

Certain devices may be used to effect administration of a therapeutic composition. For example, formulations containing desired enzymes may be given using an Ommaya reservoir which is in common use for intrathecally administering drugs for meningeal carcinomatosis (Lancet 2: 983-84, 1963). More specifically, in this method, a ventricular tube is inserted through a hole formed in the anterior horn and is connected to an Ommaya reservoir installed under the scalp, and the reservoir is subcutaneously punctured to intrathecally deliver the particular enzyme being replaced, which is injected into the reservoir.

*Slow release / sustained delivery*

In another embodiment of the method of the invention, the pharmaceutically acceptable formulation provides sustained delivery, e.g., "slow release" of the enzyme or other pharmaceutical composition used in the present invention, to a subject for at least one, two, three, four weeks or longer periods of time after the pharmaceutically acceptable formulation is administered to the subject.

As used herein, the term "sustained delivery" refers to continual delivery of a pharmaceutical formulation of the invention *in vivo* over a period of time following administration, preferably at least several days, a week or several weeks. Sustained delivery of the composition can be demonstrated by, for example, the continued therapeutic effect of the enzyme over time (e.g., sustained delivery of the enzyme can be demonstrated by continued reduced amount of storage granules in the subject). Alternatively, sustained delivery of the enzyme may be demonstrated by detecting the presence of the enzyme in vivo over time.

*Delivery to Target Tissues*

As discussed above, one of the surprising and important features of the present invention is that therapeutic agents, in particular, replacement enzymes administered using inventive methods and compositions of the present invention are able to effectively and extensively diffuse across the brain surface and penetrate various layers or regions of the brain, including deep brain regions. In addition, inventive methods and compositions of the present invention effectively deliver therapeutic agents (e.g., an ASA enzyme) to various tissues, neurons or cells of spinal cord, including the lumbar region, which is hard to target by existing CNS delivery methods such as ICV injection. Furthermore, inventive methods and compositions of the present invention deliver sufficient amount of therapeutic agents (e.g., an ASA enzyme) to blood stream and various peripheral organs and tissues.

Thus, in some embodiments, a therapeutic protein (e.g., an ASA enzyme) is delivered to the central nervous system of a subject. In some embodiments, a therapeutic protein (e.g., an ASA enzyme) is delivered to one or more of target tissues of brain, spinal cord, and/or peripheral organs. As used herein, the term "target tissues" refers to any tissue that is affected by the lysosomal storage disease to be treated or any tissue in which the deficient lysosomal enzyme is normally expressed. In some embodiments, target tissues include those tissues in which there is a detectable or abnormally high amount of enzyme substrate, for example stored in the cellular lysosomes of the tissue, in patients suffering from or susceptible to the lysosomal storage disease. In some embodiments, target tissues include those tissues that display disease-associated pathology, symptom, or feature. In some embodiments, target tissues include those tissues in which the deficient lysosomal enzyme is normally expressed at an elevated level. As used herein, a target tissue may be a brain target tissue, a spinal cord target tissue and/or a peripheral target tissue. Exemplary target tissues are described in detail below.

*Brain Target Tissues*

In general, the brain can be divided into different regions, layers and tissues. For example, meningeal tissue is a system of membranes which envelops the central nervous system, including the brain. The meninges contain three layers, including dura matter, arachnoid matter, and pia matter. In general, the primary function of the meninges and of the cerebrospinal fluid is to protect the central nervous system. In some embodiments, a therapeutic protein in accordance with the present invention is delivered to one or more layers of the meninges.

The brain has three primary subdivisions, including the cerebrum, cerebellum, and brain stem. The cerebral hemispheres, which are situated above most other brain structures and are covered with a cortical layer. Underneath the cerebrum lies the brainstem, which resembles a stalk on which the cerebrum is attached. At the rear of the brain, beneath the cerebrum and behind the brainstem, is the cerebellum.

The diencephalon, which is located near the midline of the brain and above the mesencephalon, contains the thalamus, metathalamus, hypothalamus, epithalamus, prethalamus, and pretectum. The mesencephalon, also called the midbrain, contains the tectum, tegumentum, ventricular mesocoelia, and cerebral peduncels, the red nucleus, and the cranial nerve III nucleus. The mesencephalon is associated with vision, hearing, motor control, sleep/wake, alertness, and temperature regulation.

Regions of tissues of the central nervous system, including the brain, can be characterized based on the depth of the tissues. For example, CNS (e.g., brain) tissues can be characterized as surface or shallow tissues, mid-depth tissues, and/or deep tissues.

According to the present invention, a therapeutic protein (e.g., a replacement enzyme) may be delivered to any appropriate brain target tissue(s) associated with a particular disease to be treated in a subject. In some embodiments, a therapeutic protein (e.g., a replacement enzyme) in accordance with the present invention is delivered to surface or shallow brain target tissue. In some embodiments, a therapeutic protein in accordance with the present invention is delivered to mid-depth brain target tissue. In some embodiments, a therapeutic protein in accordance with the present invention is delivered to deep brain target tissue. In some embodiments, a therapeutic protein in accordance with the present invention is delivered to a combination of surface or shallow brain target tissue, mid-depth brain target tissue, and/or deep brain target tissue. In some embodiments, a therapeutic protein in accordance with the present invention is delivered to a deep brain tissue at least 4 mm, 5 mm, 6 mm, 7 mm, 8 mm, 9 mm, 10 mm or more below (or internal to) the external surface of the brain.

In some embodiments, therapeutic agents (e.g., enzymes) are delivered to one or more surface or shallow tissues of cerebrum. In some embodiments, the targeted surface or shallow tissues of the cerebrum are located within 4 mm from the surface of the cerebrum. In some embodiments, the targeted surface or shallow tissues of the cerebrum are selected from pia mater tissues, cerebral cortical ribbon tissues, hippocampus, Virchow Robin space, blood vessels within the VR space, the hippocampus, portions of the hypothalamus on the inferior surface of the brain, the optic nerves and tracts, the olfactory bulb and projections, and combinations thereof.

In some embodiments, therapeutic agents (e.g., enzymes) are delivered to one or more deep tissues of the cerebrum. In some embodiments, the targeted surface or shallow tissues of the cerebrum are located 4 mm (e.g., 5 mm, 6 mm, 7 mm, 8 mm, 9 mm, or 10 mm) below (or internal to) the surface of the cerebrum. In some embodiments, targeted deep tissues of the cerebrum include the cerebral cortical ribbon. In some embodiments, targeted deep tissues of the cerebrum include one or more of the diencephalon (e.g., the hypothalamus, thalamus, prethalamus, subthalamus, etc.), metencephalon, lentiform nuclei, the basal ganglia, caudate, putamen, amygdala, globus pallidus, and combinations thereof.

In some embodiments, therapeutic agents (e.g., enzymes) are delivered to one or more tissues of the cerebellum. In certain embodiments, the targeted one or more tissues of the cerebellum are selected from the group consisting of tissues of the molecular layer, tissues of the Purkinje cell layer, tissues of the Granular cell layer, cerebellar peduncles, and combination thereof. In some embodiments, therapeutic agents (e.g., enzymes) are delivered to one or more deep tissues of the cerebellum including, but not limited to, tissues of the Purkinje cell layer, tissues of the Granular cell layer, deep cerebellar white matter tissue (e.g., deep relative to the Granular cell layer), and deep cerebellar nuclei tissue.

In some embodiments, therapeutic agents (e.g., enzymes) are delivered to one or more tissues of the brainstem. In some embodiments, the targeted one or more tissues of the brainstem include brain stem white matter tissue and/or brain stem nuclei tissue.

In some embodiments, therapeutic agents (e.g., enzymes) are delivered to various brain tissues including, but not limited to, gray matter, white matter, periventricular areas, pia-arachnoid, meninges, neocortex, cerebellum, deep tissues in cerebral cortex, molecular layer, caudate/putamen region, midbrain, deep regions of the pons or medulla, and combinations thereof.

In some embodiments, therapeutic agents (e.g., enzymes) are delivered to various cells in the brain including, but not limited to, neurons, glial cells, perivascular cells and/or meningeal cells. In some embodiments, a therapeutic protein is delivered to oligodendrocytes of deep white matter.

## *Spinal Cord*

In general, regions or tissues of the spinal cord can be characterized based on the depth of the tissues. For example, spinal cord tissues can be characterized as surface or shallow tissues, mid-depth tissues, and/or deep tissues.

In some embodiments, therapeutic agents (e.g., enzymes) are delivered to one or more surface or shallow tissues of the spinal cord. In some embodiments, a targeted surface or shallow tissue of the spinal cord is located within 4 mm from the surface of the spinal cord. In some embodiments, a targeted surface or shallow tissue of the spinal cord contains pia matter and/or the tracts of white matter.

In some embodiments, therapeutic agents (e.g., enzymes) are delivered to one or more deep tissues of the spinal cord. In some embodiments, a targeted deep tissue of the spinal cord is located internal to 4 mm from the surface of the spinal cord. In some embodiments, a targeted deep tissue of the spinal cord contains spinal cord grey matter and/or ependymal cells.

In some embodiments, therapeutic agents (e.g., enzymes) are delivered to neurons of the spinal cord.

## *Peripheral Target Tissues*

As used herein, peripheral organs or tissues refer to any organs or tissues that are not part of the central nervous system (CNS). Peripheral target tissues may include, but are not limited to, blood system, liver, kidney, heart, endothelium, bone marrow and bone marrow derived cells, spleen, lung, lymph node, bone, cartilage, ovary and testis. In some embodiments, a therapeutic protein (e.g., a replacement enzyme) in accordance with the present invention is delivered to one or more of the peripheral target tissues.

*Biodistribution and bioavailability*

In various embodiments, once delivered to the target tissue, a therapeutic agent (e.g., an ASA enzyme) is localized intracellularly. For example, a therapeutic agent (e.g., enzyme) may be localized to exons, axons, lysosomes, mitochondria or vacuoles of a target cell (e.g., neurons such as Purkinje cells). For example, in some embodiments intrathecally-administered enzymes demonstrate translocation dynamics such that the enzyme moves within the perivascular space (e.g., by pulsation-assisted convective mechanisms). In addition, active axonal transport mechanisms relating to the association of the administered protein or enzyme with neurofilaments may also contribute to or otherwise facilitate the distribution of intrathecally-administered proteins or enzymes into the deeper tissues of the central nervous system.

In some embodiments, a therapeutic agent (e.g., an ASA enzyme) delivered according to the present invention may achieve therapeutically or clinically effective levels or activities in various targets tissues described herein. As used herein, a therapeutically or clinically effective level or activity is a level or activity sufficient to confer a therapeutic effect in a target tissue. The therapeutic effect may be objective (i.e., measurable by some test or marker) or subjective (i.e., subject gives an indication of or feels an effect). For example, a therapeutically or clinically effective level or activity may be an enzymatic level or activity that is sufficient to ameliorate symptoms associated with the disease in the target tissue (e.g., GAG storage).

**Therapeutically effective dose and administration interval**

A therapeutically effective amount is commonly administered in a dosing regimen that may comprise multiple unit doses. For any particular therapeutic protein, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the patient; the time of administration, route of administration, and/or rate of excretion or metabolism of the specific fusion protein employed; the duration of the treatment; and like factors as is well known in the medical arts.

In some embodiments, a therapeutically effective dose is or greater than about 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, or about 200 mg. In particular embodiments, a therapeutically effective dose is or is greater than about 150 mg. In some embodiments, a therapeutically effective dose is or is less than about 500 mg, 450 mg, 400 mg, 350 mg, 300 mg, 250 mg, 200 mg, or 150 mg. In particular embodiments, a therapeutically effective dose is less than about 200 mg. In some embodiments, a therapeutically effective dose ranges between about 10-200 mg, about 20-200 mg, about 30-200 mg, about 40-200 mg, about 50-200 mg, about 160-200 mg, about 70-200 mg, about 80-200 mg, about 90-200 mg, about 100-200 mg, about 125-200, about 150-200, about 50-300 mg, about 75-300 mg, about 100-300 mg, about 150-300 or about 10-50 mg. In some embodiments, a therapeutically effective dose ranges between about 100 mg and about 200 mg.

In some embodiments, the therapeutically effective dose ranges from about 0.005 mg/kg brain weight to 500 mg/kg brain weight, e.g., from about 0.005 mg/kg brain weight to 400 mg/kg brain weight, from about 0.005 mg/kg brain weight to 300 mg/kg brain weight, from about 0.005 mg/kg brain weight to 200 mg/kg brain weight, from about 0.005 mg/kg brain weight to 100 mg/kg brain weight, from about 0.005 mg/kg brain weight to 90 mg/kg brain weight, from about 0.005 mg/kg brain weight to 80 mg/kg brain weight, from about 0.005 mg/kg brain weight to 70 mg/kg brain weight, from about 0.005 mg/kg brain weight to 60 mg/kg brain weight, from about 0.005 mg/kg brain weight to 50 mg/kg brain weight, from about 0.005 mg/kg brain weight to 40 mg/kg brain weight, from about 0.005 mg/kg brain weight to 30 mg/kg brain weight, from about 0.005 mg/kg brain weight to 25 mg/kg brain weight, from about 0.005 mg/kg brain weight to 20 mg/kg brain weight, from about 0.005 mg/kg brain weight to 15 mg/kg brain weight, from about 0.005 mg/kg brain weight to 10 mg/kg brain weight.

In some embodiments, the therapeutically effective dose is greater than about 0.1 mg/kg brain weight, greater than about 0.5 mg/kg brain weight, greater than about 1.0 mg/kg brain weight, greater than about 3 mg/kg brain weight, greater than about 5 mg/kg brain weight, greater than about 10 mg/kg brain weight, greater than about 15 mg/kg brain weight, greater than about 20 mg/kg brain weight, greater than about 30 mg/kg brain weight, greater than about 40 mg/kg brain weight, greater than about 50 mg/kg brain weight, greater than about 60 mg/kg brain weight, greater than about 70 mg/kg brain weight, greater than about 80 mg/kg brain weight, greater than about 90 mg/kg brain weight, greater than about 100 mg/kg brain weight, greater than about 150 mg/kg brain weight, greater than about 200 mg/kg brain weight, greater than about 250 mg/kg brain weight, greater than about 300 mg/kg brain weight, greater than about 350 mg/kg brain weight, greater than about 400 mg/kg brain weight, greater than about 450 mg/kg brain weight, greater than about 500 mg/kg brain weight.

In some embodiments, the therapeutically effective dose may also be defined by mg/kg body weight. As one skilled in the art would appreciate, the brain weights and body weights can be correlated. Dekaban AS. "Changes in brain weights during the span of human life: relation of brain weights to body heights and body weights," Ann Neurol 1978; 4:345-56. Thus, in some embodiments, the dosages can be converted as shown in Table 2.

**Table 2**

| Correlation between Brain Weights, body weights and ages of males | | |
| --- | --- | --- |
| Age (year) | Brain weight (kg) | Body weight (kg) |
| 3 (31-43 months) | 1.27 | 15.55 |
| 4-5 | 1.30 | 19.46 |

In some embodiments, the therapeutically effective dose may also be defined by mg/15 cc of CSF. As one skilled in the art would appreciate, therapeutically effective doses based on brain weights and body weights can be converted to mg/15 cc of CSF. For example, the volume of CSF in adult humans is approximately 150 mL (Johanson CE, et al. "Multiplicity of cerebrospinal fluid functions: New challenges in health and disease," Cerebrospinal Fluid Res. 2008 May 14;5: 10). Therefore, single dose injections of 0.1 mg to 50 mg protein to adults would be approximately 0.01 mg/15 cc of CSF (0.1 mg) to 5.0 mg/15 cc of CSF (50 mg) doses in adults.

It is to be further understood that for any particular subject, specific dosage regimens should be adjusted over time according to the individual need and the professional judgment of the person administering or supervising the administration of the enzyme replacement therapy and that dosage ranges set forth herein are exemplary only and are not intended to limit the scope or practice of the claimed invention.

In certain embodiments, recombinant arylsulfatase A enzyme is administered at an administration interval, e.g., a regular interval. For example, the administration interval can be once a week, once every two weeks, or once a month.

In certain embodiments, recombinant arylsulfatase A enzyme is administered for a treatment period. The treatment period can be predetermined or it can be adjusted depending on the patient's response to the treatment, including, but not limited to, possible adverse symptoms and/or evidence of treatment efficacy as discussed herein. For example, the treatment period can be at least 6 months, at least 9 months, at least 12 months, at least 24 months, or even greater.

It is contemplated that in some embodiments, subjects will undergo treatment over a certain treatment period, undergo a certain period during which they receive no treatment or an alternative treatment, and then undergo again another treatment period.

In some embodiments, a therapeutic agent (e.g., a replacement enzyme) delivered according to the present invention may achieve an enzymatic level or activity that is at least 5%, 10%, 20%, 30%, 40%, 50%, 60%, 70%, 80%, 90%, 95% of the normal level or activity of the corresponding lysosomal enzyme in the target tissue. In some embodiments, a therapeutic agent (e.g., a replacement enzyme) delivered according to the present invention may achieve an enzymatic level or activity that is increased by at least 1-fold, 2-fold, 3-fold, 4-fold, 5-fold, 6-fold, 7-fold, 8-fold, 9-fold or 10-fold as compared to a control or to baseline (e.g., endogenous levels or activities without the treatment). In some embodiments, a therapeutic agent (e.g., a replacement enzyme) delivered according to the present invention may achieve an increased enzymatic level or activity at least approximately 10 nmol/hr/mg, 20 nmol/hr/mg, 40 nmol/hr/mg, 50 nmol/hr/mg, 60 nmol/hr/mg, 70 nmol/hr/mg, 80 nmol/hr/mg, 90 nmol/hr/mg, 100 nmol/hr/mg, 150 nmol/hr/mg, 200 nmol/hr/mg, 250 nmol/hr/mg, 300 nmol/hr/mg, 350 nmol/hr/mg, 400 nmol/hr/mg, 450 nmol/hr/mg, 500 nmol/hr/mg, 550 nmol/hr/mg or 600 nmol/hr/mg in a target tissue.

In some embodiments, inventive methods according to the present invention are particularly useful for targeting the lumbar region. In some embodiments, a therapeutic agent (e.g., a replacement enzyme) delivered according to the present invention may achieve an increased enzymatic level or activity in the lumbar region of at least approximately 500 nmol/hr/mg, 600 nmol/hr/mg, 700 nmol/hr/mg, 800 nmol/hr/mg, 900 nmol/hr/mg, 1000 nmol/hr/mg, 1500 nmol/hr/mg, 2000 nmol/hr/mg, 3000 nmol/hr/mg, 4000 nmol/hr/mg, 5000 nmol/hr/mg, 6000 nmol/hr/mg, 7000 nmol/hr/mg, 8000 nmol/hr/mg, 9000 nmol/hr/mg, or 10,000 nmol/hr/mg.

In general, therapeutic agents (e.g., replacement enzymes) delivered according to the present invention have sufficiently long half time in CSF and target tissues of the brain, spinal cord, and peripheral organs. In some embodiments, a therapeutic agent (e.g., a replacement enzyme) delivered according to the present invention may have a half-life of at least approximately 30 minutes, 45 minutes, 60 minutes, 90 minutes, 2 hours, 3 hours, 4 hours, 5 hours, 6 hours, 7 hours, 8 hours, 9 hours, 10 hours, 12 hours, 16 hours, 18 hours, 20 hours, 25 hours, 30 hours, 35 hours, 40 hours, up to 3 days, up to 7 days, up to 14 days, up to 21 days or up to a month. In some embodiments, In some embodiments, a therapeutic agent (e.g., a replacement enzyme) delivered according to the present invention may retain detectable level or activity in CSF or bloodstream after 12 hours, 24 hours, 30 hours, 36 hours, 42 hours, 48 hours, 54 hours, 60 hours, 66 hours, 72 hours, 78 hours, 84 hours, 90 hours, 96 hours, 102 hours, or a week following administration. Detectable level or activity may be determined using various methods known in the art.

In certain embodiments, a therapeutic agent (e.g., a replacement enzyme) delivered according to the present invention achieves a concentration of at least 30 μg/ml in the CNS tissues and cells of the subject following administration (e.g., one week, 3 days, 48 hours, 36 hours, 24 hours, 18 hours, 12 hours, 8 hours, 6 hours, 4 hours, 3 hours, 2 hours, 1 hour, 30 minutes, or less, following intrathecal administration of the pharmaceutical composition to the subject). In certain embodiments, a therapeutic agent (e.g., a replacement enzyme) delivered according to the present invention achieves a concentration of at least 20 μg/ml, at least 15 μg/ml, at least 10 μg/ml, at least 7.5 μg/ml, at least 5 μg/ml, at least 2.5 μg/ml, at least 1.0 μg/ml or at least 0.5 μg/ml in the targeted tissues or cells of the subject(e.g., brain tissues or neurons) following administration to such subject (e.g., one week, 3 days, 48 hours, 36 hours, 24 hours, 18 hours, 12 hours, 8 hours, 6 hours, 4 hours, 3 hours, 2 hours, 1 hour, 30 minutes, or less following intrathecal administration of such pharmaceutical compositions to the subject).

**Therapeutic Formulations**

A recombinant ASA or a pharmaceutical composition containing the same can be formulated with a physiologically acceptable carrier or excipient to prepare a pharmaceutical composition. The carrier and therapeutic agent can be sterile. The formulation should suit the mode of administration.

Suitable pharmaceutically acceptable carriers include but are not limited to water, salt solutions (e.g., NaCl), saline, buffered saline, alcohols, glycerol, ethanol, gum arabic, vegetable oils, benzyl alcohols, polyethylene glycols, gelatin, carbohydrates such as lactose, amylose or starch, sugars such as mannitol, sucrose, or others, dextrose, magnesium stearate, talc, silicic acid, viscous paraffin, perfume oil, fatty acid esters, hydroxymethylcellulose, polyvinyl pyrolidone, etc., as well as combinations thereof. The pharmaceutical preparations can, if desired, be mixed with auxiliary agents (e.g., lubricants, preservatives, stabilizers, wetting agents, emulsifiers, salts for influencing osmotic pressure, buffers, coloring, flavoring and/or aromatic substances and the like) which do not deleteriously react with the active compounds or interference with their activity. In some embodiments, a water-soluble carrier suitable for intravenous administration is used.

The composition or medicament, if desired, can also contain minor amounts of wetting or emulsifying agents, or pH buffering agents. The composition can be a liquid solution, suspension, emulsion, tablet, pill, capsule, sustained release formulation, or powder. The composition can also be formulated as a suppository, with traditional binders and carriers such as triglycerides. Oral formulation can include standard carriers such as pharmaceutical grades of mannitol, lactose, starch, magnesium stearate, polyvinyl pyrollidone, sodium saccharine, cellulose, magnesium carbonate, etc.

The composition or medicament can be formulated in accordance with the routine procedures as a pharmaceutical composition adapted for administration to human beings. For example, in some embodiments, a composition for intravenous administration typically is a solution in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water, saline or dextrose/water. Where the composition is administered by injection, an ampule of sterile water for injection or saline can be provided so that the ingredients may be mixed prior to administration.

In some embodiments, arylsulfatase A is formulated in an isotonic solution such as 154 mM NaCl, or 0.9% NaCl and 10-50 mM sodium phosphate pH 6.5-8.0 or sodium phosphate, glycine, mannitol or the corresponding potassium salts. In embodiments, the osmolality of a formulation is about 250 to about 350 mOsmol/kg (e.g., about 255 to about 320 mOsmol/kg, about 260 to about 310 mOsmol/kg, or about 280 to about 300 mOsmol/kg).

In another embodiment, the ASA is formulated in a physiological buffer, such as:

a) formulation buffer I containing (in mM): $Na_2HPO_4$ (3.50 - 3.90), $NaH_2PO_4$ (0 - 0.5), Glycine (25 - 30), Mannitol (230 - 270), and water for injection; or

b) formulation buffer II containing (in mM): Tris-HCl (10), Glycine (25 - 30), Mannitol (230 - 270), and water for injection.

Arylsulfatase A purified by a method herein can be used as a medicament for reducing the sphingolipid 3-O-sulfogalactosylceramide (galactosyl sulphatide) levels within cells in the peripheral nervous system and/or within the central nervous system in a subject suffering from and/or being diagnosed with Metachromatic Leukodystrophy. The administration of ASA will lead to decreased impairment of motor-learning skills and or to increased nerve motor conduction velocity and/or nerve conduction amplitude. As used herein, the term "therapeutically effective amount" is largely determined based on the total amount of the therapeutic agent contained in the pharmaceutical compositions of the present invention. Generally, a therapeutically effective amount is sufficient to achieve a meaningful benefit to the subject (e.g., treating, modulating, curing, preventing and/or ameliorating the underlying disease or condition). For example, a therapeutically effective amount may be an amount sufficient to achieve a desired therapeutic and/or prophylactic effect, such as an amount sufficient to modulate lysosomal enzyme receptors or their activity to thereby treat such lysosomal storage disease or the symptoms thereof (e.g., a reduction in or elimination of the presence or incidence of "zebra bodies" or cellular vacuolization following the administration of the compositions of the present invention to a subject). Generally, the amount of a therapeutic agent (e.g., a recombinant lysosomal enzyme) administered to a subject in need thereof will depend upon the characteristics of the subject. Such characteristics include the condition, disease severity, general health, age, sex and body weight of the subject. One of ordinary skill in the art will be readily able to determine appropriate dosages depending on these and other related factors. In addition, both objective and subjective assays may optionally be employed to identify optimal dosage ranges.

In embodiments, characteristic features of purified recombinant ASA protein (e.g., a characteristic glycan map such as purified recombinant ASA protein having a threshold amount of mannose-6-phosphated recombinant ASA protein) as well as other properties such as a characteristic maximum level of Host Cell Protein (HCP), a characteristic maximum level of Host Cell DNA (HCD), and/or a particular specific activity) can result desirable properties of compositions comprising a purified recombinant ASA protein (e.g., improved stability to storage, improved therapeutic properties, and the like).

In embodiments, the present invention provides a method of purifying a recombinant ASA protein from an impure preparation (e.g., unprocessed biological materials, such as, ASA-containing cell culture medium) using a process involving only a single step of post-chromatographic ultrafiltration/diafiltration. In embodiments, this single step UF/DF process is achieved by pooling eluate from the chromatography steps and adjusting the pH of the pooled eluate to or greater than about 6.0. In some embodiments, this simplified process is combined with high loading capacity chromatography steps to facilitate large scale production of recombinant ASA protein.

Various aspects of the invention are described in further detail in the following subsections. The use of subsections is not meant to limit the invention. Each subsection may apply to any aspect of the invention. In this application, the use of "or" means "and/or" unless stated otherwise.

### *Arylsulfatase A*

Arylsulfatase A (ASA, ARSA, or cerebroside-sulfatase) is an enzyme that breaks down cerebroside 3-sulfate (or sulfatide) into cerebroside and sulfate. Specifically, galactosyl sulfatide is normally metabolized by the hydrolysis of 3-O-sulphate linkage to form galactocerebroside through the combined action of the lysosomal enzyme Arylsulfatase A (EC 3.1.6.8) (Austin et al. Biochem J. 1964, 93, 15C-17C) and a sphingolipid activator protein called saposin B. A deficiency of Arylsulfatase A occurs in all tissues from patients with the late infantile, juvenile, and adult forms of Metachromatic Leukodystrophy (MLD). As used herein, the Arylsulfatase A protein will be termed "ASA" or "ARSA" and the saposin B will be termed "Sap-B".

Arylsulfatase A is an acidic glycoprotein with a low isoelectric point. Above pH 6.5, the enzyme exists as a monomer with a molecular weight of approximately 100 kDa. ASA exists as a 480 kDa octamer in acidic conditions (pH ≤ about 5.0), but dissociates into dimers at neutral pH levels. In human urine, the enzyme consists of two non-identical subunits of 63 and 54 kDa (Laidler PM et al. Biochim Biophys Acta. 1985, 827, 73-83). Arylsulfatase A purified from human liver, placenta, and fibroblasts also consist of two subunits of slightly different sizes varying between 55 and 64 kDa (Draper RK et al. Arch Biochemica Biophys. 1976, 177, 525-538, Waheed A et al. Hoppe Seylers Z Physiol Chem. 1982, 363, 425-430, Fujii T et al. Biochim Biophys Acta. 1992, 15 1122, 93-98). As in the case of other lysosomal enzymes, arylsulfatase A is synthesized on membrane-bound ribosomes as a glycosylated precursor. It then passes through the endoplasmic reticulum and Golgi, where its N-linked oligosaccharides are processed with the formation of phosphorylated and sulfated oligosaccharide of the complex type (Waheed A et al. Biochim Biophys Acta. 1985, 847, 53-61, Braulke T et al. Biochem Biophys Res Commun. 1987, 143, 178-185). In normal cultured fibroblasts, a precursor polypeptide of 62 kDa is produced, which translocates via mannose-6-phosphate receptor binding (Braulke T et al. J Biol Chem. 1990, 265, 6650-6655) to an acidic prelysosomal endosome (Kelly BM et al. Eur J Cell Biol. 1989, 48, 71-78).

The methods described herein can be used to purify arylsulfatase A from any source, e.g., from tissues, or cultured cells (e.g., human cells (e.g., fibroblasts) that recombinantly produce arylsulfatase A). Arylsulfatase A of any origin, including, but not limited to human and other animals, such as mammals, can be produced by the methods described herein.

The length (18 amino acids) of the human Arylsulfatase A signal peptide is based on the consensus sequence and a specific processing site for a signal sequence. Hence, from the deduced human ASA cDNA (EMBL GenBank accession numbers J04593 and X521151) the cleavage of the signal peptide occurs in all cells after residue number 18 (Ala), resulting in the mature form of the human arylsulfatase A. As used herein, recombinant arylsulfatase A will be abbreviated "rASA". The mature form of arylsulfatase A including the mature form of human arylsulfatase A will be termed "mASA" and the mature recombinant human ASA will be termed "mrhASA".

Multiple forms of arylsulfatase A have been demonstrated on electrophoresis and isoelectric focusing of enzyme preparations from human urine (Luijten JAFM et al. J Mol Med. 1978, 3, 213), leukocytes (Dubois et al. Biomedicine. 1975, 23, 116-119, Manowitz P et al. Biochem Med Metab Biol. 1988, 39, 117-120), platelets (Poretz et al. Biochem J. 1992, 287, 979-983), cultured fibroblasts (Waheed A et al. Hoppe Seylers Z Physiol Chem. 1982, 363, 425-430, Stevens RL et al. Biochim Biophys Acta. 1976, 445, 661-671, Farrell DF et al. Neurology. 1979, 29, 16-20) and liver (Stevens RL et al. Biochim Biophys Acta. 1976, 445, 661-671, Farrell DF et al. Neurology. 1979, 29, 16-20, Sarafian TA et al. Biochem Med. 1985, 33, 372-380). Treatment with endoglycosidase H, sialidase, and alkaline phosphatase reduces the molecular size and complexity of the electrophoretic pattern, which suggests that much of the charge heterogeneity of arylsulfatase A is due to variations in the carbohydrate content of the enzyme.

The active site of arylsulfatase A contains an essential histidine residue (Lee GD and Van Etten RL, Arch Biochem Biophys. 1975, 171, 424-434) and two or more arginine residues (James GT, Arch Biochem Biophys. 1979, 97, 57-62). Many anions are inhibitors of the enzyme at concentrations in the millimolar range or lower.

The human arylsulfatase A gene structure has been described. As used herein, this gene will be termed "ARSA." However, "ARSA" may also refer to arylsulfatase A protein in some cases. The ARSA gene is located near the end of the long arm of chromosome 22 (22ql3.31-qter), it spans 3.2 kb (Kreysing et al. Eur J Biochem. 1990, 191, 627-631) and consists of eight exons specifying the 507 amino acid enzyme unit (Stein et al. J Biol Chem. 1989, 264, 1252-1259). Messenger RNAs of 2.1, 3.7, and 4.8 kb have been detected in fibroblast cells, with the 2.1-kb message apparently responsible for the bulk of the active arylsulfatase A generated by the cell (Kreysing et al. Eur J Biochem. 1990, 191, 627-631). The ARSA sequence has been deposited at the EMBL GenBank with the accession number X521150. Differences between the published cDNA and the coding part of the ARSA were described by Kreysing et al. (Eur J Biochem. 1990, 191, 627-631). The cDNA sequence originally described by Stein et al. (J Biol Chem. 1989, 264, 1252-1259) and the cDNA sequence described by Kreysing et al. (Eur J Biochem. 1990, 191, 627-631) have been deposited at the EMBL GenBank with the following accession numbers J04593 and X521151, respectively.

Several polymorphisms and more than 40 disease-related mutations have been identified in the ARSA gene (Gieselmann et al. Hum Mutat. 1994, 4, 233-242, Barth et al. Hum Mutat. 1995, 6, 170-176, Draghia et al. Hum Mutat. 1997, 9, 234-242). The disease-related mutations in the ARSA gene can be categorized in two broad groups that correlate fairly well with the clinical phenotype of MLD. One group (I) produces no active enzyme, no immunoreactive protein, and expresses no ASA activity when introduced into cultured animal cell lines. The other group (A) generates small amounts of cross-reactive material and low levels of functional enzyme in cultured cells. Individuals homozygous for a group (I) mutation, or having two different mutations from this group, express late infantile MLD. Most individuals with one group (I)-type and one group (A)-type mutation develop the juvenile-onset form, whereas those with two group (A)-type mutations generally manifest adult MLD. Some of the mutations have been found relatively frequently, whereas others have been detected only in single families. It is possible to trace specific mutations through members of many families, however general carrier screening is not yet feasible.

In addition to the disease-related mutations described above, several polymorphisms have been identified in the ARSA gene. Extremely low ASA activity has been found in some clinically normal parents of MLD patients and also in the general population. This so-called pseudodeficiency ASA has been associated with a common polymorphism of the ARSA gene (Gieselmann et al. Dev Neurosci. 1991, 13, 222-227).

***Recombinant ASA Protein***

As used herein, the term "recombinant ASA protein" refers to any molecule or a portion of a molecule that can substitute for at least partial activity of naturally-occurring Arylsulfatase A (ASA) protein or rescue one or more phenotypes or symptoms associated with ASA-deficiency. As used herein, the terms "recombinant ASA enzyme" and "recombinant ASA protein", and grammatical equivalents, are used inter-changeably. In some embodiments, the present invention is used to purify a recombinant ASA protein that is a polypeptide having an amino acid sequence substantially similar or identical to mature human ASA protein.

Typically, human ASA is produced as a precursor molecule that is processed to a mature form. This process generally occurs by removing the 18 amino acid signal peptide. Typically, the precursor form is also referred to as full-length precursor or full-length ASA protein, which contains 507 amino acids. The N-terminal 18 amino acids are cleaved, resulting in a mature form that is 489 amino acids in length. Thus, it is contemplated that the N-terminal 18 amino acids is generally not required for the ASA protein activity. The amino acid sequences of the mature form (SEQ ID NO:1) and full-length precursor (SEQ ID NO:2) of a typical wild-type or naturally-occurring human ASA protein are shown in Table 1.

**TABLE 1. Human Arylsulfatase A**

| Mature Form | RPPNIVLIFADDLGYGDLGCYGHPSSTTPNLDQLAAGGLRFT DFYVPVSLCTPSRAALLTGRLPVRMGMYPGVLVPSSRGGLP LEEVTVAEVLAARGYLTGMAGKWHLGVGPEGAFLPPHQGF HRFLGIPYSHDQGPCQNLTCFPPATPCDGGCDQGLVPIPLLA NLSVEAQPPWLPGLEARYMAFAHDLMADAQRQDRPFFLYY ASHHTHYPQFSGQSFAERSGRGPFGDSLMELDAAVGTLMTA IGDLGLLEETLVIFTADNGPETMRMSRGGCSGLLRCGKGTTY EGGVREPALAFWPGHIAPGVTHELASSLDLLPTLAALAGAPL PNVTLDGFDLSPLLLGTGKSPRQSLFFYPSYPDEVRGVFAVR TGKYKAHFFTQGSAHSDTTADPACHASSSLTAHEPPLLYDLS KDPGENYNLLGGVAGATPEVLQALKQLQLLKAQLDAAVTF GPSQVARGEDPALQICCHPGCTPRPACCHCPDPHA (SEQ ID NO:1) |
| --- | --- |
| Full-Length Precursor | MGAPRSLLLALAAGLAVARPPNIVLIFADDLGYGDLGCYGH PSSTTPNLDQLAAGGLRFTDFYVPVSLCTPSRAALLTGRLPV RMGMYPGVLVPSSRGGLPLEEVTVAEVLAARGYLTGMAGK WHLGVGPEGAFLPPHQGFHRFLGIPYSHDQGPCQNLTCFPPA TPCDGGCDQGLVPIPLLANLSVEAQPPWLPGLEARYMAFAH DLMADAQRQDRPFFLYYASHHTHYPQFSGQSFAERSGRGPF GDSLMELDAAVGTLMTAIGDLGLLEETLVIFTADNGPETMR MSRGGCSGLLRCGKGTTYEGGVREPALAFWPGHIAPGVTHE LASSLDLLPTLAALAGAPLPNVTLDGFDLSPLLLGTGKSPRQ SLFFYPSYPDEVRGVFAVRTGKYKAHFFTQGSAHSDTTADP ACHASSSLTAHEPPLLYDLSKDPGENYNLLGGVAGATPEVL QALKQLQLLKAQLDAAVTFGPSQVARGEDPALQICCHPGCT PRPACCHCPDPHA (SEQ ID NO:2) |

Thus, in some embodiments, a recombinant ASA protein purified by embodiments of the present invention is mature human ASA protein (SEQ ID NO: 1). In some embodiments, a recombinant ASA protein purified by embodiments of the present invention may be a homologue or an analogue of mature human ASA protein. For example, a homologue or an analogue of mature human ASA protein may be a modified mature human ASA protein containing one or more amino acid substitutions, deletions, and/or insertions as compared to a wild-type or naturally-occurring ASA protein (e.g., SEQ ID NO: 1), while retaining substantial ASA protein activity. Thus, in some embodiments, a recombinant ASA protein purified by embodiments of the present invention is substantially homologous to mature human ASA protein (SEQ ID NO: 1). In some embodiments, a recombinant ASA protein purified by embodiments of the present invention has an amino acid sequence at least 50%, 55%, 60%, 65%, 70%, 75%, 80%, 85%, 90%, 91%, 92%, 93%, 94%, 95%, 96%, 97%, 98%, 99% or more homologous to SEQ ID NO: 1. In some embodiments, a recombinant ASA protein purified by embodiments of the present invention is substantially identical to mature human ASA protein (SEQ ID NO: 1). In some embodiments, a recombinant ASA protein purified by embodiments of the present invention has an amino acid sequence at least 50%, 55%, 60%, 65%, 70%, 75%, 80%, 85%, 90%, 91%, 92%, 93%, 94%, 95%, 96%, 97%, 98%, 99% or more identical to SEQ ID NO: 1. In some embodiments, a recombinant ASA protein purified by embodiments of the present invention contains a fragment or a portion of mature human ASA protein.

Alternatively, a recombinant ASA protein purified by embodiments of the present invention is full-length ASA protein. In some embodiments, a recombinant ASA protein may be a homologue or an analogue of full-length human ASA protein. For example, a homologue or an analogue of full-length human ASA protein may be a modified full-length human ASA protein containing one or more amino acid substitutions, deletions, and/or insertions as compared to a wild-type or naturally-occurring full-length ASA protein (e.g., SEQ ID NO:2), while retaining substantial ASA protein activity. Thus, in some embodiments, a recombinant ASA protein purified by embodiments of the present invention is substantially homologous to full-length human ASA protein (SEQ ID NO:2). In some embodiments, a recombinant ASA protein purified by embodiments of the present invention has an amino acid sequence at least 50%, 55%, 60%, 65%, 70%, 75%, 80%, 85%, 90%, 91%, 92%, 93%, 94%, 95%, 96%, 97%, 98%, 99% or more homologous to SEQ ID NO:2. In some embodiments, a recombinant ASA protein purified by embodiments of the present invention is substantially identical to SEQ ID NO:2. In some embodiments, a recombinant ASA protein purified by embodiments of the present invention has an amino acid sequence at least 50%, 55%, 60%, 65%, 70%, 75%, 80%, 85%, 90%, 91%, 92%, 93%, 94%, 95%, 96%, 97%, 98%, 99% or more identical to SEQ ID NO:2. In some embodiments, a recombinant ASA protein purified by embodiments of the present invention contains a fragment or a portion of full-length human ASA protein. As used herein, a full-length ASA protein typically contains signal peptide sequence.

Homologues or analogues of human ASA proteins can be prepared according to methods for altering polypeptide sequence known to one of ordinary skill in the art such as are found in references that compile such methods. In some embodiments, conservative substitutions of amino acids include substitutions made among amino acids within the following groups: (a) M, I, L, V; (b) F, Y, W; (c) K, R, H; (d) A, G; (e) S, T; (f) Q, N; and (g) E, D. In some embodiments, a "conservative amino acid substitution" refers to an amino acid substitution that does not alter the relative charge or size characteristics of the protein in which the amino acid substitution is made.

In some embodiments, recombinant ASA proteins may contain a moiety that binds to a receptor on the surface of target cells to facilitate cellular uptake and/or lysosomal targeting. For example, such a receptor may be the cation-independent mannose-6-phosphate receptor (CI-MPR) which binds the mannose-6-phosphate (M6P) residues. In addition, the CI-MPR also binds other proteins including IGF-II. In some embodiments, a recombinant ASA protein contains M6P residues on the surface of the protein. In particular, a recombinant ASA protein may contain bis-phosphorylated oligosaccharides which have higher binding affinity to the CI-MPR. In some embodiments, a suitable enzyme contains up to about an average of about at least 20% bis-phosphorylated oligosaccharides per enzyme. In other embodiments, a suitable enzyme may contain about 10%, 15%, 18%, 20%, 25%, 30%, 35%, 40%, 45%, 50%, 55%, 60% bis-phosphorylated oligosaccharides per enzyme.

In some embodiments, recombinant ASA enzymes may be fused to a lysosomal targeting moiety that is capable of binding to a receptor on the surface of target cells. A suitable lysosomal targeting moiety can be IGF-I, IGF-II, RAP, p97, and variants, homologues or fragments thereof (e.g., including those peptide having a sequence at least 70%, 75%, 80%, 85%, 90%, or 95% identical to a wild-type mature human IGF-I, IGF-II, RAP, p97 peptide sequence). The lysosomal targeting moiety may be conjugated or fused to an ASA protein or enzyme at the N-terminus, C-terminus or internally.

**Purification of Recombinant Arylsulfatase A**

Embodiments of the invention include purification processes for the production of Arylsulfatase A ("ASA"), particularly recombinant human ASA ("rhASA"), drug substances.

A variety of techniques, in whole or in part, optionally with modifications as described herein, may be used to produce purified ASA drug substance.

For example, an exemplary purification process comprises one or more of the following steps (e.g., 1, 2, 3, 4, 5, 6, or all 7 of these steps):

• Thawing and pooling of rhASA unpurified bulk (UPB).

• Capture and filtration of UPB via ultrafiltration and diafiltration ("UFDF" or "Capture UFDF").

• Following filtration, the captured material may be viral inactivated before chromatographic purification.

• A process comprising the successive use of four chromatographic columns: an anion exchange column (e.g., a Fractogel TMAE HiCap resin column), a ceramic hydroxyapatite Type I (HA) column, a hydrophobic interaction column (HIC; e.g., a Phenyl Sepharose FF column), and a cation exchange (e.g., SP) column.

- Pooling of the SP eluate and pH adjustment of the pooled eluate (e.g., adjustment to a pH of about 5.5 to about 6.5 (e.g., about 6.0).

- The resultant pH-adjusted cation exchange eluate is then viral filtered followed by a single concentration and diafiltration step to achieve a final target protein concentration.

- Addition of polysorbate 20 (P20) to the eluate (e.g., prior to frozen storage).

As used herein, a "contaminant" is a material that is different from the desired polypeptide product, e.g., arylsulfatase A (ASA). The contaminant may be a variant of the desired polypeptide (e.g., a deamidated variant or an amino-aspartate variant of the desired polypeptide) or another molecule, for example, polypeptide, nucleic acid, and endotoxin.

As used herein, by "purifying" a polypeptide from a composition or sample comprising the polypeptide and one or more contaminants is meant increasing the degree of purity of the polypeptide in the composition or sample by removing (completely or partially) at least one contaminant from the composition or sample.

A "purification step" may be part of an overall purification process resulting in a composition comprising at least about 20%, 30%, 40%, 50%, 60%, 70%, 80%, 90%, 95%, 99%, 99.5%, 99.6%, 99.7%, 99.8%, or 99.9% by weight of the polypeptide of interest, based on total weight of the composition.

The purity of arylsulfatase A can be measured by, e.g., one or more of: host cell protein (HCP) Western blot, SDS-PAGE Coomassie staining, SDS-PAGE silver staining, reverse phase HPLC, and size exclusion HPLC.

The arylsulfatase A used in, e.g., the compositions and methods described herein, may also be described by a characteristic glycan map (e.g., any of the exemplary glycan maps described herein).

In some embodiments, the specific activity of the purified arylsulfatase A is at least about 50 U/mg, 60 U/mg, 70 U/mg, 80 U/mg, 90 U/mg, 100 U/mg, 110 U/mg, 120 U/mg, 130 U/mg, 140 U/mg, e.g., as determined by a method described herein. In some embodiments, the purified recombinant ASA has a specific activity ranging from about 50-200 U/mg (e.g., about 50-190 U/mg, 50-180 U/mg, 50-170 U/mg, 50-160 U/mg, 50-150 U/mg, 50-140 U/mg, 50-130 U/mg, 50-120 U/mg, 50-110 U/mg, 50-100 U/mg, 60-140 U/mg, 60-130 U/mg, 60-120 U/mg, 60-110 U/mg, 60-100 U/mg, 70-140 U/mg, 70-130 U/mg, 70-120 U/mg, 70-110 U/mg, 70-100 U/mg, 80-140 U/mg, 80-130 U/mg, 80-120 U/mg, 80-110 U/mg, 80-100 U/mg, 90-140 U/mg, 90-130 U/mg, 90-120 U/mg, 90-110 U/mg, 90-100 U/mg, 100-140 U/mg, 100-130 U/mg, 100-120 U/mg, 100-110 U/mg, 110-140 U/mg, 110-130 U/mg, 110-120 U/mg, 120-140 U/mg, 120-130 U/mg, or 130-140 U/mg), e.g., as determined by a method described herein.

A starting material for the purification process is any impure preparation. For example, an impure preparation may be unprocessed cell culture medium containing recombinant ASA protein secreted from the cells (e.g., mammalian cells) producing ASA protein or raw cell lysates containing ASA protein. In some embodiments, an impure preparation may be partially processed cell medium or cell lysates. For example, cell medium or cell lysates can be concentrated, diluted, treated with viral inactivation, viral processing or viral removal. In some embodiments, viral removal may utilize nanofiltration and/or chromatographic techniques, among others. In some embodiments, viral inactivation may utilize solvent inactivation, detergent inactivation, pasteurization, acidic pH inactivation, and/or ultraviolet inactivation, among others. Cell medium or cell lysates may also be treated with protease, DNases, and/or RNases to reduce the level of host cell protein and/or nucleic acids (e.g., DNA or RNA). In some embodiments, unprocessed or partially processed biological materials (e.g., cell medium or cell lysate) may be frozen and stored at a desired temperature (e.g., 2-8 °C, -4 °C, -25 °C, -75 °C) for a period time and then thawed for purification. As used herein, an impure preparation is also referred to as starting material or loading material.

The purification methods described herein can include, but not limited to, one or more of the following steps: depth filtration, viral inactivation, ion exchange chromatography (e.g., anion exchange chromatography, and/or cation exchange chromatography), mixed mode chromatography, hydrophobic interaction chromatography, ultrafiltration/diafiltration, and viral removal filtration. In some embodiments, the purification methods described herein further include affinity chromatography.

In the chromatography steps, the appropriate volume of resin used when packed into a chromatography column is reflected by the dimensions of the column, i.e., the diameter of the column and the height of the resin, and varies depending on e.g., the amount of protein in the applied solution and the binding capacity of the resin used. However, it is within the scope of the present disclosure to increase the scale of the production process as well as the purification process in order to obtain production and purification of ASA on an industrial scale. Accordingly parameters such as column size, diameter, and flow rate can be increased in order to comply with the speed and efficiency of such large-scale production. In some embodiments, the diameter of the column ranges from about 50-100 mm, the volume ranges from about 100-300 ml, and flow rate is between about 40-400 cm/hour (e.g., between about 100 cm/hour and 150 cm/hour) or about 5 to 100 ml.

***Ultrafiltration - Capture***

In some embodiments of the invention, the purification methods disclosed herein include one or more steps of upstream ultrafiltration to capture ASA (e.g., human recombinant ASA) produced from a perfusion bioreactor. Ultrafiltration, as used herein, refers to membrane filtration with filter pore sizes on the magnitude of 0.001 and 0.1 $\mu$m, which may be used for concentrating and desalting dissolved molecules (proteins, peptides, nucleic acids, carbohydrates, and other biomolecules), exchanging buffers, and gross fractionation. Methods of ultrafiltration for use in embodiments of the invention include tangential flow ultrafiltration or crossflow filtration.

Tangential flow filtration and ultrafiltration, as used herein, refers to arrangements where the feed stream passes parallel to the membrane face as one portion passes through the membrane (permeate) while the remainder (retentate) is recirculated back to the feed reservoir. In some embodiments, pore size of tangential flow ultrafiltration filters is chosen to allow recombinant ASA to permeate through the filter. In other embodiments, pore size is chosen so as to retain substantially all ASA in the feed passing over the filter. As noted elsewhere, ASA exists as a 480 kDa octamer in acidic conditions (pH ≤ about 5.0), but dissociates into dimers at neutral pH levels. Thus, the pH of the feed may be adjusted in combination with selection of appropriate pore size to either retain ASA on the filter membrane or allow it to pass through as a permeate.

Pore size may be selected with molecular weight cutoffs of at least 10 kDa, at least 20 kDa, at least 30 kDa, at least 40 kDa, at least 50 kDa, at least 60 kDa, at least 70 kDa, at least 80 kDa, at least 90 kDa, at least 100 kDa, at least 300 kDa, at least 400 kDa or at least 500 kDa. For example, a filter with a pore size of at least 10 kDa will retain in the feed a majority of proteins with molecular weights of approximately 11 kDa or higher. As another example, a filter of a pore size of at least 400 kDa will retain a majority of proteins with molecular weights higher than 400 kDa. In some embodiments, the feed retention rate is at least 75%, at least 80%, at least 85%, at least 90%, at least 95% or higher. Likewise, pore size may be selected for isolation of permeates of particular size. For example, a filter with a pore size of at least 500 kDa will allow a majority of proteins with molecular weights less than 500 kDa to permeate through. In some embodiments, the permeation rate is at least 75%, at least 80%, at least 85%, at least 90%, at least 95% or higher.

Filtration area or capacity may also be optimized for use in the processes disclosed herein. Considerations impacting selection of filtration area include robustness, cost, feed flow rate (i.e., crossflow velocity), transmembrane pressure, permeate flux rate, plant fit and throughput. In some embodiments, the permeate flux is about 50 -100 liter per meter$^2$ per hour ("LMH"). In some embodiments, the feed flow is about 250 - 600 LMH, inclusive. In some embodiments, the feed flow is about 250 - 350 LMH, inclusive. In some embodiments, the feed flow is about 175-245 LMH, inclusive. In some embodiments, the feed flow is about 170 - 230 LMH, inclusive. In some embodiments, the feed flow is about 120 - 160 LMH, inclusive. In some embodiments, the feed flow is about 15 - 30 LMH, inclusive. In some embodiments, the fee flow is about 11-21 LMH, inclusive. In some embodiments, the filtration area is about 0.02 m$^2$, about 0.14 m$^2$, about 0.7 or about 3.5 m$^2$. In particular embodiments, the transmembrane pressure is about 55-60 psi, inclusive. In some embodiments, the transmembrane pressure is about 15 - 25 psi, inclusive. In some embodiments, the transmembrane pressure is about 10 - 20 psi, inclusive. In some embodiments, the transmembrane pressure is about 5-15 psi.

Ultrafiltration filters for use in embodiments of the invention may comprise membrane materials known to those of skill in the art, including but not limited to polyethersulfone and stabilized cellulose.

One exemplary filter cassette for use in embodiments of the invention is the Sartorius ECO®. Another exemplary filter cassette for use in embodiments of the invention is the Sartorius HYDROSART® 30 kD Standard membrane. A still further exemplary filter cassette for use in embodiments of the invention is a Cuno depth filter ZB. In some embodiments of the invention, a Cuno Zeta Plus depth filter is used.

***Depth filtration***

The purification methods described herein can include one or more steps of depth filtration. Depth filters are the variety of filters that use a porous filtration medium to retain particles throughout the medium, rather than just on the surface of the medium. They contain filtration media having a graded density, which allows larger particles to be trapped near the surface of the filter while smaller particles penetrate the larger open areas at the surface of the filter, only to be trapped in the smaller openings nearer to the center of the filter. Although certain embodiments employ depth filtration steps only during the upstream recovery phase (i.e., before subsequent chromatographic purification steps), other embodiments employ depth filters during one or more additional phases of purification. In some embodiments of the invention, a Cuno Zeta Plus depth filter is used.

Depth filtration may optionally be followed by a 0.45 micron (± 2 $\mu$m) filtration to remove particulates and reduce bioburden in preparation for downstream processing.

*Viral Inactivation*

The purification methods described herein can include one or more steps of viral inactivation. In some embodiments, the viral inactivation comprises a solvent and/or a detergent. The solvent or detergent can include, for example, polysorbate 20, polysorbate 80, Tri-n-Butyl-Phosphate (TnBP), or a combination thereof. Viral inactivation may involve 3-24 hours of incubation in the solvent or detergent. In another embodiment, the viral inactivation comprises virus filtration, e.g., by using a Planova™ filter.

It is understood that these methods are intended to give rise to a preparation of an enzyme, which is substantially free of infectious viruses and which can be denoted a "virus-safe product". In addition, it is contemplated that the various methods can be used independently or in combination.

Virus-inactivation can be accomplished by the addition of one or more "virus-inactivating agents" to a solution comprising the enzyme. In some embodiments, a virus-inactivating step is performed prior to chromatographic purification steps (i.e., before loading the impure preparation onto the first chromatography column) in order to assure that the agent is not present in the final product in any amounts or concentrations that will compromise the safety of the product when used as a pharmaceutical or when the product is used for the preparation of a pharmaceutical; other embodiments employ depth filters during one or more additional phases of purification. For example, in some embodiments, an inventive method according to the invention further includes a step of viral removal after the last chromatography column.

The term "virus-inactivating agent" is intended to denote an agent (e.g., detergent) or a method, which can be used in order to inactivate lipid-enveloped viruses as well as non-lipid enveloped viruses. The term "virus-inactivating agent" is to be understood as encompassing both a combination of such agents and/or methods, whenever that is appropriate, as well as only one type of such agent or method.

Typical virus-inactivating agents are detergents and/or solvents, most typically detergent-solvent mixtures. It is to be understood that the virus inactivating agent is optionally a mixture of one or more detergents with one or more solvents. A wide variety of detergents and solvents can be used for virus inactivation. The detergent may be selected from the group consisting of non-ionic and ionic detergents and is selected to be substantially non-denaturing. Typically, a non-ionic detergent is used as it facilitates the subsequent elimination of the detergent from the rASA preparation in the subsequent purification steps. Suitable detergents are described, e.g. by Shanbrom et al., in US Patent 4,314,997, and US Patent 4,315,919. Typical detergents are those sold under the trademarks Triton X-100 and Tween 20 or Tween 80. Preferred solvents for use in virus-inactivating agents are di- or tri-alkylphosphates as described e.g. by Neurath and Horowitz in US Patent 4,764,369. A typical solvent is tri(n-butyl) phosphate (TnBP). An especially preferred virus-inactivating agent for the practice of the present invention is Tween 80, but, alternatively, other agents or combinations of agents can be used. The typical agent added in such a volume that the concentration of Tween-80 in the ASA-containing solution is within the range of about 0.5-4.0% by weight, preferably at a concentration of about 1% by weight. TnBP can then be added to a final concentration of 0.3% calculated based on the new volume of the sample containing ASA.

The virus-inactivation step is conducted under conditions inactivating enveloped viruses resulting in a substantially virus-safe rhASA-containing solution. In general, such conditions include a temperature of 4-37°C, such as 19-28°C, 23-27°C, typically about 25°C, and an incubation time found to be effective by validation studies. Generally, an incubation time of 1-24 hours is sufficient, preferably 10-18 hours, such as about 14 hours, to ensure sufficient virus inactivation. However, the appropriate conditions (temperature and incubation times) depend on the virus-inactivating agent employed, pH, and the protein concentration and lipid content of the solution.

It is contemplated that other methods for removal of or inactivating virus can also be employed to produce a virus-safe product, such as the addition of methylene blue with subsequent inactivation by radiation with ultraviolet light.

The purification methods described herein can include one or more steps of viral removal filtration. Typically, virus filtration is performed after purification of the enzyme by one or more steps of chromatography. In some embodiments, the virus filtration step is performed by passage of the ASA containing solution which is a result of a purification step through a sterile filter and subsequently passage of the sterile filtered solution through a nanofilter. By "sterile filter" is meant a filter, which will substantially remove all micro-organisms capable of propagating and/ or causing infection. Whereas it is typical that the filter has a pore size of about 0.1 micron, the pore size could range between about 0.05 and 0.3 micron. It may be feasible to replace or combine virus filtration of the sample as performed in the purification process with contacting the sample with a detergent.

Some embodiments of the invention include at least two steps of viral inactivation and/or filtration. For example, viral inactivation before column chromatography may be combined viral removal after all of the chromatographic steps have been completed. Post-chromatographic viral removal can be done before or after one or more steps of ultrafiltration/diafiltration (UFDF) (e.g., tangential flow ultrafiltration). In a specific example, eluate is obtained from a final step of chromatographic purification (e.g., cation exchange (SP) chromatography), and the pH of the eluate pool is adjusted about 5.5, about 6.0, about 6.5 or about 7.0, followed by viral filtration. Thus, in certain embodiments, a single step of UFDF is preceded by viral filtration (e.g., using a Planova™ filter). In other example, eluate is obtained from a final step of chromatographic purification (e.g., cation exchange (SP) chromatography), is subjected to a first step of UFDF without pH adjustment, followed by viral filtration and a second step of UFDF.

In certain embodiments, pH-adjusted cation exchange eluate pool is viral filtered on a Planova 20N filter. In some embodiments, the yield relative to input following viral filtration of pH-adjusted cation exchange eluate is between about 90 - 100%; i.e., about 90%, about 95%, about 96%, about 97%, about 98%, about 99% or more, as assessed by A280 absorbance. Thus, in some embodiments, essentially no recombinant ASA is lost during viral filtration. The yield for viral filtration is significant as it verifies that pH adjustment to about 6.0 allows octamers of ASA (which are about 20 nm in diameter) to dissociate into dimeric form. Thus, the pore size of a viral filter may be selected to ensure that only the dimeric form is filtered (i.e., that the octameric form may be retained by the filter, or cause viral filter plugging). For examples, a viral filter with a pore size of 20 nm will retain the octameric form of ASA, but not the dimeric form.

### Affinity chromatography

The purification methods described herein can include one or more steps of affinity chromatography (e.g., immuno-affinity chromatography, immobilized metal ion affinity chromatography, and/or immobilized ligand affinity chromatography).

Briefly, affinity chromatography is a chromatographic technique which relies on highly specific interactions, such as, for example, between a receptor and ligand, an antigen and antibody, or an enzyme and substrate. As will be known by the person skilled in the art, selective molecules employed in an affinity chromatography step in the purification methods described herein may be based on various properties (e.g., three dimensional structure, glycosylation, etc.) of recombinantly produced ASA that can be exploited by the selective molecule. Exemplary selective molecules (or capture reagents) that can be utilized in an affinity chromatography step include protein A, protein G, an antibody, a metal ion (e.g., nickel), specific substrate, ligand or antigen. In some embodiments, a suitable selective molecule for an affinity chromatography step of the present invention utilizes an anti-Arylsulfatase A antibody (e.g., an anti-human Arylsulfatase A antibody). Suitable anti-Arylsulfatase A antibodies may be obtained commercially or through immunization of non-human animals (e.g., a mouse, rat, rabbit, chicken, goat, sheep, horse or other suitable animal for producing antibodies against a human protein).

Generally, a molecule of interest (e.g., recombinant ASA) is trapped on a solid or stationary phase or medium through interaction with a selective molecule, while other, undesired molecules are not trapped as they are not bound by the selective molecule(s). The solid medium may then be removed from the mixture, optionally washed, and the molecule of interest released from the entrapment by elution. In some embodiments, affinity columns may be eluted by changing the ionic strength through a gradient. For example, salt concentrations, pH, pI, and ionic strength may be used to separate or to form the gradient to separate.

In some embodiments, a recombinant ASA protein may be produced with a tag in order to facilitate purification by affinity chromatography. As will be known by the person skilled in the art, protein tags may include, for example, glutathione-S-transferase (GST), hexahistidine (His), maltose-binding protein (MBP), among others. In some embodiments, lectins are used in affinity chromatography to separate components within the sample. For example, certain lectins specifically bind a particular carbohydrate molecule and can be used to separate glycoproteins from non-glycosylated proteins, or one glycoform from another glycoform.

### Ion exchange chromatography

The purification methods described herein can include one or more steps of ion exchange chromatography (e.g., anion exchange chromatography and/or cation exchange chromatography).

As will be known by the person skilled in the art, ion exchangers (e.g., anion exchangers and/or cation exchangers) may be based on various materials with respect to the matrix as well as to the attached charged groups. For example, the following matrices may be used, in which the materials mentioned may be more or less crosslinked: agarose based (such as SEPHAROSE™ CL-6B, SEPHAROSE™ Fast Flow and SEPHAROSE™ High Performance), cellulose based (such as DEAE SEPHACEL®), dextran based (such as SEPHADEX®), silica based and synthetic polymer based.

The ion exchange resin can be prepared according to known methods. Typically, an equilibration buffer, which allows the resin to bind its counter ions, can be passed through the ion exchange resin prior to loading the sample or composition comprising the polypeptide and one or more contaminants onto the resin. Conveniently, the equilibration buffer can be the same as the loading buffer, but this is not required.

In an optional embodiment of the invention, the ion exchange resin can be regenerated with a regeneration buffer after elution of the polypeptide, such that the column can be re-used. Generally, the salt concentration and/or pH of the regeneration buffer can be such that substantially all contaminants and the polypeptide of interest are eluted from the ion exchange resin. Generally, the regeneration buffer has a very high salt concentration for eluting contaminants and polypeptide from the ion exchange resin.

*Anion Exchange Chromatography*

Embodiments of the invention include, for example, providing a sample of arylsulfatase A (e.g., recombinant arylsulfatase A), and subjecting the sample to anion exchange chromatography, e.g., anion exchange chromatography described herein. For the anion exchange resin, the charged groups which are covalently attached to the matrix can be, for example, diethylaminoethyl (DEAE), quaternary aminoethyl (QAE), and/or quaternary ammonium (Q). In some embodiments, the anion exchange resin employed is a Q Sepharose column. The anion exchange chromatography can be performed using, e.g., Q SEPHAROSE™ Fast Flow, Q SEPHAROSE™ High Performance, Q SEPHAROSE™ XL, CAPTO™ Q, DEAE, TOYOPEARL GIGACAP® Q, FRACTOGEL® TMAE (trimethylaminoethyl, a quarternary ammonia resin), ESHMUNO™ Q, NUVIA™ Q, or UNOSPHERE™ Q. Other anion exchangers can be used within the scope of the invention, including but not limited to, but are not limited to, quaternary amine resins or "Q-resins" (e.g., CAPTOTM-Q, Q-SEPHAROSE®, QAE SEPHADEX®); diethylaminoethane (DEAE) resins (e.g., DEAE-TRISACRYL®, DEAE SEPHAROSE®, benzoylated naphthoylated DEAE, diethylaminoethyl SEPHACEL®); AMBERJET® resins; AMBERLYST® resins; AMBERLITE® resins (e.g., AMBERLITE® IRA-67, AMBERLITE® strongly basic, AMBERLITE® weakly basic), cholestyramine resin, ProPac® resins (e.g., PROPAC® SAX-10, PROPAC® WAX-10, PROPAC® WCX-10); TSK-GEL® resins (e.g., TSKgel DEAE-NPR; TSKgel DEAE-5PW); and ACCLAIM® resins.

In embodiments, the anion exchange chromatography is performed using FRACTOGEL® TMAE (trimethylaminoethyl, a quarternary ammonia resin).

In some embodiments, subjecting the sample of arylsulfatase A to the anion exchange chromatography is performed at a temperature about 23°C or less, about 18°C or less, or about 16°C or less, e.g., about 23°C, about 20°C, about 18°C, or about 16°C.

Typical mobile phases for anionic exchange chromatography include relatively polar solutions, such as water, acetonitrile, organic alcohols such as methanol, ethanol, and isopropanol, or solutions containing 2-(N-morpholino)-ethanesulfonic acid (MES). Thus, in certain embodiments, the mobile phase includes about 0%, 1%, 2%, 4%, 6%, 8%, 10%, 12%, 14%, 16%, 18%, 20%, 25%, 30%, 35%, 40%, 45%, 50%, 55%, 60%, 65%, 70%, 75%, 80%, 85%, 90%, 95%, or about 100% polar solution. In certain embodiments, the mobile phase comprises between about 1% to about 100%, about 5% to about 95%, about 10% to about 90%, about 20% to about 80%, about 30% to about 70%, or about 40% to about 60% polar solution at any given time during the course of the separation.

In certain embodiments, rhASA is loaded at a binding capacity about 23 AU/L resin or less, e.g., about 19 AU/L resin or less, about 15 AU/L resin or less, or about 12 AU/L resin or less, e.g., between about 12 AU/L resin and about 15 AU/L resin, or between about 15 AU/L resin and about 19 AU/L resin. In some embodiments, the sample of arylsulfatase A is loaded onto the anion exchange chromatography column at a binding capacity at least about 4.5 g/L resin (e.g., at least about 5 g/L resin, 6 g/L resin, 7 g/L resin, 8 g/L resin, 9 g/L resin, 10 g/L resin, 11 g/L resin, 12 g/L resin, 13 g/L resin, 14 g/L resin, or 15 g/L resin). In some embodiments, the sample of arylsulfatase A is loaded onto the anion exchange chromatography column at a binding capacity ranging between about 4.5-20 g/L resin (e.g., ranging between about 5-20 g/L resin; 5-19 g/L resin, 5-18 g/L resin, 5-17 g/L resin, 5-16 g/L resin, 5-15 g/L resin, 7.5-20 g/L resin, 7.5-19 g/L resin, 7.5-18 g/L resin, 7.5-17 g/L resin, 7.5-16 g/L resin, 7.5-15 g/L resin, 10-20 g/L resin, 10-19 g/L resin, 10-18 g/L resin, 10-17 g/L resin, 10-16 g/L resin, or 10-15 g/L resin).

The aqueous solution comprising the ASA and contaminant(s) can be loaded onto the anionic resin as a mobile phase using a loading buffer that has a salt concentration and/or a pH such that the polypeptide and the contaminant bind to the anion exchange resin. The resin can then be washed with one or more column volumes of loading buffer followed by one or more column volumes of wash buffer wherein the salt concentration is increased. Finally, the ASA can be eluted by an elution buffer of increasing salt concentration. Optionally, elution of the enzyme may also be mediated by gradually or stepwise decreasing the pH. The fractions containing ASA activity can be collected and combined for further purification.

In some embodiments, loading the sample of arylsulfatase A onto the anion exchange chromatography column is performed with a loading buffer. In one embodiment, the loading buffer does not contain sodium chloride. In another embodiment, the loading buffer contains sodium chloride. For example, the sodium chloride concentration of the loading buffer is from about 1 mM to about 25 mM, e.g., from about 1 mM to about 10 mM, from about 1 mM to about 5 mM, or from about 5 mM to about 10 mM. In some embodiments, salt concentration in the mobile phase is a gradient (e.g., linear or non-linear gradient). In some embodiments, salt concentration in the mobile phase is constant. In some embodiments, salt concentration in the mobile phase may increase or decrease stepwise. In some embodiments, loading the sample of arylsulfatase A onto the anion exchange chromatography column is performed at a pH from about 5 to about 9, e.g., from about 6 to about 8, e.g., about 7.

In some embodiments, washing the anion exchange chromatography column is performed with one or more washing buffers. For example, washing the anion exchange column can include two or more (e.g., a first and a second) washing steps, each using a different washing buffer. In one embodiment, the washing buffer does not contain sodium chloride. In another embodiment, the washing buffer contains sodium chloride. For example, the sodium chloride concentration of the washing buffer is from about 50 mM to about 200 mM, e.g., from about 50 mM to about 150 mM, from about 100 mM to about 200 mM, or from about 100 mM to about 150 mM, e.g., about 80 mM, about 100 mM, about 120 mM, or about 140 mM. In some embodiments, washing the anion exchange chromatography column is performed at a pH from about 5 to about 9, e.g., from about 6 to about 8, e.g., about 7.

In one embodiment, the elution buffer contains sodium phosphate. For example, the sodium phosphate concentration of the elution buffer is from about 20 mM to about 50 mM, e.g., from about 25 mM to about 45 mM, e.g., about 30 mM, about 35 mM, or about 40 mM. In another embodiment, the elution buffer does not contain sodium chloride. In yet another embodiment, the elution buffer contains sodium chloride. For example, the sodium chloride concentration of the elution buffer is from about 200 mM to about 300 mM, e.g., from about 240 mM to about 280 mM. In some embodiments, eluting the arylsulfatase A from the anion exchange chromatography column is performed at a pH from about 5 to about 9, e.g., from about 6 to about 8, e.g., about 7.

In some embodiments, eluting the arylsulfatase A from the anion exchange chromatography column includes one or more steps of elution peak collection. For example, the elution peak collection starts from about 50 mAU at the ascending side to about 50 mAU at the descending side, e.g., from about 100 mAU at the ascending side to about 50 mAU at the descending side, from about 200 mAU at the ascending side to about 50 mAU at the descending side, from about 50 mAU at the ascending side to about 100 mAU at the descending side, from about 50 mAU at the ascending side to about 200 mAU at the descending side, or from about 100 mAU at the ascending side to about 100 mAU at the descending side, e.g., as determined by spectrophotometry, e.g., at 280 nM.

It is apparent to the person of ordinary skill in the art that numerous different buffers may be used in the loading, washing, and elution steps. Typically, however, the column can be equilibrated with 1-10 column washes of a buffer comprising 0.05 M MES-Tris, pH 7.0. As of convenience the sample can be loaded in the buffer from the previous step of the purification process, or the sample can be loaded using a loading buffer. The column can be washed with 1-10 column volumes of the buffer used for equilibration, followed by a washing buffer comprising 0.02 MES-Tris, 0.12 M NaCl, pH 7.0. Alternatively, the column can be equilibrated, loaded, and washed with any other equilibration, loading, and washing buffers described herein for anion exchange chromatography. The sample can be eluted in a buffer comprising 0.02 MES-Tris, 0.26 M NaCl, pH 7.0. Alternatively, the sample can be eluted in any other elution buffer described herein for anion exchange chromatography.

The loading buffer, washing buffer, and elution buffer described herein can include one or more buffering agents. For example, the buffering agent can be TRIS, HEPES, MOPS, PIPES, SSC, MES, sodium phosphate, sodium acetate, or a combination thereof. The concentration of the buffering agent is between about 1 mM and about 500 mM, e.g., between about 10 mM and about 250 mM, between about 20 mM and about 100 mM, between about 1 mM and 5 mM, between about 5 mM and 10 mM, between about 10 mM and 50 mM, or between about 50 mM and about 100 mM, e.g., about 1 mM, about 5 mM, about 10 mM, about 20 mM, about 30 mM, about 40 mM, or about 50 mM.

Yield, activity and purity following anion exchange chromatography may vary. In some embodiments, the arylsulfatase A activity yield is at least about 75%, e.g., at least about 85%, e.g., between about 85% and about 99%, or between about 90% and about 99%. In some embodiments, the protein yield (AU or Absorbance Units) is from about 10% to 50%, e.g., from about 20% to about 35%, or from about 25% to about 30%, e.g., as determined by spectrophotometry, e.g., at 280 nm. In some embodiments (e.g., those using a TMAE column as described below), the elution pool protein activity yield (AU or Absorbance Units) is from about 70% to 400%, e.g., from about 80% to about 390%, or from about 90% to about 350%, or from about 100% to 150%, greater than at least 95%, e.g., as determined by spectrophotometry, e.g., at 280 nm. In some embodiments (e.g., those using a TMAE column as described below), the host cell protein (HCP) log reduction value (LRV) is between about 0.5 and about 1.1, e.g., between about 0.6 and 0.9, or between about 0.7 and 0.8. In some embodiments (e.g., those using a TMAE column as described below), the purity is at least 75%, e.g., at least 80%, at least 85%, at least 90% or higher, as determined by, for example, capillary electrophoresis-SDS PAGE. In preferred embodiments, the activity yield, HCP LRV and purity (as determined by capillary electrophoresis-SDS PAGE) following anion exchange chromatography are at least about 90%, at least about 0.6 and at least about 80%, respectively.

In preferred embodiments of the invention, an anionic exchange column with a high loading capacity is used. In certain embodiments of the invention, the column is characterized by a loading range between about 3-20 g/L (i.e., about 5-15 g/L, about 10-15 g/L, about 10-20 g/L). In some embodiments, the loading capacity is significantly greater than 4.3 g/L (e.g., is or greater than about 10 g/L, 12.5 g/L, 15 g/L, 17.5 g/L, or 20 g/L). In certain embodiments, the binding capacity of the resin is between about 75-100 AU/L (e.g. about 75 AU/L, about 80 AU/L, about 85 AU/L, about 90 AU/L, about 95 AU/L). In certain embodiments, the loading capacity is greater than about 80 AU/L. In some embodiments, the high load capacity column is a TMAE column. In particular embodiments, the column is selected from the group consisting of a Fractogel® TMAE column, a Nuvia Q column, a Q Sepharose Fast Flow column, a Capto Q column, a Q Sepharose XL column, a Eshmuno Q column, a UNOsphere Q column, or a GigaCap Q column.

In particular embodiments of the invention, a TMAE column is pre-equilibrated with a buffer comprising about 20 mM MES-Tris and 1000 mM NaCl at a pH of 7.0. In certain embodiments, the column is equilibrated with a buffer comprising 50 mM MES-Tris at a pH of 7.0. In some embodiments, the load flow rate of the TMAE column is about 75-125 cm/hr (i.e., about 75-115 cm/hr, about 75-110 cm/hr, about 75-105 cm/hr, about 75-100 cm/hr, about 85-115 cm/hr, about 85-110 cm/hr, about 85-105 cm/hr, about 85-100 cm/hr, about 95-115 cm/hr, about 95-110 cm/hr, about 95-105 cm/hr, about 95-100 cm/hr, about 100-120 cm/hr, about 100-115 cm/hr, about 100-110 cm/hr, about 100 cm/hr). Loading conditions may be optimized and assessed by A280 absorbance as described herein.

In particular embodiments utilizing a TMAE column (e.g., a Fractogel TMAE column), very little product is lost in the flow through during loading, even at loading capacities greater than 15 g/L. The capability of increasing loading capacity while minimizing flow-through loss is a significant improvement in purification methodology. In particular embodiments of the invention, the amount of flow-through product loss is less than 30% of the load (e.g. less than about 25%, less than about 20%, less than about 15%, less than about 10%, or less than about 5%).

After loading, in some embodiments, a TMAE column is washed at least once. In particular embodiments, the column is washed twice. A first or second wash buffer may comprise an optimized level of sodium chloride. In some embodiments, the amount of sodium chloride is a first or second wash buffer is between about 50-150 mM (e.g. about 50-140 mM, about 50-130 mM, about 50-120 mM, about 50-110 mM, about 50-100 mM, about 50-90 mM, about 50-80 mM, about 80-150 mM, about 80-140 mM, about 80-130 mM, about 80-120 mM, about 80-110 mM, about 80-100 mM, about 80-90 mM, about 80 mM, or about 120 mM). In some embodiments, a first wash buffer comprises 50 mM MES-Tris at pH 7.0. In some embodiments, a second wash buffer comprises, 20 mM MES-Tris, 100 mM NaCl at pH 7.0. Further optimization of wash conditions, particularly second wash conditions, is encompassed within embodiments of the present invention. For example, increasing the salt concentration of a second wash may improve host cell protein (HCP) log reduction values (LRV) and overall purity, but decrease both activity and A280 yield. As described herein, particular washing conditions must be balanced with the elution conditions described below in order to provide the optimal combination of purity, activity and yield.

In embodiments of the invention, recombinant ASA bound to a TMAE column is eluted with an elution buffer. In some embodiment, the amount of sodium chloride in the elution buffer is optimized. In particular embodiments, the amount of sodium chloride in the elution buffer is between about 150-300 mM (e.g. about 150-290 mM, about 150-280 mM, about 150-270 mM, about 150-260 mM, about 150-250 mM, about 150-240 mM, about 150-230 mM, about 150-220 mM, about 150-210 mM, about 170-290 mM, about 170-280 mM, about 170-270 mM, about 170-260 mM, about 170-250 mM, about 170-240 mM, about 170-230 mM, about 170-220 mM, about 170-210 mM about 180-290 mM about 180-280 mM, about 180-270 mM, about 180-260 mM, about 180-250 mM, about 180-240 mM, about 180-230 mM, about 180-220 mM, about 180-210 mM, about 180, about 220 or about 260). In a particular example, the elution buffer comprises 50 mM MES-Tris and 1M NaCl at a pH of 7.0. In some embodiments, the A280 yield following elution is greater than 60% of the load (e.g., about 60%, about 70%, about 80% or higher). Further optimization of elution conditions is encompassed within embodiments of the present invention. For example, increase elution salt concentration (i.e., conductivity) provides better yield but results in poorer purity and HCP removal. And as noted above, particular washing conditions must be balanced with the elution conditions in order to provide the optimal combination of purity, activity and yield.

### *Cation Exchange Chromatography*

In some embodiments, the method further includes subjecting the sample of arylsulfatase A to cation exchange chromatography, e.g., sulfopropyl (SP) cation exchange chromatography, e.g., as described herein. In some embodiments, the sample of arylsulfatase A is subjected to anion exchange chromatography prior to cation exchange chromatography. In a typical embodiment, the cation exchange chromatography comprises sulfopropyl (SP) cation exchange chromatography, but other cation chromatography membranes or resins can be used, for example, a MUSTANG™ S membrane, an S- SEPHAROSE™ resin, or a Blue SEPHAROSE™ resin. In some embodiments, the method further comprises concentrating and/or filtering the sample of arylsulfatase A, e.g., by ultrafiltration and/or diafiltration, e.g., by tangential flow ultrafiltration. The cation exchange chromatography can be performed at an optimized temperature, e.g., as described herein, to enhance target binding and/or decrease impurity binding. For example, the cation exchange chromatography can be performed at a temperature of about 23°C, 18°C, 16°C, or less.

In one embodiment, the cation exchange chromatography includes sulfopropyl (SP) cation exchange chromatography. In another embodiment, the cation exchange chromatography is a polishing step. The cation exchange chromatography (e.g., sulfopropyl (SP) cation exchange chromatography) can be performed using, e.g., one or more of: TOYOPEARL® SP-650, TOYOPEARL® SP-550, TSKGEL® SP-3PW, TSKGEL® SP-5PW, SP SEPHAROSE™ Fast Flow, SP SEPHAROSE™ High Performance, SP SEPHAROSE™ XL, SARTOBIND® S membrane, POROS® HS50, UNOSPHERE™ S, and MACROCAP™ S.

The aqueous solution comprising the arylsulfatase A and contaminant(s) can be loaded onto the cationic resin using a loading buffer that has a salt concentration and/or a pH such that the polypeptide and the contaminant bind to the cation exchange resin. The resin can then be washed with one or more column volumes of equilibration butter or loading buffer, and optionally followed by one or more column volumes of wash buffer wherein the salt concentration is increased. Finally, the arylsulfatase A can be eluted in an elution buffer. The fractions containing arylsulfatase A activity can be collected and combined for further purification.

In a typical embodiment, the NaCl concentration and/or pH of the loading buffer, washing buffer, and/or elution buffer, can be optimized, e.g., as described herein, to enhance target binding and/or decrease impurity binding. In some embodiments, the NaCl concentration in the loading buffer is about 20 mM, 15 mM, 10 mM, or less. In some embodiments, the loading buffer has a pH of about 4.5, 4.3, 4.0, or less. In some embodiments, the NaCl concentration in the washing buffer is about 20 mM, 15 mM, 10 mM, or less. In some embodiments, the NaCl concentration in the elution buffer is about 55 mM, 50 mM, 45 mM, 40 mM, or less.

In some embodiments, subjecting the sample of arylsulfatase A to a cation exchange chromatography includes: loading the sample of arylsulfatase A onto a cation chromatography column (e.g., a sulfopropyl (SP) cation exchange column), washing the cation exchange chromatography column, and eluting the arylsulfatase A from the column. In some embodiments, the columns can be equilibrated with more than 3, e.g., 5 to 10 column volumes of 0.01 M NaAc, 0.01 M NaCl, 0.03 M acetic acid, pH 4.2.

In some embodiments, the sample can be loaded in the buffer from the previous step of the purification process, or the sample can be loaded using a loading buffer. In one embodiment, the loading buffer contains sodium chloride. For example, the sodium chloride concentration of the loading buffer is from about 1 mM to about 25 mM, e.g., from about 5 mM to about 20 mM, e.g., about 5 mM, about 10 mM, about 15 mM, or about 20 mM. In another embodiment, the loading buffer contains sodium acetate. For example, the sodium acetate concentration of the loading buffer is from about 10 mM to about 100 mM, e.g., about 20 mM, about 40 mM, or about 60 mM. In some embodiments, loading the sample of arylsulfatase A onto the cation exchange chromatography column is performed at a pH from about 3.0 and about 6.0, e.g., from about 4.0 and about 5.0, e.g., about 4.0, about 4.3, or about 4.5. In some embodiments, the sample of arylsulfatase A is loaded onto the cation exchange chromatography column at a binding capacity about 15 AU/L resin or less, e.g., about 14 AU/L resin or less, or about 12 AU/L resin or less, e.g., between about 10 AU/L resin and about 14 AU/L resin, or between about 10 AU/L resin and about 12 AU/L resin.

In some embodiments, washing the cation exchange chromatography column is performed with one or more washing buffers. For example, washing the cation exchange column can include two or more (e.g., a first and a second) washing steps, each using a different washing buffer. The column can be washed with 1-10 column volumes of the buffer used for equilibration. Alternatively, the column can be equilibrated, loaded, and washed with any other equilibration, loading, and washing buffers described herein for cation exchange chromatography. In one embodiment, the washing buffer contains sodium chloride. For example, the sodium chloride concentration of the washing buffer is from about 1 mM to about 25 mM, e.g., from about 5 mM to about 20 mM, or from about 10 mM to about 15 mM, e.g., about 5 mM, about 10 mM, about 15 mM, or about 20 mM. In another embodiment, the washing buffer contains sodium acetate. For example, the sodium acetate concentration of the loading buffer is from about 10 mM to about 100 mM, e.g., about 20 mM, about 40 mM, or about 60 mM. In some embodiments, washing the cation exchange chromatography column is performed at a pH from about 3.0 and about 6.0, e.g., from about 4.0 and about 5.0, e.g., about 4.0, about 4.3, or about 4.5.

In some embodiments, eluting the arylsulfatase A from the cation exchange chromatography column is performed with an elution buffer. In one embodiment, the elution buffer contains sodium chloride. For example, the sodium chloride concentration of the elution buffer is from about 25 mM to about 75 mM, e.g., from about 45 mM to about 60 mM, e.g., about 45 mM, about 50 mM, about 55 mM, or about 55 mM. In some embodiments, eluting the arylsulfatase A from the cation exchange chromatography column is performed at a pH from about 3.0 and about 6.0, e.g., from about 4.0 and about 5.0, e.g., about 4.0, about 4.3, or about 4.5. Thus, as one particular example, the sample can be eluted in a buffer comprising 0.02 M NaAc, 0.05 M NaCl, pH 4.5. Alternatively, the sample can be eluted in any other elution buffer described herein for cation exchange chromatography.

In some embodiments, eluting the arylsulfatase A from the cation exchange chromatography column includes one or more steps of elution peak collection. For example, the elution peak collection starts from about 50 mAU at the ascending side to about 50 mAU at the descending side, e.g., from about 100 mAU at the ascending side to about 50 mAU at the descending side, from about 200 mAU at the ascending side to about 50 mAU at the descending side, from about 50 mAU at the ascending side to about 100 mAU at the descending side, from about 50 mAU at the ascending side to about 200 mAU at the descending side, or from about 100 mAU at the ascending side to about 100 mAU at the descending side, e.g., as determined by spectrophotometry, e.g., at 280 nM. Collected eluate peaks may be pooled.

The loading buffer, washing buffer, and elution buffer described herein can include one or more buffering agents. For example, the buffering agent can be TRIS, HEPES, MOPS, PIPES, SSC, MES, sodium phosphate, sodium acetate, or a combination thereof. The concentration of the buffering agent is between about 1 mM and about 500 mM, e.g., between about 10 mM and about 250 mM, between about 20 mM and about 100 mM, between about 1 mM and 5 mM, between about 5 mM and 10 mM, between about 10 mM and 50 mM, or between about 50 mM and about 100 mM, e.g., about 1 mM, about 5 mM, about 10 mM, about 20 mM, about 30 mM, about 40 mM, or about 50 mM.

In some embodiments, subjecting the sample of arylsulfatase A to the cation exchange chromatography is performed at a temperature about 23°C or less, about 18°C or less, or about 16°C or less, e.g., about 23°C, about 20°C, about 18°C, or about 16°C. In some embodiments, subjecting the sample of arylsulfatase A to the cation exchange chromatography is performed between about 23°C and about 16°C, e.g., at about 23°C, about 20°C, about 18°C, or about 16°C, and loading the sample of arylsulfatase A onto the cation exchange chromatography column is performed at a pH between about 4.5 and about 4.3, e.g., at about 4.5, about 4.4, or about 4.3. In some embodiments, subjecting the sample of arylsulfatase A to the cation exchange chromatography is performed at about 23°C and loading the sample of arylsulfatase A onto the cation exchange chromatography column is performed at a pH about 4.5. In some embodiments, subjecting the sample of arylsulfatase A to the cation exchange chromatography is performed at about 23°C and loading the sample of arylsulfatase A onto the cation exchange chromatography column is performed at a pH about 4.3. In some embodiments, subjecting the sample of arylsulfatase A to the cation exchange chromatography is performed at about 18°C and loading the sample of arylsulfatase A onto the cation exchange chromatography column is performed at a pH about 4.5. In some embodiments, subjecting the sample of arylsulfatase A to the cation exchange chromatography is performed at about 18°C and loading the sample of arylsulfatase A onto the cation exchange chromatography column is performed at a pH about 4.3.

The yield following cation exchange chromatography may vary. In some embodiments, the arylsulfatase A activity yield is at least about 75%, e.g., at least about 80%, e.g., between about 80% and about 105%. In some embodiments, the protein yield (AU or Absorbance Units) is from about 65% to 100%, e.g., from about 70% to about 95%, e.g., as determined by spectrophotometry, e.g., at 280 nm.

The purity and activity following cation exchange chromatography is greatly improved. In some embodiments, the host cell protein (HCP) log reduction value (LRV) is between about 1.0 and about 2.5, e.g., between about 1.5 and about 2.0 or between about 1.7 and about 1.9. The specific activity of the purified arylsulfatase A can be at least from about 50 U/mg to about 140 U/mg, e.g., at least about 70 U/mg, at least about 90 U/mg, at least about 100 U/mg, or at least about 120 U/mg, e.g., as determined by a method described herein. In some embodiments, the arylsulfatase A is purified to at least about 95%, at least about 98%, at least about 99%, at least about 99.5%, at least about 99.6%, at least about 99.7%, at least about 99.8%, or at least about 99.9%. The purity of arylsulfatase A can be measured by, e.g., one or more of: host cell protein (HCP) Western blot, SDS-PAGE Coomassie staining, SDS-PAGE silver staining, reverse phase HPLC, and size exclusion HPLC. In certain embodiments, decreasing the salt concentration of the loading buffer and lowering its pH enhances binding ASA to the cation exchange column but does not impact impurity binding. In other words, an optimal balance of salt concentration and pH, as set forth above, can increase yield after cation exchange chromatography without adversely affecting purity.

In some embodiments, the pH of a cation exchange eluate pool may be adjusted. In certain embodiments, the pH is adjusted immediately prior to viral filtration. Cation exchange eluate (e.g., SP eluate) may be pH adjusted to about 5.5, about 6.0 about 6.5 or about 7.0 using a pH adjustment buffer comprising 0.25M sodium phosphate, 1.33M sodium chloride, 0.34M sodium citrate, pH 7.0. In certain embodiments, the pH-adjusted SP eluate pool is viral filtered on a Planova 20N filter. In some embodiments, the yield relative to input following viral filtration of pH-adjusted cation exchange eluate is between about 90 - 100%; i.e., about 90%, about 95%, about 96%, about 97%, about 98%, about 99% or more, as assessed by A280 absorbance. The yield for viral filtration is significant as it verifies that pH adjustment to about 6.0 allows octamers of ASA (which are about 20 nm in diameter) to dissociate into dimeric form. Thus, the pore size of a viral filter may be selected to ensure that only the dimeric form is filtered (i.e., that the octameric form may be retained by the filter, or cause viral filter plugging). For examples, a viral filter with a pore size of 20 nm will retain the octameric form of ASA, but not the dimeric form.

*Mixed-Mode Chromatography*

The purification methods described herein can include one or more steps of mixed-mode chromatography. Mixed-mode chromatography is a type of chromatography in which several modes of separation are applied to resolve a mixture of different molecules, typically in liquid chromatography. For example, a mixed-mode separation can include combinational phases with ion-exchange and reversed phase characteristics at the same time. These stationary phases with more than one interaction type are available from several column manufacturers.

In one aspect, the disclosure features a method of purifying arylsulfatase A from a sample, where the method includes, for example, providing a sample of arylsulfatase A (e.g., recombinant arylsulfatase A), and subjecting the sample of arylsulfatase A to mixed mode chromatography, e.g., mixed mode chromatography described herein, such as a method including ceramic hydroxyapatite (HA) chromatography, e.g., hydroxyapatite type I or type II chromatography. In some embodiments, the mixed mode chromatography is performed using one or more of: CHT™ Ceramic Hydroxyapatite Type I Media, CHT™ Ceramic Hydroxyapatite Type II Media, BIO-GEL® HT Hydroxyapatite, and BIO-GEL® HTP Hydroxyapatite.

In some embodiments, subjecting the sample of arylsulfatase A to mixed mode chromatography includes: loading the sample of arylsulfatase A onto a mixed mode chromatography column (e.g., HA chromatography), washing the mixed mode chromatography column, and eluting the arylsulfatase A from the column. In some embodiments, subjecting the sample of arylsulfatase A to the mixed mode exchange chromatography is performed at a temperature about 23°C or less, about 18°C or less, or about 16°C or less, e.g., about 23°C, about 20°C, about 18°C, or about 16°C.

In some embodiments, loading the sample of arylsulfatase A onto the mixed mode chromatography column is performed with a loading buffer. In one embodiment, the loading buffer contains sodium phosphate. For example, the sodium phosphate concentration of the loading buffer is from about 1 mM to about 10 mM, e.g., from about 1 mM to about 5 mM, from about 5 mM to about 10 mM, e.g., about 1 mM, about 2 mM, or about 5 mM. In another embodiment, the loading buffer contains sodium chloride. For example, the sodium chloride concentration of the loading buffer is from about 100 mM to about 400 mM, e.g., from about 200 to about 300 mM, e.g., about 220 mM, about 240 mM, about 260 mM, or about 280 mM.

In some embodiments, loading the sample of arylsulfatase A onto the mixed mode chromatography column is performed at a pH from about 5 to about 9, e.g., from about 6 to about 8, e.g., about 7.

In some embodiments, the mixed-mode chromatography includes ceramic hydroxyapatite (HA) chromatography. Hydroxyapatite (HAP) usually refers to the crystalline form of calcium phosphate. The mechanism of HAP involves non-specific interactions between negatively charged protein carboxyl groups and positively charged calcium ions on the resin, and positively charged protein amino groups and negatively charged phosphate ions on the resin. Basic or acidic proteins can be adsorbed selectively onto the column by adjusting the buffer's pH; elution can be achieved by varying the buffer's salt concentration. Again, it is evident that numerous buffer compositions as well as combinations of buffers can be employed. Typically, however, the column can be equilibrated with 1-10 column washes of a buffer comprising 0.001 M NaPO4, 0.02 M MES-Tris, 0.26 M NaCl, pH 7.0. As of convenience the sample can be loaded in the buffer from the previous step of the purification process, or the sample can be loaded using a loading buffer. The column can be washed with 1-10 column volumes of the buffer used for equilibration, followed by a washing buffer comprising 0.005 M NaPO4, 0.02 M MES-Tris, 0.26 M NaCl, pH 7.0. Alternatively, the column can be equilibrated, loaded, and washed with any other equilibration, loading, and washing buffers described herein for mixed mode chromatography. The sample can be eluted in a buffer comprising 0.04 M NaPO4, pH 7.0. Optionally, the column can be stripped by washing with 1-10 column volumes of 0.4 M NaPO4, pH 12. Alternatively, the sample can be eluted in any other elution buffer described herein for mixed mode chromatography.

In some embodiments, washing the mixed mode chromatography column is performed with one or more washing buffers. For example, washing the mixed mode chromatography column can include two or more (e.g., a first and a second) washing steps, each using a different washing buffer.

In one embodiment, the washing buffer contains sodium phosphate. For example, the sodium phosphate concentration of the washing buffer is from about 1 mM to about 10 mM, e.g., from about 1 mM to about 5 mM, from about 5 mM to about 10 mM, e.g., about 1 mM, about 5 mM, or about 10 mM. In another embodiment, the washing buffer contains sodium chloride. For example, the sodium chloride concentration of the washing buffer is from about 50 mM to about 600 mM, e.g., from about 100 mM to about 500 mM, or from about 200 to about 400 mM, e.g., about 220 mM, about 240 mM, about 260 mM, or about 280 mM.

In some embodiments, washing the mixed mode chromatography column is performed at a pH from about 5 to about 9, e.g., from about 6 to about 8, e.g., about 7.

In some embodiments, eluting the arylsulfatase A from the mixed mode chromatography column is performed at a pH from about 5 to about 9, e.g., from about 6 to about 8, e.g., about 7. In some embodiments, eluting the arylsulfatase A from the mixed mode chromatography column includes one or more steps of elution peak collection. For example, the elution peak collection starts from about 50 mAU at the ascending side to about 50 mAU at the descending side, e.g., from about 100 mAU at the ascending side to about 50 mAU at the descending side, from about 200 mAU at the ascending side to about 50 mAU at the descending side, from about 50 mAU at the ascending side to about 100 mAU at the descending side, from about 50 mAU at the ascending side to about 200 mAU at the descending side, or from about 100 mAU at the ascending side to about 100 mAU at the descending side, e.g., as determined by spectrophotometry, e.g., at 280 nM.

The loading buffer, washing buffer, and elution buffer described herein can include one or more buffering agents. For example, the buffering agent can be TRIS, HEPES, MOPS, PIPES, SSC, MES, sodium phosphate, sodium acetate, or a combination thereof. The concentration of the buffering agent is between about 1 mM and about 500 mM, e.g., between about 10 mM and about 250 mM, between about 20 mM and about 100 mM, between about 1 mM and 5 mM, between about 5 mM and 10 mM, between about 10 mM and 50 mM, or between about 50 mM and about 100 mM, e.g., about 1 mM, about 5 mM, about 10 mM, about 20 mM, about 30 mM, about 40 mM, or about 50 mM.

In some embodiments, the purification of ASA by mixed mode chromatography succeeds the purification by ion-exchange chromatography (e.g., anion exchange chromatography). It is contemplated, however, that these steps could be performed in the reverse order.

Yield following mixed mode chromatography may vary. In some embodiments, the arylsulfatase A activity yield is at least about 80%, e.g., at least about 90%, e.g., between about 80% and about 115%. In some embodiments, the protein yield (AU or Absorbance Units) is from about 30% to 80%, e.g., from about 35% to about 75%, or from about 50% to about 70%, e.g., as determined by spectrophotometry, e.g., at 280 nm.

Purity following mixed mode chromatography is greatly improved. In some embodiments, the specific activity of the purified arylsulfatase A is at least from about 50 U/mg to about 140 U/mg, e.g., at least about 70 U/mg, at least about 90 U/mg, at least about 100 U/mg, or at least about 120 U/mg, e.g., as determined by a method described herein. In some embodiment, the arylsulfatase A is purified to at least about 95%, at least about 98%, at least about 99%, at least about 99.5%, at least about 99.6%, at least about 99.7%, at least about 99.8%, or at least about 99.9%. The purity of arylsulfatase A can be measured by, e.g., one or more of: host cell protein (HCP) Western blot, SDS-PAGE Coomassie staining, SDS-PAGE silver staining, reverse phase HPLC, and size exclusion HPLC. In some embodiments, the host cell protein (HCP) log reduction value (LRV) is between about 0.3 and about 0.6, e.g., between about 0.4 and 0. 5.

## *Hydrophobic Interaction Chromatography (HIC)*

The purification methods described herein can include subjecting the sample of arylsulfatase A to hydrophobic interaction chromatography (HIC). In one embodiment, the hydrophobic interaction chromatography includes phenyl chromatography. In other embodiments, the hydrophobic interaction chromatography includes butyl chromatography or octyl chromatography. In some embodiments, subjecting the sample of arylsulfatase A to HIC is performed at a temperature about 23°C or less, about 18°C or less, or about 16°C or less, e.g., about 23°C, about 20°C, about 18°C, or about 16°C. In some embodiments, the sample of arylsulfatase A is subjected to mixed mode chromatography prior to HIC.

Hydrophobic interaction chromatography utilizes the attraction of a given molecule for a polar or non-polar environment, and in terms of protein, this propensity is governed by the hydrophobicity or hydrophilicity of residues on the exposed, outer surface of a protein. Thus, proteins are fractionated based upon their varying degrees of attraction to a hydrophobic matrix, typically an inert support with alkyl linker arms of 2-18 carbons in chain length. The stationary phase consists of small non-polar groups (butyl, octyl, or phenyl) attached to a hydrophilic polymer backbone (e.g., cross-linked Sepharose™, dextran, or agarose). Thus, the HIC column is typically a butyl SEPHAROSE™ column or a phenyl SEPHAROSE™ column, most typically a phenyl SEPHAROSE™ column.

In some embodiments, the hydrophobic interaction chromatography includes phenyl chromatography using one or more of Phenyl SEPHAROSE™ High Performance, Phenyl SEPHAROSE™ 6 Fast Flow (low sub), or Phenyl SEPHAROSE™ 6 Fast Flow (high sub).

In some embodiments, subjecting the sample of arylsulfatase A to hydrophobic interaction chromatography includes: loading the sample of arylsulfatase A onto a HIC column, washing the HIC column, and eluting the arylsulfatase A from the column. Loading, washing and elution in HIC basically follow the same principle as described above for the ion-exchange chromatography, but often nearly opposite conditions to those used in ion exchange chromatography are applied. Thus, the HIC process involves the use of a high salt loading buffer, which unravels the protein to expose hydrophobic sites. The protein is retained by the hydrophobic ligands on the column, and is exposed to a gradient of buffers containing decreasing salt concentrations. As the salt concentration decreases, the protein returns to its native conformation and eventually elutes from the column. Alternatively proteins may be eluted with PEG.

In some embodiments, loading the sample of arylsulfatase A onto the HIC column is performed with a loading buffer. In one embodiment, the loading buffer contains sodium chloride. For example, the sodium chloride concentration of the loading buffer is from about 0.5 M to about 2.5 M, e.g., about 1 M or about 1.5 M. In another embodiment, the loading buffer contains sodium phosphate. For example, the sodium phosphate concentration of the loading buffer is from about 10 mM to about 100 mM, e.g., about 25 mM, about 50 mM, or about 75 mM. In some embodiments, loading the sample of arylsulfatase A onto the HIC column is performed at a pH from about 5 to about 7, e.g., from about 5.5 to about 6.5, e.g., about 5.5, about 6.0, or about 6.5. In some embodiments, the sample of arylsulfatase A is loaded onto the HIC column at a binding capacity about 12 AU/L resin or less, e.g., about 10 AU/L resin or less, about 9 AU/L resin or less, about 7 AU/L resin or less, or about 5 AU/L resin or less, e.g., between about 5 AU/L resin and about 9 AU/L resin, or between about 5 AU/L resin and about 7 AU/L resin.

The use of phenyl SEPHAROSE™ as solid phase in the HIC is typical in the present disclosure. Again, it is readily apparent that, when it comes to the exact conditions as well as the buffers and combinations of buffers used for the loading, washing and elution processes, a large number of different possibilities exist. In a typical embodiment, the column can be equilibrated in a buffer which contains 0.05 M NaPO4, 1 M NaCl, pH 5.5. As of convenience the sample can be loaded in the buffer from the previous step of the purification process, or the sample can be loaded using a loading buffer.

In some embodiments, washing the HIC column is performed with one or more washing buffers. For example, washing the HIC column can include two or more (e.g., a first and a second) washing steps, each using a different washing buffer. In some embodiments, the washing buffer contains sodium chloride. For example, the sodium chloride concentration of the washing buffer is from about 100 mM to about 1.5 M, e.g., from about 250 mM to about 1 M, e.g., about 250 mM, about 500 mM, about 750 mM, or about 1 M. In another embodiment, the washing buffer contains sodium phosphate. For example, the sodium phosphate concentration of the loading buffer is from about 10 mM to about 100 mM, e.g., about 25 mM, about 50 mM, or about 75 mM. In some embodiments, washing the HIC column is performed at a pH from about 5 to about 7, e.g., from about 5.5 to about 6.5, e.g., about 5.5, about 6.0, or about 6.5. For example, washing can be performed using 1-2 column washes of equilibration buffer followed by 1-5 column volumes of 0.02 M MES, 0.05 M NaPO4, 0.5 M NaCl, pH 5.5. Alternatively, the column can be equilibrated, loaded, and washed with any other equilibration, loading, and washing buffers described herein for HIC.

In some embodiments, eluting the arylsulfatase A from the HIC column is performed with an elution buffer. In some embodiments, the elution buffer contains sodium chloride. For example, the sodium chloride concentration of the elution buffer is from about 30 mM to about 100 mM, e.g., from about 45 mM to about 85 mM, e.g., about 50 mM, about 60 mM, about 70 mM, or about 80 mM. In some embodiments, eluting the arylsulfatase A from the HIC column is performed at a pH from about 5 to about 9, e.g., from about 6 to about 8, e.g., about 7. For example, arylsulfatase A can be eluted using 0.02 M MES-Tris, 0.06 M NaCl, pH 7.0. Alternatively, the sample can be eluted in any other elution buffer described herein for HIC.

In some embodiments, eluting the arylsulfatase A from the HIC column includes one or more steps of elution peak collection. For example, the elution peak collection starts from about 50 mAU at the ascending side to about 50 mAU at the descending side, e.g., from about 100 mAU at the ascending side to about 50 mAU at the descending side, from about 200 mAU at the ascending side to about 50 mAU at the descending side, from about 50 mAU at the ascending side to about 100 mAU at the descending side, from about 50 mAU at the ascending side to about 200 mAU at the descending side, or from about 100 mAU at the ascending side to about 100 mAU at the descending side, e.g., as determined by spectrophotometry, e.g., at 280 nM.

In some embodiments, the purification of arylsulfatase A by HIC succeeds the purification by ion-exchange chromatography (e.g., anion exchange chromatography) and/or mixed mode chromatography. It is contemplated, however, that these steps could be performed in the reverse order.

The loading buffer, washing buffer, and elution buffer described herein can include one or more buffering agents. For example, the buffering agent can be TRIS, HEPES, MOPS, PIPES, SSC, MES, sodium phosphate, sodium acetate, or a combination thereof. The concentration of the buffering agent is between about 1 mM and about 500 mM, e.g., between about 10 mM and about 250 mM, between about 20 mM and about 100 mM, between about 1 mM and 5 mM, between about 5 mM and 10 mM, between about 10 mM and 50 mM, or between about 50 mM and about 100 mM, e.g., about 1 mM, about 5 mM, about 10 mM, about 20 mM, about 30 mM, about 40 mM, or about 50 mM.

Yield following HIC may vary. In some embodiments, the arylsulfatase A activity yield is at least about 60%, e.g., at least about 70%, e.g., between about 70% and about 100%. In some embodiments, the protein yield (AU or Absorbance Units) is from about 45% to 100%, e.g., from about 50% to about 95%, or from about 55% to about 90%, e.g., as determined by spectrophotometry, e.g., at 280 nm.

Purity following HIC is greatly improved. In some embodiments, the specific activity of the purified arylsulfatase A is at least from about 50 U/mg to about 140 U/mg, e.g., at least about 70 U/mg, at least about 90 U/mg, at least about 100 U/mg, or at least about 120 U/mg, e.g., as determined by a method described herein.

In some embodiments, the arylsulfatase A is purified to at least about 95%, at least about 98%, at least about 99%, at least about 99.5%, at least about 99.6%, at least about 99.7%, at least about 99.8%, or at least about 99.9%. The purity of arylsulfatase A can be measured by, e.g., one or more of: host cell protein (HCP) Western blot, SDS-PAGE Coomassie staining, SDS-PAGE silver staining, reverse phase HPLC, and size exclusion HPLC. In some embodiments, the host cell protein (HCP) log reduction value (LRV) is between about 0.6 and about 1.2, e.g., between about 0.7 and 0.95.

### *Ultrafiltration/Diafiltration*

The purification methods described herein can include one or more steps of downstream ultrafiltration and/or diafiltration. In some embodiments, the method further comprises concentrating and/or filtering the sample of arylsulfatase A, e.g., by ultrafiltration and/or diafiltration, e.g., by tangential flow ultrafiltration.

Ultrafiltration refers to a membrane separation process, driven by a pressure gradient, in which the membrane fractionates components of a liquid as a function of their solvated size and structure. Diafiltration is a specialized type of ultrafiltration process in which the retentate is diluted with water and re-ultrafiltered, to reduce the concentration of soluble permeate components and increase further the concentration of retained components. Ultrafiltration is often combined with diafiltration into ultrafiltration/diafiltration (UFDF) purification steps.

Embodiments of the invention utilize at least one, at least two, at least three or more downstream UFDF purification steps. One or more diafiltrations may occur within UFDF step (e.g., UFDFDF). In some embodiments, the protein yield (AU or Absorbance Units) following downstream UFDF, relative the amount from the preceding purification step, is from about 90% to 105%, e.g., from about 95% to about 100%, e.g., from about 97% to about 99%, as determined by spectrophotometry, e.g., at 280 nm. In some embodiments, essentially no protein is lost during UFDF.

In some embodiments of the invention, downstream UFDF results in rASA that is at least about 95%, at least about 97%, at least about 98%, at least about 99% or more pure, as determined by size exclusion chromatography-high performance liquid chromatography (SEC-HPLC) and/or reverse phase-high performance liquid chromatography

(RP-HPLC). In some embodiment, the arylsulfatase A is purified to at least about 95%, at least about 98%, at least about 99%, at least about 99.5%, at least about 99.6%, at least about 99.7%, at least about 99.8%, or at least about 99.9%.

The purity of arylsulfatase A can be measured by, e.g., one or more of: host cell protein (HCP) Western blot, SDS-PAGE Coomassie staining, SDS-PAGE silver staining, reverse phase HPLC, and size exclusion HPLC. The specific activity of the rASA is at least from about 60 U/mg to about 100 U/mg, e.g., at least about 65 U/mg, at least about 90 U/mg, at least about 70 U/mg, or at least about 90 U/mg, e.g., as determined by a sulfatase release assay, as described below.

In some embodiments, arylsulfatase A is purified by separation from contaminants according to their size in an acidic environment by tangential flow filtration. Arylsulfatase A forms an octamer at low pH with a theoretical molecular weight of 480 kDa and will therefore be retained by a relatively open membrane while most of the contaminants will pass this membrane (Sommerlade et al., (1994) Biochem. J., 297; 123-130; Schmidt et al., (1995) Cell, 82 271-278; Lukatela et al., (1998) Biochemistry, 37, 3654-3664).

In a typical embodiment, the diafiltration buffer comprises 0.01 M sodium phosphate-citrate, 0.137 M NaCl, pH 6.0.

In some embodiments, as the starting material for this process is a suspension of arylsulfatase A as eluted from the chromatography column in the previous step of the process, the pH in this suspension is adjusted to 4-5 by addition of 0.2-1 M Na-acetate pH 4.5. Diafiltration is then performed against 1-10 buffer volumes of Na-acetate pH 4.0-5.5 in a manner well known to somebody skilled in the art. The filtration can be performed with the application of several different filter types with nominal weight cut-off values ranging from 20-450 kDa, however it is typical to use a filter with a cut-off value ranging from 100 - 300 kDa. For further processing of the arylsulfatase A containing solution the pH is adjusted to a value within the range between 7 and 8 by addition of Tris-base to a final concentration of approximately 20-50 mM.

As an alternative to the acidic tangential flow filtration as described above, separation of ASA from the contaminants can be obtained with acidic gel filtration using essentially the same conditions and compositions of buffers. The filtration is performed at low pH through a gel filtration column, which has been equilibrated with a solution at low pH, for example, a 0.2-0.9 M solution of Na-acetate at pH 4-5. As an option, the solution of arylsulfatase A can be concentrated by tangential flow filtration through a 20-50 kDa filter prior to the gel filtration. The extent of concentration may vary considerably so that arylsulfatase A may be concentrated from about 0.1 mg/ml to about 50 mg/ml, preferably to about 5 mg/ml.

In some embodiments, the sample pool is concentrated against a Biomax A-screen, 30 kDa. Diafiltration is performed against 3-5 column washes of 20 mM Na-acetate, pH 5.4-5.7.

In embodiments, a surfactant such as polysorbate-20 (P20) is added to the compositions comprising purified ASA protein prior to cold storage. In embodiments, the composition comprises a surfactant such as P20 in a concentration of about 0.0001 %(v/v) to about 0.01 %(v/v), about 0.001 %(v/v) to about 0.01 %(v/v), about 0.001%(v/v), about 0.002 %(v/v), about 0.003 %(v/v), about 0.004 %(v/v), about 0.005 %(v/v), about 0.006 %(v/v), about 0.007 %(v/v), about 0.008 %(v/v), about 0.009 %(v/v), or about 0.01 %(v/v).

### Characterization of Purified ASA Proteins

Purified recombinant ASA protein may be characterized using various methods.

### *Purity*

The purity of purified recombinant ASA protein is typically measure by the level of various impurities (e.g., host cell protein or host cell DNA) present in the final product.

For example, the level of host cell protein (HCP) may be measured by ELISA or SDS-PAGE. In some embodiments, the purified recombinant ASA protein contains less than 150 ng HCP/mg ASA protein (e.g., less than 140, 130, 120, 110, 100, 90, 80, 70, 60, 50, 40, 30, 30, 20, 10 ng HCP/mg ASA protein). In embodiments, purified recombinant ASA protein contains less than about 100, about 80, or about 60 ng HCP/mg ASA.

In some embodiments, the purified recombinant ASA protein contains less than about 150 pg/mg, 140 pg/mg, 130 pg/mg, 120 pg/mg, 110 pg/mg, 100 pg/mg, 90 pg/mg, 80 pg/mg, 70 pg/mg, 60 pg/mg, 50 pg/mg, 40 pg/mg, 30 pg/mg, 20 pg/mg, or 10 pg/mg Host Cell DNA (HCD). In embodiments, purified recombinant ASA protein contains less than about 50, about 10, about 9, about 8, about 7, about 6, about 5, about 4, about 3, about 2, or about 1 pg HCD /mg ASA protein.

In some embodiments, the purified recombinant ASA protein, when subject to SDS-PAGE with Coomassie Brilliant Blue staining, has no new bands with intensity greater than the 0.05%, 0.01%, 0.15%, 0.2%, 0.25%, 0.3%, 0.35%, 0.4%, 0.45%, or 0.5% assay control.

In some embodiments, the purified recombinant ASA protein, when subject to SDS-PAGE with Western blotting against HCP, has no bands with intensity greater than the 15 kDa HCP band assay control, and no new bands with intensity greater than the 0.05%, 0.01%, 0.15%, 0.2%, 0.25%, 0.3%, 0.35%, 0.4%, 0.45%, 0.5%, or 1.0% assay control. In embodiments, no more than three HCP bands are detected.

In some embodiments, the purified recombinant ASA protein, when subject to SDS-PAGE with silver staining, has no new bands with intensity greater than the 0.05%, 0.01%, 0.15%, 0.2%, 0.25%, 0.3%, 0.35%, 0.4%, 0.45%, or 0.5% assay control.

In some embodiments, the host cell protein (HCP) log reduction value (LRV) is between about 0.3 and about 0.6, e.g., between about 0.4 and 0.5. Various assay controls may be used, in particular, those acceptable to regulatory agencies such as FDA.

The purity of purified recombinant ASA protein may also be determined by one or more of size exclusion chromatography-high performance liquid chromatography (SEC-HPLC), capillary electrophoresis-SDS PAGE (CE-SDS PAGE), and/or reverse phase-high performance liquid chromatography (RP-HPLC) (e.g., using columns of octadecyl (C18)-bonded silica, and carried out at an acidic pH with TFA as a counter-ion). In some embodiments of the invention, the major peak in the chromatogram is ASA. Parameters that may be altered or optimized to increase resolution include gradient conditions, organic modifier, counter ion, temperature, column pore size and particle size, solvent composition and flow rate. Purity levels may be discerned by main peak percentage, as known to those of skill in the art. For example, purity may be determined by integrating the main and side peaks observed and calculating the main peak's percentage of the total area.

In some embodiments of the invention, the purity of ASA purified by the methods disclosed herein and as determined by the main peak percentage of SEC-HPLC is greater than or equal to 95% (e.g., about 96%, about 97%, about 98%, about 99% or higher). In some embodiments of the invention, the purity of ASA purified by the methods disclosed herein and as determined by the main peak percentage of SEC-HPLC is greater than or equal to 97% (e.g., about 97%, about 98%, about 99%, or higher).

In some embodiments of the invention, the purity of ASA purified by the methods disclosed herein and as determined by main peak percentage of RP-HPLC is greater than or equal to 97% (i.e., about 97%, about 98%, about 99% or higher). In some embodiments of the invention, the purity of ASA purified by the methods disclosed herein and as determined by main peak percentage of RP-HPLC is greater than or equal to 98% (i.e., about 98%, about 99% or higher).

*Specific Activity*

Purified recombinant ASA protein may also be characterized by evaluating functional and/or biological activity. The enzyme activity of a recombinant ASA composition may be determined using methods known in the art. Typically the methods involve detecting the removal of sulfate from a synthetic substrate, which is known as sulphate release assay. One example of an enzyme activity assay involves the use of ion chromatography. This method quantifies the amount of sulfate ions that are enzymatically released by recombinant ASA from a substrate. The substrate may be a natural substrate or a synthetic substrate. In some cases, the substrate is heparin sulfate, dermatan sulfate, or a functional equivalent thereof. Typically, the released sulfate ion is analyzed by ion chromatography with a conductivity detector. In this example, the results may be expressed as U/mg of protein where 1 Unit is defined as the quantity of enzyme required to release 1 $\mu$mole sulfate ion per hour from the substrate. In some embodiments, the purified recombinant ASA has a specific activity of at least about 50 U/mg, 60 U/mg, 70 U/mg, 80 U/mg, 90 U/mg, 100 U/mg, 110 U/mg, 120 U/mg, 130 U/mg, 140 U/mg. In some embodiments, the purified recombinant ASA has a specific activity ranging from about 50-200 U/mg (e.g., about 50-190 U/mg, 50-180 U/mg, 50-170 U/mg, 50-160 U/mg, 50-150 U/mg, 50-140 U/mg, 50-130 U/mg, 50-120 U/mg, 50-110 U/mg, 50-100 U/mg, 60-200 U/mg, 60-190 U/mg, 60-180 U/mg, 60-170 U/mg, 60-160 U/mg, 60-150 U/mg, 60-140 U/mg, 60-130 U/mg, 60-120 U/mg, 60-110 U/mg, 60-100 U/mg, 70-200 U/mg, 70-190 U/mg, 70-180 U/mg, 70-170 U/mg, 70-160 U/mg, 70-150 U/mg, 70-140 U/mg, 70-130 U/mg, 70-120 U/mg, 70-110 U/mg, 70-100 U/mg, 80-200 U/mg, 80-190 U/mg, 80-180 U/mg, 80-170 U/mg, 80-160 U/mg, 80-150 U/mg, 80-140 U/mg, 80-130 U/mg, 80-120 U/mg, 80-110 U/mg, 80-100 U/mg, 90-200 U/mg, 90-190 U/mg, 90-180 U/mg, 90-170 U/mg, 90-160 U/mg, 90-150 U/mg, 90-140 U/mg, 90-130 U/mg, 90-120 U/mg, 90-110 U/mg, 90-100 U/mg, 100-200 U/mg, 100-190 U/mg, 100-180 U/mg, 100-170 U/mg, 100-160 U/mg, 100-150 U/mg, 100-140 U/mg, 100-130 U/mg, 100-120 U/mg, 100-110 U/mg, 110-200 U/mg, 110-190 U/mg, 110-180 U/mg, 110-170 U/mg, 110-160 U/mg, 110-150 U/mg, 110-140 U/mg, 110-130 U/mg, 110-120 U/mg, 120-200 U/mg, 120-190 U/mg, 120-180 U/mg, 120-170 U/mg, 120-160 U/mg, 120-150 U/mg, 120-140 U/mg, 120-130 U/mg, 130-200 U/mg, 130-190 U/mg, 130-180 U/mg, 130-170 U/mg, 130-160 U/mg, 130-150 U/mg, or 130-140 U/mg). In embodiments, specific activity of purified recombinant ASA is about 50 to about 130 U/mg, about 70 to about 100 U/mg, about 80 to about 90 U/mg, or about 75 to about 95 U/mg.

In another example, enzyme activity of a recombinant ASA composition may be determined by measuring the removal of sulfate from a 4-methylumbelliferyl-sulfate (4-MUF-sulfate) substrate to form the fluorescent methylumbelliferone. In this example, the fluorescence signal generated by a test sample can be used to calculate enzyme activity (in mU/mL) using a standard of 4-MUF. One milliunit of activity is defined as the quantity of enzyme required to convert 1 nanomole of 4-MUF-sulfate to 4-MUF in 1 minute at 37 °C. Specific activity may then calculated by dividing the enzyme activity by the protein concentration.

In some embodiments, activity is determined by hydrolysis of the synthetic, chromogenic substrate, para-Nitrocatechol sulphate (pNCS) which has an end product, para-Nitrocatechol (pNC) that absorbs light at 515 nm. The following equation may be used to calculate the enzyme activity in $\mu$mol pNCS hydrolyzed / min $\times$ ml (=Units/ml):

$$Vtot \ (ml) \ x \ \Delta A \ = \ Units/ml \ (1)$$

$$\varepsilon M \ /1000 \ x \ Vsample \ (ml) \ x \ Incubation \ time \ (min),$$

where:

$$\Delta A = absorbance \ of \ sample - absorbance \ of \ blank$$

Vtot (ml) = total reaction volume in ml (in this case 0.15 ml)
Vsample (ml) = added sample volume in ml (in this case 0.05 ml)
$\varepsilon M$ = the molar extinction coefficient for the product pNC, which in this case is 12 400 M-1 cm-1.

Equation 1 can be simplified as:

$$\Delta A \ x \ (0.15 \ / \ (12 \ 400/1000 \ x \ 0.05 \ x \ 30)) = X \ \mu mol \ / \ (minute \ x \ ml) \ (=Units/ml) \ (1)$$

To calculate the specific activity in $\mu$mol pNC consumed/(minute $\times$ mg) (= Units/mg), equation 1 is divided by the protein concentration of the sample:

$$Eq. \ 1 \ / \ Protein \ conc. \ (mg/ml) = Y \ \mu mol \ / \ (minute \ x \ mg) = Units/mg \ (2)$$

In any example, the protein concentration of a recombinant ASA composition may be determined by any suitable method known in the art for determining protein concentrations. In some cases, the protein concentration is determined by an ultraviolet light absorbance assay. Such absorbance assays are typically conducted at about a 280 nm wavelength (A280).

In some embodiments, purified recombinant ASA has a specific activity on a 4-methylumbelliferone substrate in a range of about $1.0 \times 10^3$ mU/mg to $100.0 \times 10^3$ mU/mg, about $1.0 \times 10^3$ mU/mg to $50.0 \times 10^3$ mU/mg, about $1.0 \times 10^3$ mU/mg to $40.0 \times 10^3$ mU/mg, about $1.0 \times 10^3$ mU/mg to $30.0 \times 10^3$ mU/mg, about $1.0 \times 10^3$ mU/mg to $20.0 \times 10^3$ mU/mg, about $1.0 \times 10^3$ mU/mg to $10.0 \times 10^3$ mU/mg, about $4.0 \times 10^3$ mU/mg to $8.0 \times 10^3$ mU/mg, about $4.0 \times 10^3$ mU/mg to $10.0 \times 10^3$ mU/mg, about $4.5 \times 10^3$ mU/mg to $10.0 \times 10^3$ mU/mg, about $5.0 \times 10^3$ mU/mg to $10.0 \times 10^3$ mU/mg, about $5.5 \times 10^3$ mU/mg to $15.0 \times 10^3$ mU/mg, or about $4.0 \times 10^3$ mU/mg to $20.0 \times 10^3$ mU/mg. In some embodiments, purified recombinant ASA has a specific activity on a 4-methylumbelliferone substrate of about $1.0 \times 10^3$ mU/mg, about $2.0 \times 10^3$ mU/mg, about $3.0 \times 10^3$ mU/mg, about $4.0 \times 10^3$ mU/mg, about $5.0 \times 10^3$ mU/mg, about $10.0 \times 10^3$ mU/mg, about $15.0 \times 10^3$ mU/mg, about $20.0 \times 10^3$ mU/mg, about $25.0 \times 10^3$ mU/mg, about $30.0 \times 10^3$ mU/mg, about $35.0 \times 10^3$ mU/mg, about $40.0 \times 10^3$ mU/mg, about $45.0 \times 10^3$ mU/mg, about $50.0 \times 10^3$ mU/mg, or more.

## Charge Profile

Purified recombinant ASA may be characterized by the charge profile associated with the protein. Typically, protein charge profile reflects the pattern of residue side chain charges, typically present on the surface of the protein. Charge profile may be determined by performing an ion exchange (IEX) chromatography (e.g., HPLC) assay on the protein. In some embodiments, a "charge profile" refers to a set of values representing the amount of protein that elutes from an ion exchange column at a point in time after addition to the column of a mobile phase containing an exchange ion.

Typically, a suitable ion exchange column is an anion exchange column. For example, a charge profile may be determined by strong anion exchange (SAX) chromatography using a high performance liquid chromatography (HPLC) system. In general, recombinant ASA adsorbs onto the fixed positive charge of a strong anion exchange column and a gradient of increasing ionic strength using a mobile phase at a predetermined flow rate elutes recombinant ASA species from the column in proportion to the strength of their ionic interaction with the positively charged column. More negatively charged (more acidic) ASA species elute later than less negatively charged (less acid) ASA species. The concentration of proteins in the eluate is detected by ultraviolet light absorbance (at 280 nm).

In some embodiments, recombinant ASA adsorbs at about pH 8.0 in 20 mM TRIS-HCl onto the fixed positive charge of a Mini Q PE column and a gradient of increasing ionic strength using a mobile phase consisting of 20 mM TRIS-HCL, 1 M sodium chloride, pH 8.0 at a flow rate of 0.8 ml/min elutes recombinant ASA species from the column in proportion to the strength of their ionic interaction with the positively charged column.

In some embodiments, a charge profile may be depicted by a chromatogram of absorbance units versus time after elution from the HPLC column. The chromatogram may comprise a set of one or more peaks, with each peak in the set identifying a subpopulation of recombinant ASAs of the composition that have similar surface charges.

***Glycan Mapping***

In some embodiments, a purified recombinant ASA protein may be characterized by its proteoglycan composition, typically referred to as glycan mapping. Without wishing to be bound by any theory, it is thought that glycan linkage along with the shape and complexity of the branch structure may impact in vivo clearance, lysosomal targeting, bioavailability, and/or efficacy.

Typically, a glycan map may be determined by enzymatic digestion and subsequent chromatographic analysis. Various enzymes may be used for enzymatic digestion including, but not limited to, suitable glycosylases, peptidases (e.g., endopeptidases, exopeptidases), proteases, and phosphatases. In some embodiments, a suitable enzyme is alkaline phosphatase. In some embodiments, a suitable enzyme is neuraminidase. Glycans (e.g., phosphoglycans) may be detected by chromatographic analysis. For example, phosphoglycans may be detected by High Performance Anion Exchange Chromatography with Pulsed Amperometric Detection (HPAE-PAD) or size exclusion High Performance Liquid Chromatography (HPLC). The quantity of glycan (e.g., phosphoglycan) represented by each peak on a glycan map may be calculated using a standard curve of glycan (e.g., phosphoglycan), according to methods known in the art and disclosed herein.

In embodiments, the purified recombinant ASA protein is present as species comprising: neutral recombinant ASA protein (group A), sialylated recombinant ASA protein (group B), mannose-6-phosphated recombinant ASA protein (group C), N-acetyl-glucosamine mannose-6-phosphated recombinant ASA protein (group E), or hybrid recombinant ASA protein (group E), or any combination thereof. In embodiments, the purified recombinant ASA protein is present as neutral recombinant ASA protein (group A), sialylated recombinant ASA protein (group B), mannose-6-phosphated recombinant ASA protein (group C), N-acetyl-glucosamine mannose-6-phosphated recombinant ASA protein (group E), and hybrid recombinant ASA protein (group E).

In some embodiments, a purified recombinant ASA protein according to the present invention is characterized with a glycan map comprising at least seven peak groups indicative of neutral (peak group 1), mono-sialylated (peak group 2), capped mannose-6-phosphated (peak group 3), di-sialylated (peak group 4), mono-mannose-6-phosphated (peak group 5), hybrid (peak group 6), and di-mannose-6-phosphated (peak group 7) ASA protein, respectively.

The relative amount of glycan corresponding to a group (e.g., any of groups A-E or peak groups 1-7) may be determined based on the peak group area relative to the corresponding peak group area in a predetermined reference standard.

In embodiments, group A (neutral recombinant ASA protein) may have a peak group area that ranges from about 15 to about 25% (e.g., about 16% to about 22% or about 20% to about 25%) relative to a corresponding peak group area in a reference standard. In embodiments, a peak group area of neutral recombinant ASA protein is the range characteristic of any of the exemplary formulations in Example 3, with a variation of about 1% to about 10% in either direction for each endpoint of the exemplified range (e.g., a variation of about 1% to about 5% for each endpoint of the exemplified range).

In embodiments, group B (sialylated recombinant ASA protein) may have a total peak group area of sialylated recombinant ASA protein species that ranges from about 35% to about 45% (e.g., about 35% to about 42% or about 37% to about 42%) relative to any corresponding peak group areas in a reference standard. In embodiments, a total peak group area of sialylated recombinant ASA protein is the range characteristic of any of the exemplary formulations in Example 3, with a variation of about 1% to about 10% in either direction for each endpoint of the exemplified range (e.g., a variation of about 1% to about 5% for each endpoint of the exemplified range).

In embodiments, group C (mannose-6-phosphated recombinant ASA protein) may have a total peak group area of mannose-6-phosphated recombinant ASA protein species that ranges from about 20% to about 30% (e.g., about 20% to about 26%, about 25% to about 28%, or about 27% to about 32%) relative to any corresponding peak group areas in a reference standard. In embodiments, a total peak group area of mannose-6-phosphated recombinant ASA protein is the range characteristic of any of the exemplary formulations in Example 3, with a variation of about 1% to about 10% in either direction for each endpoint of the exemplified range (e.g., a variation of about 1% to about 5% for each endpoint of the exemplified range).

In embodiments, group D (N-acetyl-glucosamine mannose-6-phosphated recombinant ASA protein) may have a total peak group area of N-acetyl-glucosamine mannose-6-phosphated recombinant ASA protein species that ranges from about 1% to about 10% (e.g., about 3% to about 5%, about 4% to about 6%, or about 4.5% to about 5.5%) relative to any corresponding peak group areas in a reference standard. In embodiments, a total peak group area of N-acetyl-glucosamine mannose-6-phosphated recombinant ASA protein in is the range characteristic of any of the exemplary formulations in Example 3, with a variation of about 1% to about 10% in either direction for each endpoint of the exemplified range (e.g., a variation of about 1% to about 5% for each endpoint of the exemplified range).

In embodiments, group E (hybrid recombinant ASA protein) may have a peak group area of hybrid recombinant ASA protein that ranges from about 5% to about 15% (e.g., about 7% to about 10%, about 7% to about 9%, or about 7.5% to about 8.5%) relative to any corresponding peak group areas in a reference standard. In embodiments, a total peak group area of hybrid recombinant ASA protein in is the range characteristic of any of the exemplary formulations in Example 3, with a variation of about 1% to about 10% in either direction for each endpoint of the exemplified range (e.g., a variation of about 1% to about 5% for each endpoint of the exemplified range).

Various reference standards for glycan mapping are known in the art and can be used to practice the present invention.

It is contemplated that the glycosylation pattern of a purified recombinant ASA protein (e.g., a composition comprising purified recombinant ASA protein having a threshold population of mannose-6-phosphated recombinant ASA protein (M6P ASA protein)) may impact the bioavailability, targeting, or efficacy of the protein.

### *Peptide Mapping*

In some embodiments, peptide mapping may be used to characterize amino acid composition, post-translational modifications, and/or cellular processing; such as cleavage of a signal peptide, and/or glycosylation. Typically, a recombinant protein may be broken into discrete peptide fragments, either through controlled or random breakage, to produce a pattern or peptide map. In some cases, a purified ASA protein may be first subjected to enzymatic digest prior to analytic analysis. Digestion may be performed using a peptidase, glycoside hydrolase, phosphatase, lipase or protease and/or combinations thereof, prior to analytic analysis. The structural composition of peptides may be determined using methods well known in the art. Exemplary methods include, but are not limited to, Mass spectrometry, Nuclear Magnetic Resonance (NMR) or HPLC.

### *Metals Analysis*

In some embodiments, a purified recombinant ASA protein may be characterized by metals analysis. Various methods of analyzing trace metals in purified drug substances are known in the art and can be used to practice the present invention.

In some embodiments, residual phosphorous is measured and compared to a reference sample. Without wishing to be bound by any particular theory, it is hypothesized that residual phosphorus contributes to maintaining drug substance pH. In some embodiments of the invention, residual phosphorous is between about 10-50 ppm (i.e., between about 10-45 ppm, about 10-40 ppm, about 10-30 ppm, about 20-50 ppm about 20-45 ppm, about 20-40 ppm, about 20-30 ppm, about 30-50 ppm, about 30-40 ppm). In some embodiments, the pH range of recombinant ASA purified according to the methods disclosed herein is between about 5-7 (i.e., between about 5.5-7.0, about 5.5-6.5, about 5.5-6.0, about 6.0-7.0, about 6.0-6.5, about 6.0-6.4, about 6.0-6.3, about 6.0-6.2, about 6.0-6.1, about 6.1-6.2).

In some embodiments, recombinant ASA purified according to the methods disclosed herein contains calcium. Without wishing to be bound by any particular theory, it is hypothesized that calcium ions present in the active site of ASA may be necessary for enzymatic activity. In some embodiments of the invention, calcium is present at levels between about 1-20 ppm (i.e., between about 1-15 ppm, about 1-10 ppm, about 5-15 ppm, about 5-10 ppm, about 10-20 ppm, about 10-15 ppm, about 10-14 ppm, about 10-13 ppm, about 10-12 ppm).

### EXAMPLES

### Example 1. Exemplary Process

Purified recombinant ASA protein was prepared according to exemplary processes described herein. Table 3 specifies certain particular features for an exemplary process for the preparation of purified recombinant ASA protein. In embodiments, a process for purifying recombinant ASA protein includes one or more of the features described in Table 3.

**Table 3**

| Process Feature | Exemplary Process |
|---|---|
| Production bioreactor | • pH=7.15<br>• Perfusion rate=1.40 VVD<br>• Target cell density=4.0E6 cells/mL |
| Capture UFDF | Cuno depth filter ZB |
| Column 1: Anion Exchange (AEX) | Fractogel TMAE HiCap resin |
| Column 3: Hydrophobic Interaction Column (HIC) | 60 cm Phenyl Sepharose FF column, NOR=2-5 AU/L resin |

(continued)

| Process Feature | Exemplary Process |
|---|---|
| UF/DF1 | • Bulk pool pH adjustment<br>• Addition of Polysorbate-20 at 0.005% (v/v) to final DS prior to frozen storage |

**Example 2. Exemplary Formulation**

Compositions comprising purified ASA protein were prepared as described herein. Table 4 provides characteristic features of a composition comprising purified ASA protein when prepared according to an exemplary process as described herein. In embodiments, a composition comprising purified recombinant ASA protein is characterized by one or more of the features described in Table 4. In embodiments, where a numerical range is provided, each boundary of the exemplified range can vary by about 1% to about 20% (e.g., about 1% to about 15%, about 1% to about 10%, about 1% to about 7.5%, about 1% to about 5%, or about 1% to about 2.5%).

**Table 4. Exemplary Composition Comprising Recombinant ASA Drug Substance (DS)**

| Feature | Example 2 |
|---|---|
| Process | Exemplary Process |
| # Lots/Scale | 2 / 200L |
| Specific Activity | 77-89 U/mg |
| Host Cell Protein (Wester) | • No HCP bands with intensity greater than the intensity of the ~ 15kDa HCP band in the assay control<br>• No more than three HCP bands detected |
| Size Exclusion HPLC | 98.8-98.9% Main Peak (1.1-1.2% HMW) |
| Reversed Phase HPLC % Formylglycine (FG) | 99.3-99.7% Main Peak<br>-- |
| SDS Page | No new bands with intensity greater than the 1% assay control |
| Glycan Map | 20.1-20.3% Neutral recombinant ASA protein |
| | 38.9-40.0% Sialic acid recombinant ASA protein |
| | 26.7-27.9% M6P recombinant ASA protein |
| | 4.9-5.0% Capped M6P recombinant ASA protein |
| | 8.1-8.3% Hybrid recombinant ASA protein |

**Example 3: Comparability Studies**

A nonclinical comparability program as an evaluation of the activity, safety and pharmacokinetics of process B rhASA compared with process A rhASA in a nonclinical comparability program (Table 5). All doses were administered intrathecally. Pharmacodynamic comparability was evaluated in immunotolerant MLD mice, using immunohistochemical staining of lysosomal-associated membrane protein-1 (LAMP-1). Pharmacokinetic comparability was assessed in juvenile cynomolgus monkeys dosed once with 6.0 mg (equivalent to 100 mg/kg of brain weight) process A or process B rhASA. Biodistribution was compared by quantitative whole-body autoradiography in rats. Potential toxicity of process B rhASA was evaluated by repeated rhASA administration at doses of 18.6 mg in juvenile cynomolgus monkeys.

**Table 5.**

| Quality attributes of process B vs process A rhASA | Potential biological implications | Nonclinical endpoints | Nonclinical studies |
|---|---|---|---|
| Increased purity | No difference in toxicity expected | Toxicity | 11-week, repeated-dose toxicology study in juvenile cynomolgus monkeys |
| Increased M6P levels | Higher cell/tissue uptake | Toxicity | 11-week, repeated-dose toxicology study in juvenile cynomolgus monkeys |
| | | Biodistribution | Rat QWBA with tissue AUC |
| | | Activity | Multiple endpoint pharmacodynamics study in immunotolerant MLD mice |
| Increased sialic acid levels | Higher serum exposure; minimal impact expected on CSF clearance | Serum and CSF pharmacokinetics | Pharmacokinetic crossover study in juvenile cynomolgus monkeys |

AUC, area under the concentration-time curve; CSF, cerebrospinal fluid; M6P, mannose-6-phosphate; MLD, metachromatic leukodystrophy; QWBA, quantitative whole-body autoradiography; rhASA, recombinant human arylsulfatase A.

***Pharmacodynamic Comparability***

Immunotolerant MLD mice are ASA knockouts (ASA-/-) that have a stably integrated transgene coding for an inactive variant of human ASA, making them immunologically tolerant to injected rhASA. Animals were assigned to one of six groups: untreated (n = 6); vehicle (154 mM NaCl, 0.005% polysorbate 20, pH 6.0, n = 4); process A rhASA at 0.04 mg (n = 9) and 0.21 mg (n = 10); process B rhASA at 0.04 mg (n = 10); and 0.21 mg (n = 10). Animals were dosed intrathecally on days 1, 8, 15, and 21 or 22. A group of seven untreated C57/B16 mice served as wild-type controls. Animals were sacrificed 24 hours after their final dose of rhASA.

Histological analysis involved immunohistochemical staining of lysosomal-associated membrane protein-1 (LAMP-1), a lysosomal protein marker used for the detection of lysosomal storage diseases and as an indicator of disease state. LAMP-1 immunostaining of mouse brain and spinal cord was performed in 5 $\mu$m paraffin sections using rabbit polyclonal anti- LAMP-1 antibody (Abcam, Cat # ab24170, Lot # ER127402-2, 1:400, Cambridge, MA, USA) and Bond™ Polymer Refine Detection Kit (Leica, Cat# DS9800, Buffalo Grove, IL, USA). LAMP-1 signal was obtained by staining with 3, 3-diaminobenzidine and followed by counterstaining with hematoxylin. The primary antibody was replaced by isotype IgG for the negative control. Digital images of LAMP-1-stained slides were created and analyzed using an Aperio scanner with ImageScope software (Leica Microsystems Inc., Buffalo Grove, IL, USA). The relative staining positivity was obtained using the following equation: positivity (%) = (LAMP-1-positive pixel number/total pixel number) $\times$ 100. Statistical comparison of the vehicle and untreated animals was performed for each tissue using a Mann-Whitney test. Based on this evaluation (p > 0.01 between groups for all tissues), data were combined into a single group (hereafter referred to as control). Data were normalized to the control group and statistical comparisons were performed using a one-way analysis of variance with Tukey's multiple comparison test. Comparisons with p < 0.01 were deemed statistically significant.

The specific activities for process A and process B rhASA were 89 U/mg and 106 U/mg, respectively, which were both well within the target range for the assay. The comparison of the two materials indicated that the greatest changes were in the M6P and sialic acid contents, and the specific activities were similar.

As shown in Figure 1A-1F, LAMP-1 staining in immunotolerant MLD mice was conducted in mice for the spinal cord, cerebellar white matter and fimbria, and for the cerebral peduncle, cerebral cortex and striatum. Regions shown to be affected by treatment with process A material from previous studies were chosen for evaluation. No statistically significant differences in LAMP-1 staining in the spinal cord, cerebellar white matter and fimbria were observed between animals dosed with process A versus process B rhASA. Similarly, no statistical differences in staining were observed between process A and process B rhASA in the cerebral peduncle, but differences were observed in the cerebral cortex and striatum. These differences were considered to be sporadic and not biologically relevant because statistical significance was reached for only one of the two doses tested.

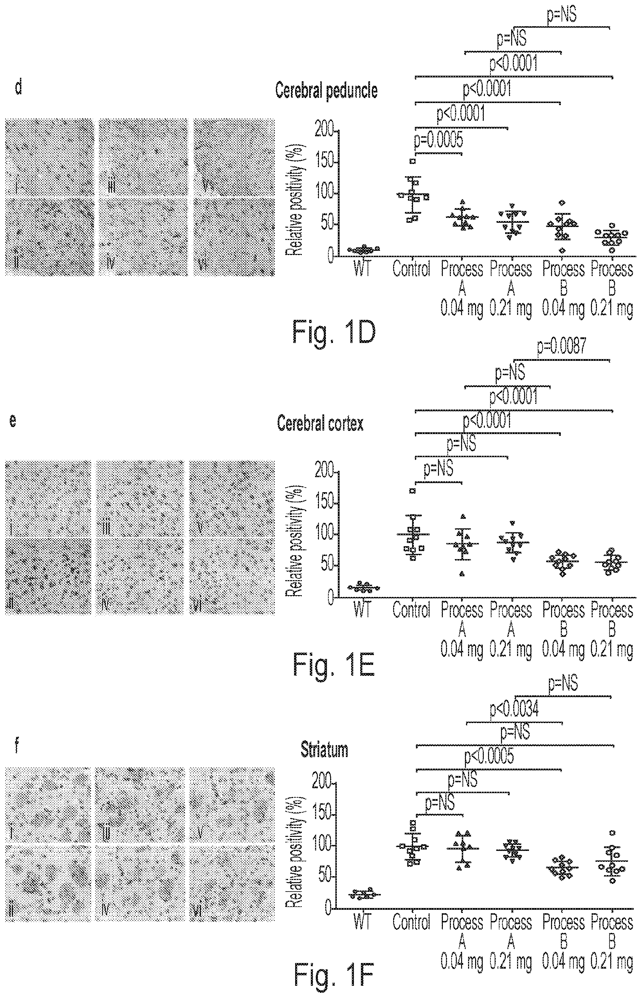

Compared with control animals, there were statistically significant reductions in LAMP-1 staining with both process A and process B rhASA in the spinal cord (0.21 mg dose groups, p < 0.0001; Fig. 1A), in the cerebellar white matter (0.04 mg and 0.21 mg dose groups, p < 0.01; Fig. 1B), in the cerebral peduncle (0.04 mg and 0.21 mg dose groups; Fig. 1D) and in fimbria (0.04 mg dose groups, p < 0.01; Fig. 1C). Statistically significant reductions in LAMP-1 staining also occurred with process B rhASA in the cerebral cortex (0.04 mg and 0.21 mg dose groups, p ≤ 0.0001; Fig. 1E) and in the striatum (0.04 mg dose group, p = 0.0005; Fig. 1F).

**_Pharmacokinetic Comparability_**

In phase 1, animals were randomized based on body weight to either process A rhASA 6.0 mg (n = 6 [3 male, 3 female]) or process B rhASA 6.0 mg (n = 6 [3 male, 3 female]) groups. An intrathecal lumbar catheter was implanted surgically following pre-treatment with subcutaneous atropine sulfate (0.01 mg/kg), followed by anesthesia under intramuscular ketamine hydrochloride (8 mg/kg) and approximately 1 L/min of oxygen and 2% isoflurane. Doses of rhASA were administered via the implanted intrathecal lumbar catheter at a dose volume of 1 mL. The 6.0 mg dose was equivalent to 100 mg/kg of brain weight when normalized to a brain weight of 60 g in the cynomolgus monkey. This dose was based on the highest dose of rhASA that was administered in the phase 1/2 clinical study in children with late-infantile MLD. In that clinical study, the highest dose administered was 100 mg, equivalent to 100 mg/kg of brain weight when normalized to a brain weight of 1 kg (determined for the age group of the study participants.

In phase 2, which started after a washout period of at least 8 days, animals were dosed with process B rhASA if they had been dosed with process A rhASA in phase 1 and, conversely, animals were dosed with process A rhASA if they had been dosed with process B rhASA in phase 1. The animals were humanely euthanized up to 24 hours after the last dose of rhASA.

Body weights, food consumption and clinical parameters were monitored, and blood and CSF samples were collected pre-dose and at pre-specified intervals post-dose. Concentrations of rhASA in serum and CSF were measured using an enzyme-linked immunosorbent assay. Quantitation of the levels of rhASA in the serum and CSF of juvenile cynomolgus monkeys was achieved using GLP-validated ELISA assay methods. Microtiter plates were coated with a rabbit-derived anti-rhASA polyclonal antibody (SH040). After incubation with samples and standards, the microtiter plates were washed and horseradish peroxidase-conjugated anti-ASA mouse monoclonal antibody (clone 19-16-3) was added to all wells. The lower limit of quantification for monkey serum was 39.1 ng/mL and for CSF was 19.5 ng/mL, and the upper limit of quantification for each method was 1250 ng/mL.

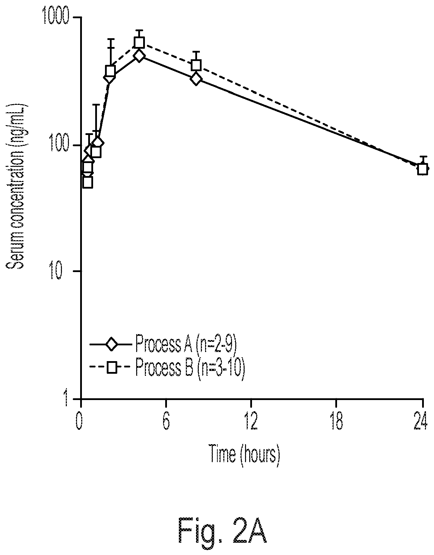

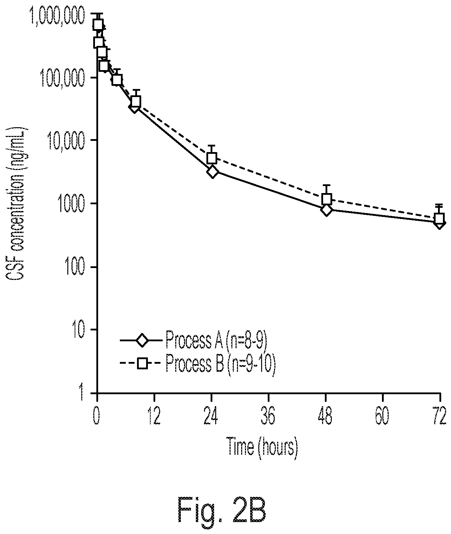

Concentration-time curves of rhASA in serum (Fig. 2A) and CSF (Fig. 2B) after a single 6.0 mg intrathecal dose in juvenile cynomolgus monkeys were similar when using process A and process B rhASA. Process A and process B rhASA were similar in terms of their pharmacokinetic parameters, including the maximum plasma concentration (Cmax), exposure (area under the concentration-time curve from time 0 to the last measurement [AUClast]) and clearance (Table 6).

**Table 6.** Mean cerebrospinal fluid and serum pharmacokinetic parameters in juvenile cynomolgus monkeys following intrathecal lumbar administration of rhASA 6.0 mg manufactured using process A or process B.

| Pharmacokinetic parameter | Process A | | | Process B | | |
|---|---|---|---|---|---|---|
| | Mean | SD | n | Mean | SD | n |
| Cerebrospinal fluid | | | | | | |
| $\lambda z$, L/h | 0.093 | 0.047 | 7 | 0.131 | 0.133 | 8 |
| $t_{1/2}$, h | 10.3 | 7.9 | 7 | 7.8 | 3.3 | 8 |
| $T_{max}$, h | 0.08 | 0.00 | 9 | 0.12 | 0.07 | 10 |
| $C_{max}$, ng/mL | 701,949 | 216,022 | 9 | 633,618 | 224,328 | 10 |
| $AUC_{last}$, h·ng/mL | 1,458,548 | 340,241 | 9 | 1,449,675 | 391,357 | 10 |
| $AUC_{inf}$, h·ng/mL | 1,498,802 | 382,825 | 7 | 1,545,927 | 389,083 | 8 |
| Vz, mL | 67.8 | 62.9 | 7 | 46.3 | 23.2 | 8 |
| CL, mL/h | 4.2 | 1.1 | 7 | 4.1 | 1.2 | 8 |
| $MRT_{inf}$, h | 5.0 | 1.3 | 7 | 5.6 | 1.9 | 8 |
| Serum | | | | | | |
| $T_{max}$, h | 4 | 2 | 9 | 4 | 1 | 10 |
| $C_{max}$, ng/mL | 558 | 145 | 9 | 647 | 190 | 10 |

(continued)

| Serum | | | | | | |
|---|---|---|---|---|---|---|
| AUC$_{last}$, h·ng/mL | 5,112 | 2,076 | 9 | 5,522 | 2,659 | 10 |

$\lambda$z, t½, Vz, CL and MRTinf could not be determined in serum samples.

AUCinf, area under the concentration-time curve from time 0 to infinity; AUClast, area under the concentration-time curve from time 0 to the last measurement; CL, clearance; Cmax, maximum plasma concentration; $\lambda$z, terminal rate constant; h, hour; MRTinf, mean residence time to infinity; n, number; ND, not detected; t½, terminal elimination phase half-life; rhASA, recombinant human arylsulfatase A; SD, standard deviation; Tmax, time to maximum plasma concentration; Vz, volume of distribution.

***Biodistribution Comparability***

The study used quantitative whole-body autoradiography (QWBA) and gamma counting after a single intrathecal dose of [125I]-rhASA. To assess tissue distribution, 28 rats implanted with intrathecal ports were randomized so that each received a dose of [125I]-rhASA 0.62 mg manufactured using either process A (14 animals) or process B (14 animals). Two rats from each dose group were euthanized at each of seven time points after dosing (approximately 1, 4, 12, 24, 48, 96 and 168 hours post-dose) and were processed for QWBA, with radioconcentration determined for selected tissues. Blood samples were collected from isoflurane anesthetized animals via cardiac puncture before euthanasia. To assess pharmacokinetic parameters, aliquots of whole blood, plasma and tissue were analyzed for total radioactivity using gamma counting. To assess rhASA excretion patterns, six male Sprague Dawley rats were assigned to a group receiving [125I]-rhASA 0.62 mg manufactured using either process A (3 animals) or process B (3 animals). Rats were housed in individual metabolism cages for separate collection of urine and feces during the 168 hours following administration of [125I]-rhASA.

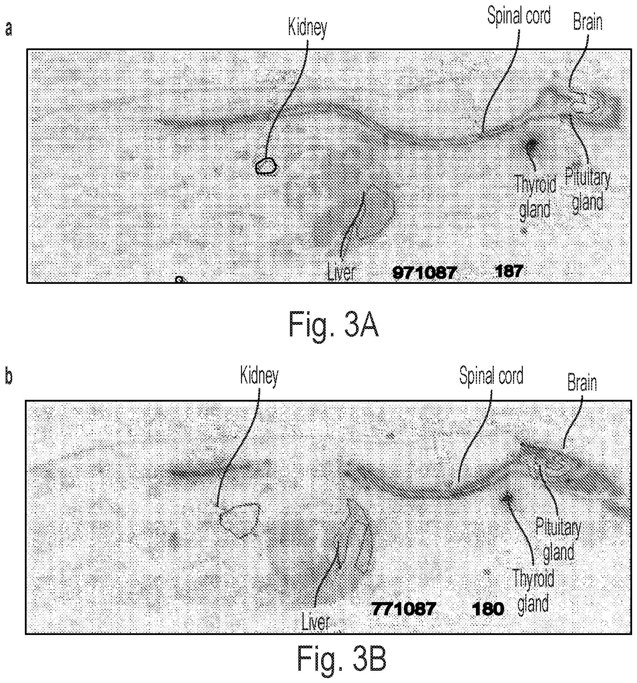

Trichloroacetic acid precipitation of plasma samples indicated that approximately 33% of 125I was unbound to rhASA. Representative whole body autoradioluminograms showed distribution of process A (Fig. 3A) and process B (Fig. 3B) [125I]-rhASA in male Sprague Dawley rats 4 hours after administration of a single intrathecal 0.62 mg dose. Cmax and AUClast for process A and process B rhASA, and the ratios of process B rhASA to process A rhASA in different tissues are shown in Table 6. The highest calculated concentration (Cmax) of [125I]-rhASA was found in the thyroid, likely due to the uptake of unbound iodine. This was followed by the pituitary, spinal cord and liver, which all had similar levels (Table 7). The lowest concentrations of [125I]-rhASA occurred in fat, testes, muscle and eyes (Table 7). When inspected visually, the apparent concentration in the spinal cord was very low relative to the concentration in the neighboring CSF and meninges, suggesting that the calculated values in the spinal cord may have been overestimated. The close proximity to regions with a high concentration of 125I and small quantitation area may have contributed to the overestimation of spinal cord values.

**Table 7.** Pharmacokinetic parameters of rhASA equivalents following a single intrathecal 0.62 mg dose of process A or process B [125I]-rhASA in male Sprague Dawley rats.

| Tissue | C$_{max}$, ng Eq/g | | | AUC$_{last}$, ng Eq·h/g | | |
|---|---|---|---|---|---|---|
| | **Process A** | **Process B** | **Ratio B to A** | **Process A** | **Process B** | **Ratio B to A** |
| Plasma | 1,219 | 1,471 | **1.21** | 27,600 | 35,200 | **1.28** |
| Whole blood | 989 | 1,038 | **1.05** | 21,300 | 25,900 | **1.22** |
| Adrenal gland | 2,484 | 965 | **0.39** | 37,200 | 81,600 | **2.19** |
| Bone marrow (femur) | 1,261 | 1,036 | **0.82** | 84,900 | 81,100 | **0.96** |
| Bone (femur) | 449 | 594 | **1.32** | 43,900 | 36,300 | **0.83** |
| Brain | 1,439 | 2,310 | **1.61** | 93,800 | 135,000 | **1.44** |
| Eye | 342 | 349 | **1.02** | 20,800 | 12,400 | **0.60** |
| Fat | 223 | 186 | **0.83** | 13,000 | 7890 | **0.61** |
| Harderian gland | 404 | 383 | **0.95** | 31,800 | 18,300 | **0.58** |
| Heart | 495 | 547 | **1.11** | 10,500 | 10,600 | **1.01** |
| Kidney | 3,690 | 867 | **0.23** | 36,500 | 53,700 | **1.47** |
| Kidney (cortex) | 2,586 | 914 | **0.35** | 42,400 | 60,700 | **1.43** |
| Kidney (medulla) | 3,610 | 905 | **0.25** | 31,500 | 46,900 | **1.49** |
| Large intestine | 779 | 780 | **1.00** | 27,400 | 33,200 | **1.21** |

(continued)

| Tissue | $C_{max}$, ng Eq/g | | | $AUC_{last}$, ng Eq·h/g | | |
|---|---|---|---|---|---|---|
| | Process A | Process B | Ratio B to A | Process A | Process B | Ratio B to A |
| Liver | 4,452 | 4,484 | **1.01** | 226,000 | 325,000 | **1.44** |
| Lung | 654 | 1,005 | **1.54** | 32,900 | 26,400 | **0.80** |
| Muscle (femoral) | 290 | 344 | **1.19** | 24,800 | 17,100 | **0.69** |
| Pancreas | 873 | 561 | **0.64** | 17,000 | 13,400 | **0.79** |
| Pituitary gland | 6,869 | 6,689 | **0.97** | 348,000 | 527,000 | **1.51** |
| Prostate | 650 | 534 | **0.82** | 16,300 | 19,800 | **1.21** |
| Skin | 672 | 588 | **0.88** | 26,000 | 59,100 | **2.27** |
| Small intestine | 1,107 | 858 | **0.78** | 18,200 | 25,500 | **1.40** |
| Spinal cord | 5,955 | 8,933 | **1.50** | 452,000 | 344,000 | **0.76** |
| Spleen | 1,625 | 1,501 | **0.92** | 70,300 | 91,200 | **1.30** |
| Stomach | 2,258 | 1,291 | **0.57** | 43,600 | 63,700 | **1.46** |
| Testis | 272 | 278 | **1.02** | 18,600 | 10,200 | ***0.55*** |
| Thymus | 492 | 426 | **0.87** | 7,900 | 15,000 | **1.90** |

AUClast, area under the concentration-time curve from time 0 to the last measurement; Cmax, maximum plasma concentration; rhASA, recombinant human arylsulfatase A.

Cmax values for process B rhASA were approximately 1.5-fold higher than those of process A [125I]-rhASA for brain, spinal cord and lung (highest ratios), and approximately 0.2-0.6-fold lower than those of process A [125I]-rhASA for kidney, adrenal gland, stomach and pancreas (lowest ratios; Table 5). Differences in exposure (AUClast) were less than two-fold for all tissues except skin and adrenal gland, which had ratios of 2.27 and 2.19, respectively, for process B to process A [125I]-rhASA (Table 5). The terminal elimination phase half-life in plasma was 37.2 hours with process A and 42.5 hours with process B [125I]-rhASA.

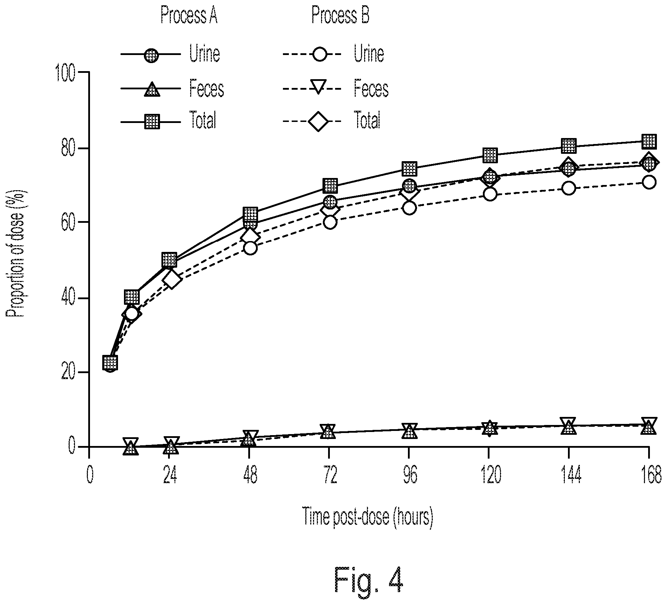

Excretion profiles in urine and feces were assessed as shown in Figure 4. Excretion patterns were similar for both process A and process B [125I]-rhASA. Most process A and process B product was excreted in the urine (76% and 71%, respectively), indicating that rhASA is systemically cleared primarily via renal elimination, and about 6% was excreted in the feces for both products. The proportion of the administered dose recovered in the carcass was 6% for process A product and 8% for process B product, with the majority located in the thyroid, CSF and meninges. The total recovery (all excreta and carcass combined) was 90% and 83% for animals dosed with process A or process B [125I]-rhASA, respectively.

### *Toxicology*

An intrathecal port catheter was implanted surgically following pre-treatment with meloxicam (0.2 mg/kg), followed by ketamine hydrochloride (5 mg/kg) and medetomidine (0.06 mg/kg). Doses of rhASA were administered by slow-bolus delivery using the intrathecal port catheter system. Animals were dosed every 2 weeks for 11 weeks (six doses in total) with either process B rhASA 18.6 mg (n = 8 [4 male, 4 female]) or vehicle control (NaCl 154 mM, 0.005% polysorbate 20, pH 6.0; n = 8 [4 male, 4 female]) at a dose volume of 0.6 mL. The maximum feasible dose of rhASA of 18.6 mg was used in this study and was based on the highest tolerable volume in the juvenile cynomolgus monkey (0.6 mL) and on the concentration of the process B rhASA formulation (31.0 mg/mL).

Assessments included clinical examinations (body weight; food consumption; physical, cardiovascular [electrocardiogram and blood pressure], neurological and ophthalmological evaluations) and pathological examinations (serum chemistry; hematology; coagulation; urinalysis; CSF cell count and CSF chemistry). Antigenicity of process B rhASA was assessed in serum and CSF. Physical and neurological examinations were performed on unsedated animals twice during the pre-dose phase (before surgery and ≥5 days after surgery), and once during weeks 1, 5 and 9 of the dosing phase (approximately 24 hours after dosing) as well as once before necropsy. Electrocardiography investigations were performed on non-anesthetized, temporarily restrained animals during the pre-dose phase (after surgery) and in week 11 of the dosing phase (close to dosing and 3 hours post-dose). All animals were humanely euthanized 24 hours after the final dose. Complete sets of tissues were collected, but only the target tissues identified in the original study with process A (i.e. spinal cord and dorsal root ganglia) were subjected to histopathological evaluation.

The intrathecal administration of process B rhASA was well tolerated by juvenile cynomolgus monkeys, with no drug-related effects on clinical, neurological or pathological examinations.

The intrathecal administration of process B rhASA at 18.6 mg every 2 weeks for 11 weeks to juvenile cynomolgus monkeys (six doses in total) was the no observable adverse effect level. Administration of process B rhASA was associated with infiltrates with eosinophils in the following regions: meningeal areas around the brain and spinal cord; perivascular areas in the brain and spinal cord; pericanalicular (adjacent to the central canal) areas in the spinal cord; and perineurium/epineurium areas around the spinal nerve roots. None of these process B rhASA-related changes were of a severity that would reasonably be expected to alter the function of the nervous system and, based on morphology alone, none of these process B rhASA-related changes were considered to be adverse effects. The presence of the infiltrates in the spinal cord was consistent with what would be expected in an animal with a protein administered to the intrathecal space.

Mean concentration of process B rhASA was 544 ng/mL in serum and 2185 ng/mL in CSF on day 2; at week 11, it was 37 ng/mL in serum and 171 ng/mL in CSF **(Figure 2)**. All rhASA-dosed animals developed anti-drug antibodies by week 10 with higher anti-drug antibody levels observed in serum than in the CSF (mean: 391,462 ng/mL vs 7,383 ng/mL, respectively); anti-drug antibodies did not affect rhASA exposure to target tissues as confirmed by immunohistochemistry and tissue ELISA (data not shown).

**Example 4: Pharmacokinetic/pharmacodynamic profiles of recombinant human arylsulfatase A in patients with MLD**

Following intrathecal administration in patients with MLD, the pharmacokinetic/pharmacodynamic (PK/PD) profiles of rhASA were investigated. The effect of rhASA on sulfatide levels in cerebrospinal fluid (CSF), and changes in Gross Motor Function Measure-88 (GMFM-88) total score in patients with MLD receiving rhASA were used to assess its clinical effects and determine the optimum dosing regimen.

All patients received rhASA every other week for up to 338 weeks via an intrathecal drug delivery device (IDDD). Patients in cohorts 1, 2 and 3 were less than 12 years of age at enrollment in the original dose-escalation study and received 10, 30 and 100 mg of rhASA, respectively. Patients in cohort 4 were less than 8 years of age at enrollment and received 100 mg of rhASA that had been manufactured using a revised process. All patients received drug product formulated at 30 mg/ml rhASA in an aqueous isotonic solution containing 154 mM sodium chloride and 0.005% polysorbate 20 at pH 6.0 delivered by IT administration via an in-dwelling IDDD. Results of several efficacy measures indicate that rhASA may be associated with slowed disease progression.

Patients who completed the dose-escalation study could continue to receive rhASA every other week via IDDD through the extension study. Serum rhASA concentration was assessed following the initial rhASA dose and at week 38 of treatment. Patients in cohort 4 received rhASA that had been manufactured using a revised process. Blood samples were taken ≤ 1 hour before and 0.5, 1, 2, 4, 8, 12, 24 and 48 hours following IT injection. rhASA concentration in CSF was assessed every 2 or 4 weeks during the dose-escalation study, and quarterly through weeks 52-104 and biannually through weeks 104-338 in the extension study. GMFM-88 observations were assessed at weeks 0, 16, 24 and 40 in the dose-escalation study, and quarterly through weeks 52-104 and biannually through weeks 104-338 in the extension study.

Assessments included treatment-emergent adverse events (AEs); serum chemistries; 12-lead electrocardiogram (ECG) findings; vital signs; physical examinations; cell counts, glucose and protein levels in cerebrospinal fluid (CSF); and anti-rhASA antibodies in CSF and serum. AEs were defined as all adverse events that occurred between the time of the first dose of investigational product or device implant surgery (whichever occurred earlier) and the last follow-up date in the study. The follow-up evaluation occurred 4 weeks after the last rhASA injection or 2 weeks after removal of the last IDDD, whichever occurred later for patients who did not enroll in the extension study. For individuals who enrolled in the extension, the follow-up evaluation occurred at the end-of-study visit (week 40).

Serum and CSF samples were first screened for anti-drug antibodies (ADA) using an electrochemiluminescence bridging immunoassay (Meso Scale Diagnostics, LLC, Rockville, MD) that utilized biotinylated and sulfo-tagged rhASA for capture and detection, respectively. ADA-positive samples were confirmed by ligand competitive binding using the same bridging immunoassay and subsequently assessed for antibody titer and neutralizing activity using an arylsulfatase A activity assay with 4-nitrocatechol sulfate as the substrate.

Pharmacokinetic evaluations of rhASA in CSF and serum after administration, and change from baseline in motor function were assessed. Serum and CSF samples were collected from patients at weeks 0 (first dose) and 38 (last dose). rhASA concentration was determined using validated enzyme-linked immunosorbent assay methods. Change from baseline in motor function was evaluated using the GMFM-88 total score (0-100%). Additional endpoints included: CSF sulfatide and lysosulfatide levels, assessed by liquid chromatography with tandem mass spectrometric detection; N-acetyl aspartate (NAA)/creatine and choline/creatine levels in the deep white and gray matter of the brain, assessed by proton magnetic resonance spectroscopy (MRS), change from baseline in the total MLD severity score based on brain magnetic resonance imaging (MRI); and peripheral nerve function, evaluated via electroneurography. The upper limits of normal for CSF sulfatide and lysosulfatide were defined as 0.113 μg/mL and 0.0277 ng/mL, respectively, based on the highest concentration observed in samples from 60 children who did not have leukodystrophy but who had undergone a spinal tap (and therefore may have other neurological conditions). Each patient had a serially measured MRI of the brain. Based on a visual scoring method of the MRI, a total MLD severity score (range: 0-34) was calculated for each individual at each time point where higher scores indicated more severe brain involvement. Evaluation of peripheral nerve function via electroneurography was also performed. Nerve conduction velocity, amplitude, distal latency, and F-wave latency were measured and transformed to z-scores if population mean and standard deviation (SD) were available in the appropriate age ranges.

Treatment with rhASA was well tolerated, with no deaths, no discontinuations due to adverse events (AEs), and no life-threatening AEs reported. IT rhASA did not appear to have high immunogenic potential and, although 10 patients developed treatment-induced antibodies in serum, there was no correlation between serum antibody response and AEs.

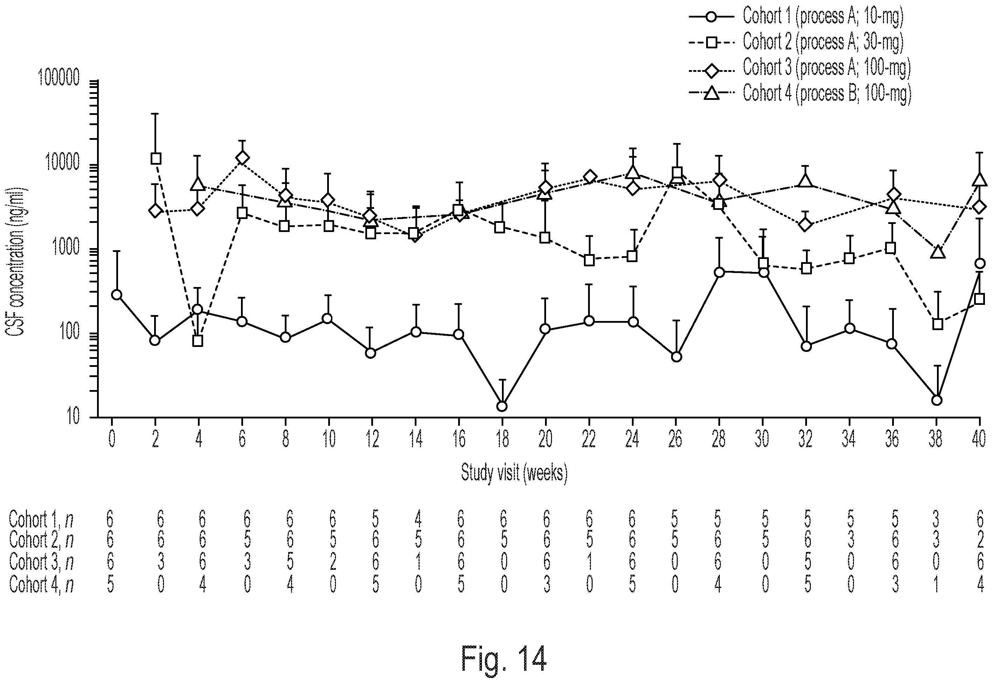

The concentration of rhASA increased slowly in serum following IT administration (mean $t_{max}$ range 5.1-18.2 hours), with the maximum observed serum concentration ($C_{max}$) and area under the serum concentration-time curve increasing in relation to dose. rhASA concentration generally increased in a dose-dependent manner in CSF, with measurable concentrations detected at each visit before dosing, suggesting that rhASA persists in the CSF throughout the 2 weeks between doses. The mean CSF concentrations of rhASA by dose cohort and treatment week are presented in Figure 14. Pharmacokinetic results were similar in patients who received 100-mg rhASA manufactured using either process A or process B.

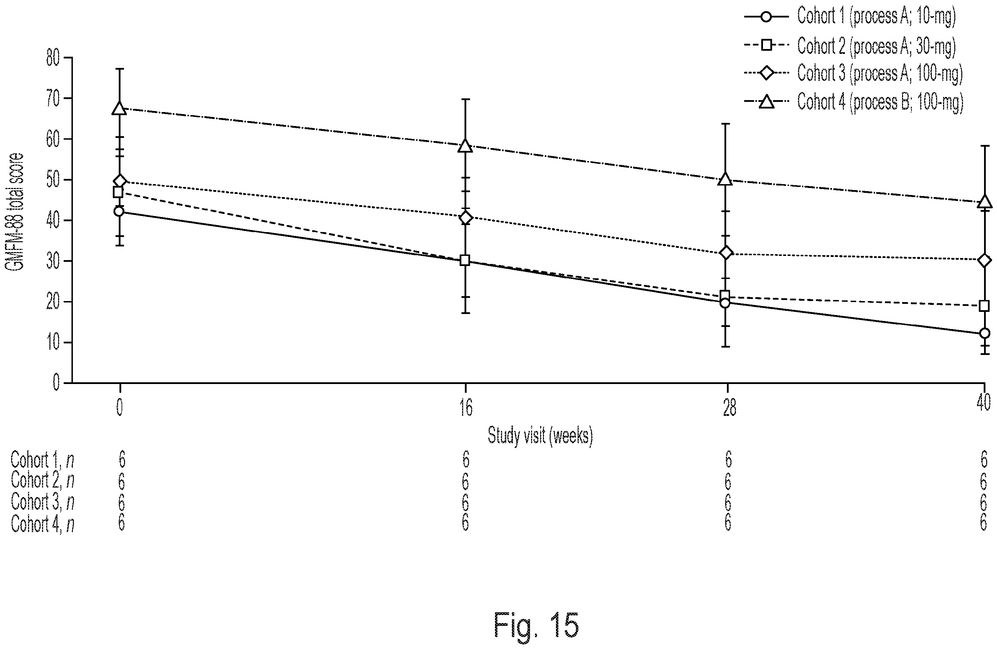

Decreases in mean GMFM-88 total score from baseline were observed after rhASA treatment in each of the four cohorts (Figure 15). The mean (SD) decline at week 40 in GMFM-88 total score compared with baseline in the two 100-mg cohorts (cohorts 3 and 4) was similar (-19.0 [16.12] versus -22.6 [25.48], respectively). However, these decreases were less pronounced than those observed in the 10-mg and 30-mg cohorts (cohorts 1 and 2; -29.2 [24.45] and -27.7 [17.76], respectively). The change from baseline in GMFM-88 total scores over time are summarized in Table 8. The decreases in gross motor function observed in all patients, showed a trend for smaller declines as rhASA dose levels increased. Patients receiving 100-mg rhASA (cohort 3; process A), in which the MLD severity score remained stable.

**Table 8.**

Change from baseline in GMFM-88 total scores

| Visit | Manufacturing process A | | | Manufacturing process B |
| | Cohort 1 rhASA 10-mg EOW (*n* = 6) | Cohort 2 rhASA 30-mg EOW (*n* = 6) | Cohort 3 rhASA 100-mg EOW (*n* = 6) | Cohort 4 rhASA 100-mg EOW (*n* = 6) |
|---|---|---|---|---|
| **Week 16** | | | | |
| *n* | 6 | 6 | 6 | 6 |
| Mean (SD) | -11.8 (25.24) | -17.1 (9.67) | -8.8 (11.37) | -8.8 (14.47) |
| 95% CI | -38.3, 14.6 | -27.3, -7.0 | -20.7, 3.2 | -24.0, 6.4 |
| Median | -1.6 | -16.5 | -9.2 | -8.1 |
| Range | -49.0, 10.0 | -32.0, -7.0 | -24.0, 6.0 | -25.0, 13.0 |
| **Week 28** | | | | |
| *n* | 6 | 6 | 6 | 6 |
| Mean (SD) | -21.8 (22.55) | -25.8 (17.90) | -17.8 (14.13) | -17.1 (18.48) |
| 95% CI | -45.5, 1.9 | -44.6, -7.1 | -32.7, -3.0 | -36.5, 2.2 |
| Median | -26.3 | -22.2 | -20.5 | -15.7 |
| Range | -50.0, 9.0 | -57.0, -7.0 | -34.0, 4.0 | -40.0, 4.0 |
| **Week 40** | | | | |
| *n* | 6 | 6 | 6 | 6 |
| Mean (SD) | -29.2 (24.45) | -27.7 (17.76) | -19.0 (16.12) | -22.6 (25.48) |
| 95% CI | -54.9, -3.5 | -46.4, -9.1 | -35.9, -2.1 | -49.3, 4.2 |
| Median | -36.0 | -23.3 | -26.4 | -31.6 |
| Range | -54.0, 6.0 | -59.0, -6.0 | -32.0, 4.0 | -51.0, 16.0 |

CSF sulfatide levels were assessed as an indicator of drug activity in the CNS. As shown in Figure 16A, mean CSF sulfatide levels fell below the upper limit of normal by study end in cohorts 3 and 4 (0.07 [0.03] and 0.08 [0.03], respectively). Decreases in mean CSF lysosulfatide levels were also observed in all cohorts over time (Figure 16B). Mean CSF lysosulfatide levels were consistently below the upper limit of normal from week 2 in cohort 3, week 4 in cohort 4, and week 12 in cohort 2. This data suggest that rhASA is active in the CNS.

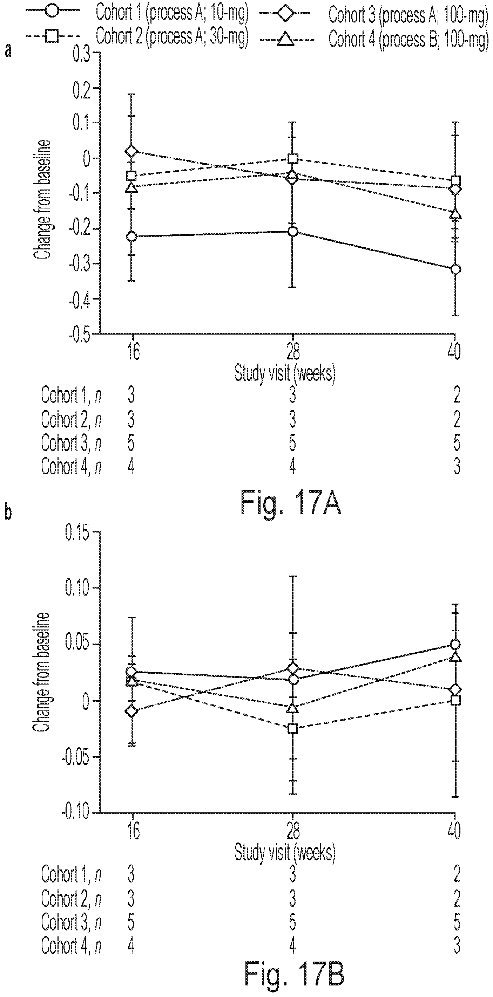

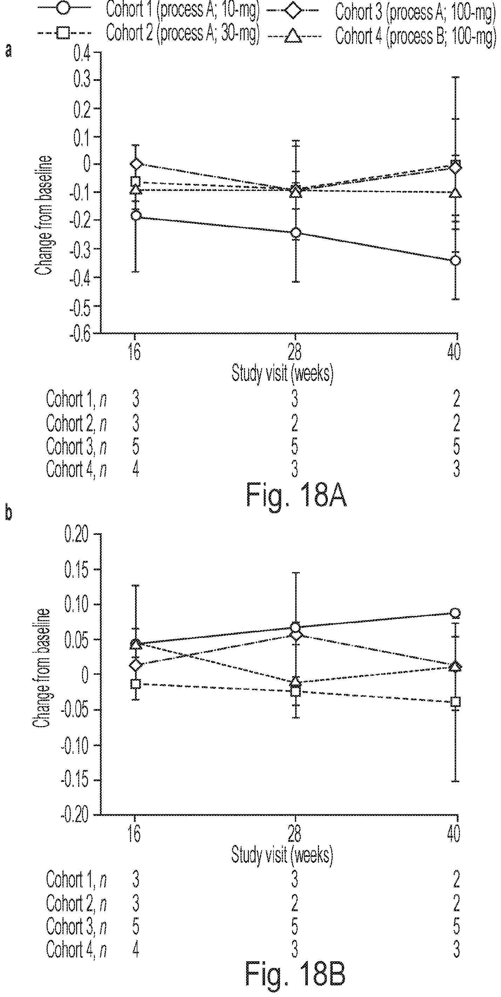

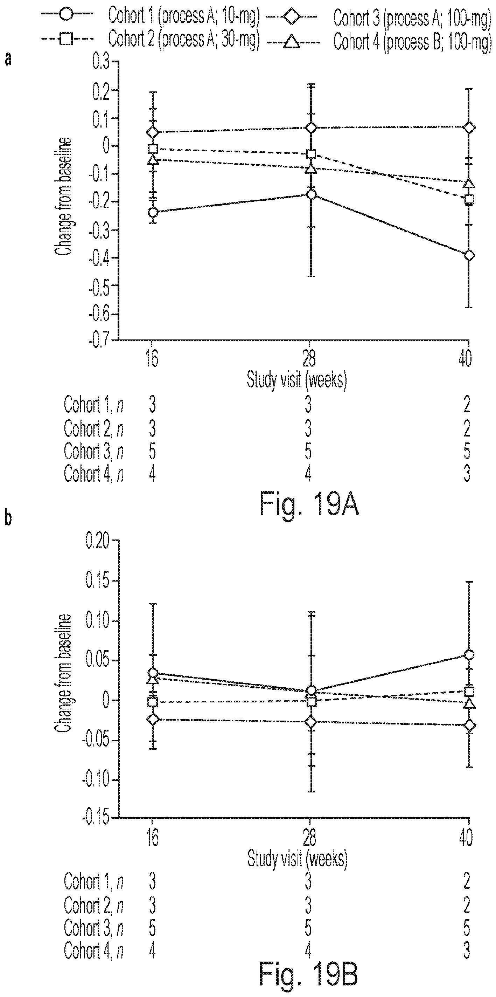

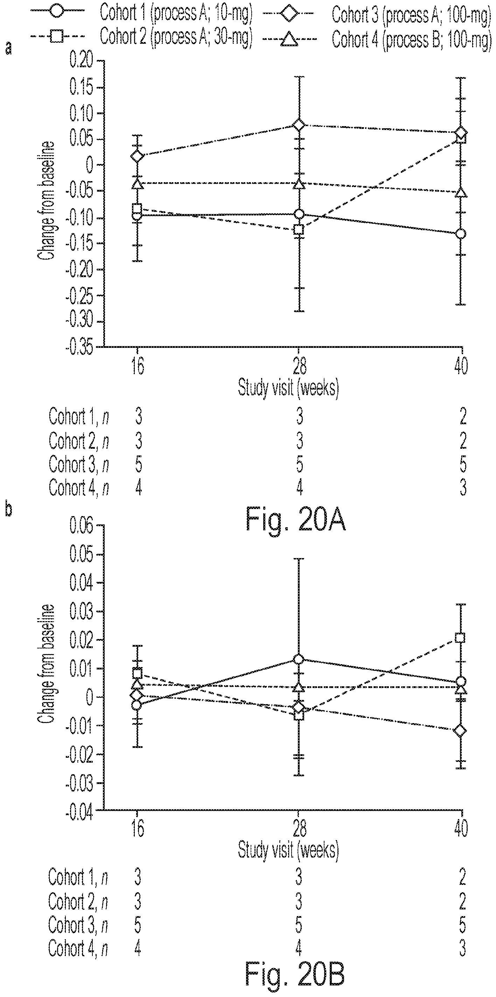

NAA may be used as a surrogate measure of the number and/or density of mature neurons and axons, and in neurological disorders decreased NAA concentration are associated with neuronal and axonal injury and loss. Change in NAA/creatine ratio was assessed as an indicator of MLD severity in patients (Figure 17A). In right frontal white matter, the ratio of NAA/creatine decreased from baseline to study end, although this was less pronounced for patients treated with 30 mg or 100 mg rhASA (cohorts 2-4) compared with those treated with 10 mg (cohort 1) (Figure 17A). Similar results were seen in right frontal-parietal white matter and right parieto-occipital white matter (Figures 18A and 19A). The NAA/creatine ratio and rhASA dose in gray matter is shown in Figure 20A).

The ratio of choline/creatine was also assessed in all cohorts. Choline levels have been shown to be elevated in patients with MLD, and may result from increased membrane turnover associated with demyelination. In right frontal white matter, right frontal-parietal white matter and right parieto-occipital white matter, choline/creatine ratios were consistently increased compared with baseline in patients treated with the lowest dose (10-mg; cohort 1), but there was no clear trend in those treated with 30-mg or 100-mg (cohorts 2-4; Figure 17B, 18B and 19B). In midline occipital gray matter, the choline/creatine ratio was variable in cohorts 1 and 2, but decreased compared with baseline in cohort 3 and remained stable in cohort 4 (Figure 20B).

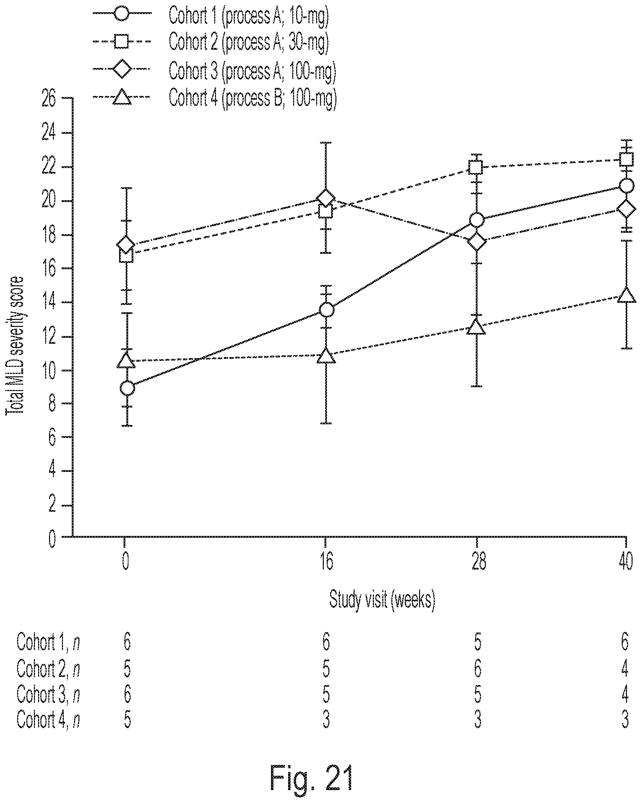

Mean total MLD severity scores increased in patients treated with 10-mg, 30-mg and 100-mg (B) rhASA over time (cohorts 1, 2 and 4), indicating disease progression (Figure 21). The biggest increase from baseline was evident in patients who received 10-mg rhASA (cohort 1; mean change, 11.0). In contrast, mean total MLD severity scores remained stable in patients treated with 100-mg (A) rhASA (cohort 3), possibly indicating disease stabilization in the CNS (Figure 21).

Significant degradation in nerve conduction capability in the median motor thenar was observed at baseline in all cohorts (Table 9). Similar results were also found in sensory nerves (data not shown). Nerve function did not appear to worsen in the four cohorts at later time points.

**Table 9.** Z-scores by visit for electroneurography in median motor thenar.

| | Manufacturing process A | | | Manufacturing Process B |
|---|---|---|---|---|
| | Cohort 1 rhASA 10-mg EOW (n = 6) | Cohort 2 rhASA 30-mg EOW (n = 6) | Cohort 3 rhASA 100-mg EOW (n = 6) | Cohort 4 rhASA 100-mg EOW (n = 6) |
| **Elbow to wrist conduction velocity (m/s)** | | | | |
| **Baseline** | | | | |
| n | 6 | 6 | 6 | 5 |
| Mean (SD) | - 7.9 (0.39) | -6.9 (1.56) | -5.6 (2.78) | -6.7 (2.52) |
| Median | -8.0 | -6.4 | -6.1 | -5.5 |
| Range | -8.4, -7.5 | -9.4, -5.2 | -8.3, -1.9 | -10.5, -4.6 |
| | | | | |
| **Week 16** | | | | |
| n | 5 | 6 | 6 | 4 |
| Mean (SD) | -8.3 (0.42) | -7.3 (1.73) | -5.0 (4.52) | -7.7 (2.19) |
| Median | -8.4 | -7.3 | -5.9 | -7.3 |
| Range | -8.7, -7.9 | -9.4, -4.3 | -10.5, 1.7 | -10.5, -5.5 |
| **Week 28** | | | | |
| n | 5 | 5 | 4 | 5 |
| Mean (SD) | -8.3 (0.33) | -6.3 (2.54) | -6.4 (4.74) | -6.6 (1.36) |
| Median | -8.2 | -7.3 | -7.6 | -7.2 |
| Range | -8.6, -7.9 | -8.5, -2.0 | -10.5, 0.2 | -7.7, -4.6 |
| **Week 40** | | | | |
| n | 3 | 5 | 6 | 5 |
| Mean (SD) | -8.2 (0.19) | -7.1 (1.67) | -5.5 (3.86) | -6.9 (2.54) |
| Median | -8.2 | -7.5 | -6.3 | -6.0 |
| Range | -8.4, -8.1 | -8.5, -4.3 | -10.2, -0.9 | -10.5, -4.0 |

(continued)

| Wrist amplitude baseline to peak (mV) | | | |
|---|---|---|---|
| **Baseline** | | | |
| n | 6 | 5 | 5 | 5 |
| Mean (SD) | -1.6 (0.45) | -1.3 (1.11) | -1.3 (0.29) | -0.9 (0.46) |
| Median | -1.8 | -1.7 | -1.2 | -1.0 |
| Range | -2.0, -0.9 | -2.6, 0.1 | -1.6, -0.8 | -1.5, -0.3 |
| **Week 16** | | | |
| n | 6 | 6 | 6 | 4 |
| Mean (SD) | -1.6 (0.62) | -1.5 (0.43) | -1.1 (0.75) | -1.3 (0.24) |
| Median | -1.9 | -1.4 | -1.5 | -1.3 |
| Range | -2.1, -0.4 | -2.2, -1.1 | -1.6, 0.1 | -1.4, -0.9 |
| **Week 28** | | | |
| n | 5 | 5 | 4 | 5 |
| Mean (SD) | -1.5 (0.79) | -1.4 (0.56) | -1.5 (0.57) | -0.7 (0.46) |
| Median | -1.7 | -1.4 | -1.2 | -0.9 |
| Range | -2.0, -0.1 | -2.2, -0.8 | -2.3, -1.1 | -1.1, 0.1 |
| **Week 40** | | | |
| n | 3 | 5 | 6 | 5 |
| Mean (SD) | -1.5 (0.84) | -1.3 (0.46) | -1.1 (0.43) | -1.0 (0.38) |
| Median | -1.9 | -1.4 | -1.1 | -0.9 |
| Range | -2.2, -0.6 | -1.6, -0.5 | -1.6, -0.5 | -1.4, -0.5 |
| Wrist-APB distal latency (ms) | | | |
| **Baseline** | | | |
| n | 6 | 5 | 5 | 5 |
| Mean (SD) | 15.3 (5.35) | 8.2 (5.69) | 10.3 (9.76) | 5.8 (3.49) |
| Median | 14.9 | 5.9 | 8.0 | 4.7 |
| Range | 8.7, 23.8 | 3.3, 17.5 | -0.8, 23.1 | 3.0, 11.9 |
| **Week 16** | | | |
| n | 6 | 6 | 6 | 4 |
| Mean (SD) | 12.9 (4.26) | 8.1 (4.82) | 8.1 (8.33) | 8.8 (5.35) |
| Median | 12.4 | 6.8 | 6.1 | 7.7 |
| Range | 7.2, 19.8 | 4.0, 17.0 | -0.3, 19.3 | 4.0, 15.9 |
| **Week 28** | | | |
| n | 5 | 5 | 4 | 5 |
| Mean (SD) | 12.9 (4.63) | 7.9 (4.94) | 6.7 (6.54) | 6.8 (4.68) |
| Median | 10.5 | 6.7 | 5.9 | 4.3 |
| Range | 9.4, 20.5 | 4.2, 16.3 | -0.1, 15.0 | 2.4, 13.1 |
| **Week 40** | | | |
| n | 3 | 5 | 6 | 5 |
| Mean (SD) | 13.8 (5.79) | 8.6 (5.66) | 7.4 (7.07) | 7.1 (8.17) |
| Median | 11.0 | 6.1 | 7.0 | 3.8 |
| Range | 10.0, 20.5 | 3.1, 14.9 | -0.8, 17.5 | 2.4, 21.7 |
| F-wave latency (ms) | | | |
| **Baseline** | | | |
| n | 2 | 3 | 2 | 5 |
| Mean (SD) | 40.0 (25.02) | 19.4 (14.99) | 11.1 (17.54) | 14.5 (18.54) |
| Median | 40.0 | 27.8 | 11.1 | 6.3 |

(continued)

| F-wave latency (ms) | | | | |
|---|---|---|---|---|
| **Baseline** Range | 22.4, 57.7 | 2.1, 28.3 | -1.3, 23.5 | 2.8, 47.1 |
| **Week 16** n Mean (SD) | 3 48.0 (20.74) | 3 22.3 (16.67) | 2 4.6 (8.23) | 3 26.2 (22.46) |
| Median Range | 59.5 24.1, 60.4 | 30.2 3.1, 33.5 | 4.6 -1.3, 10.4 | 17.3 9.6, 51.8 |
| **Week 28** n Mean (SD) Median Range | 2 39.2 (21.07) 39.2 24.3, 54.1 | 4 22.6 (19.87) 23.4 2.5, 41.1 | 2 4.4 (8.53) 4.4 -1.6, 10.4 | 2 4.8 (2.12) 4.8 3.3, 6.3 |
| **Week 40** n Mean (SD) Median Range | 1 52.3 (N/A) 52.3 52.3, 52.3 | 5 27.3 (23.40) 23.4 2.6, 60.4 | 2 4.6 (8.80) 4.6 -1.6, 10.8 | 3 6.3 (3.31) 6.3 3.0, 9.6 |

Several studies have also reported changes in metabolite levels in the CNS in patients with neurological disorders, including MLD. For example, decreases in NAA concentration are associated with neuronal and axonal injury and loss, while choline levels have been shown to be elevated in patients with MLD. As expected, we found that the ratio of NAA/creatine generally decreased over time in the brain white matter. However, this was less pronounced with 100-mg doses than with 10-mg or 30-mg, potentially indicating less neuronal damage at the highest dose. In addition, the ratio of choline/creatine in the white matter of the brain was consistently increased compared with baseline in patients treated with the lowest dose (10-mg; cohort 1), but results were more variable in patients treated with higher doses. We did not observe any clear trends in levels of other metabolites including lactate, myo-inositol and glutamine + glutamate in our study. Analyses of metabolite levels may need to be performed in more patients and different areas of the brain to clearly establish the impact of rhASA treatment.

Overall, these findings indicate that IT rhASA treatment may be associated with disease stabilization in the CNS, particularly at a dose of 100-mg EOW. However, the small sample size means that our results should be interpreted with caution. In particular, the number of patients with data available at each time point varied for some parameters and further studies will be needed over a longer period of time and in more patients to establish the long-term efficacy of rhASA in patients with MLD. In addition, there was some variability in the age at enrollment between patients and cohorts, which may have implications for comparison of treatment responses between different dose groups. Similarly, data on metabolite levels obtained via MRS were not normalized to take patient age into account.

Despite these limitations, our findings demonstrate that IT rhASA in children with MLD is generally well tolerated. Although there was a general decline in motor function over time, there was evidence of reduced disease progression in patients who received rhASA, particularly at the highest dose. An extension study is ongoing to evaluate long-term safety, immunogenicity and clinical outcomes associated with rhASA treatment, and a later clinical phase study of rhASA at higher doses will begin recruiting patients soon. It is hoped that results from these studies will help to guide dosing and establish whether rhASA could be considered as a potential treatment for patients with MLD in the future.

**Example 5: Modeling and simulation of recombinant human arylsulfatase A in patients with MLD**

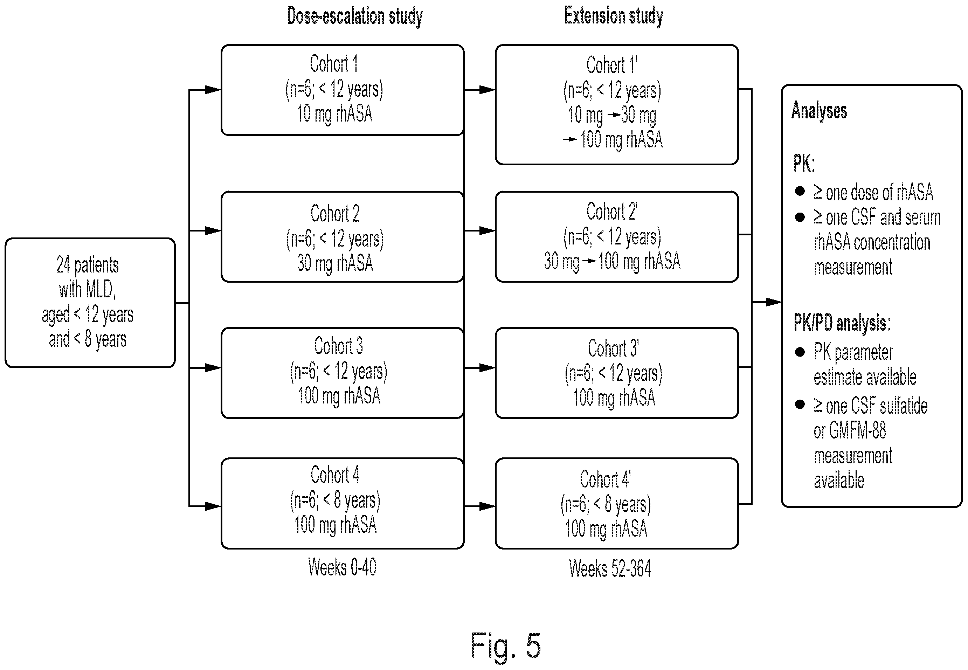

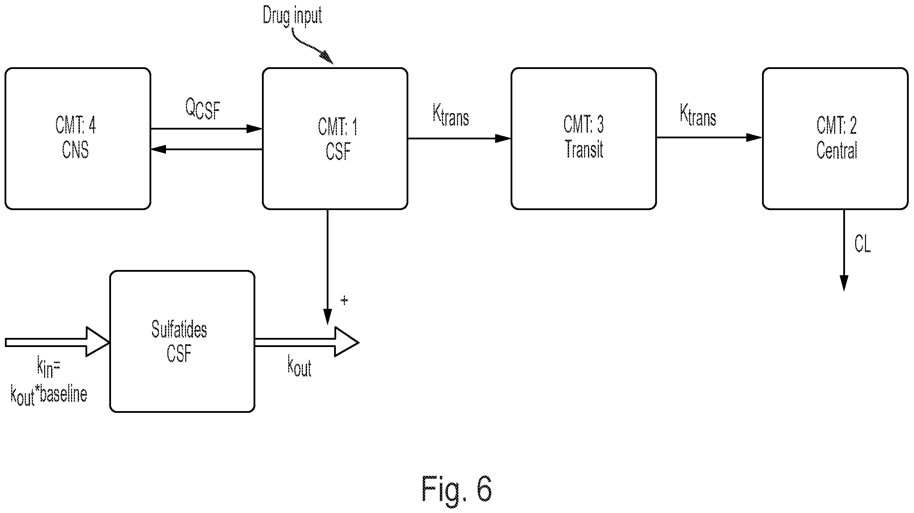

Data from the clinical study described in Example 4 was used for pharmacokinetic (PD) and pharmacodynamic (PD) modeling and simulation of recombinant human arylsulfatase A in patients with MLD. As described in Figure 5, for PK analysis all patients received at least one dose of rhASA and with at least one rhASA concentration measurement in serum or CSF. For PK/PD analysis: all patients with a PK parameter estimate available from the PK analysis and with at least one CSF sulfatide or GMFM-88 total score measurement available. A population PK model based on serum and CSF concentrations of rhASA was developed as shown in Figure 6. Elimination of rhASA was represented by a two compartment model in the central nervous system (CNS), with volumes of distribution, VCNS and VCSF, and intercompartmental clearance, QCSF. A hypothetical transit compartment was used to characterize the clearance of rhASA from the CSF and its delayed appearance in the serum.

PK/PD relationships were modeled including the effect of rhASA on sulfatide concentration measured in CSF and the exposure-response (E-R) relationship of rhASA to GMFM-88 total score. The PK driver for the PK/PD relationship was the patient-specific, time-continuous PK profiles in the CSF and the CNS. PD effects were modelled sequentially and all PK-related parameters were fixed during estimation of PD parameters. The CSF sulfatide measurement time course was characterized using an indirect response PK/PD model, with rhASA modeled as enhancing the elimination of sulfatide. The population PK model-predicted rhASA concentration in the CNS compartment was used as the driver of PD response. The E-R relationship of rhASA exposure to GMFM-88 total score was evaluated using a beta-regression model constrained by a logit link function to restrict the model predictions to lie within the range of 0-100%.

The population PK, PK/PD and E-R models were used to simulate expected rhASA concentration-time profiles and GMFM-88 total scores. 750 patient profiles were simulated; 250 randomly sampled uniformly from each age group. All data assembly, plots and summary tables were prepared using R (v3.3 or higher; Lucent Technologies, Swindon, UK; http://www.rproject.org). The population PK, PK/PD and E-R modelling analyses and simulations were performed using NONMEM software (v7.3 or higher; ICON Development Solutions, Ellicott City, MD, USA).

As shown in Figure 8, four different dosing scenarios were used. Scenario 1 included 100 mg every other week, scenario 2 included 100 mg every week for 12 weeks (initial weekly dosing), then 100 mg every other week, scenario 3 included 150 mg every week for 12 weeks (initial weekly dosing), then 150 mg every other week, and scenario 4: age-adjusted dosing weekly for 12 weeks (initial weekly dosing), then every other week (scenario 4: 80 mg, < 8 months; 120 mg, 8-< 30 months; 150 mg, $\geq$ 30 months). All data assembly, plots and summary tables were prepared using R (v3.3 or higher; Lucent Technologies, Swindon, UK; http://www.rproject.org). 750 patient profiles were simulated; 250 randomly sampled uniformly from each age group. The population PK, PK/PD and E-R modelling analyses and simulations were performed using NONMEM software (v7.3 or higher; ICON Development Solutions, Ellicott City, MD, USA).

Patients (n = 24) were equally distributed across the four cohorts. At baseline, mean patient age was 44.9 (standard deviation [SD], 22.8; range, 19.0-107.0) months and mean weight was 15.4 (SD, 4.14; range, 10.5-24.8) kg.

The fixed effects were estimated with good precision, with the exception of the proportionality coefficients of VCSF (76.1% RSE), VCNS (121% RSE) and Ktrans (40.2% RSE). The precision of the estimated inter-individual variability was poor. The model suggested rapid distribution of rhASA into brain tissue and systemic circulation: median distributive half-life of rhASA in the CNS was 1.02 (range, 0.394-1.66) hours and median half-life of distribution from the CSF to serum was 1.19 (range, 0.555-2.09) hours. However, median terminal half-life of rhASA in the CNS was approximately 20 days (477 [range, 256-1010] hours) (Table 10). The median transit rate constant (expressed as a transit half-life) of 1.19 (range, 0.555-2.09) hours is consistent with CSF physiological turnover (approximately 6 hours).

**Table 10.** Summary statistics of individual PK parameter estimates

| rhASA PK parameter | Mean (SD) | Median (range) |
|---|---|---|
| **CSF** | | |
| Half-life $_{CFS\text{-}serum}$ hours | 1.28 (0.421) | 1.19 (0.555-2.09) |
| Transit rate $_{CSF\text{-}serum}$ 1/hour | 0.61 (0.22) | 0.58 (0.332-1.25) |
| Intercompartmental $0_{CSF}$ L/hour | 0.0028 (0.0002) | 0.0028 (0.0025-0.0032) |
| Distributive half-life $_{CFS}$ hours | 1.06 (0.33) | 1.02 (0.394-1.66) |
| Terminal half-life $_{CSF}$ hours | 499 (176) | 477 (256-1010) |
| Distribution volume $_{CFS}$ L | 1.65 (0.49) | 1.58 (0.963-3.17) |
| Distribution volume $_{CFS}$ L | 0.031 (0.018) | 0.027 (0.0063-0.099) |
| **Serum** | | |
| Clearance systemic L/hour | 3.04 (0.60) | 2.80 (2.30-4.39) |
| Elimination half-life, hours | 16.5 (5.72) | 14.5 (8.83-30.6) |
| Distribution volume $_{systemic}$ L | 73.7 (33.20) | 71.5 (32.3-155) |
| CNS, central nervous system; CSF, cerebrospinal fluid; PK, pharmacokinetic; $0_{CSF}$; intercompartmental clearance in the CNS; rhASA, recombinant human arylsulfatase A; SD, standard deviation | | |

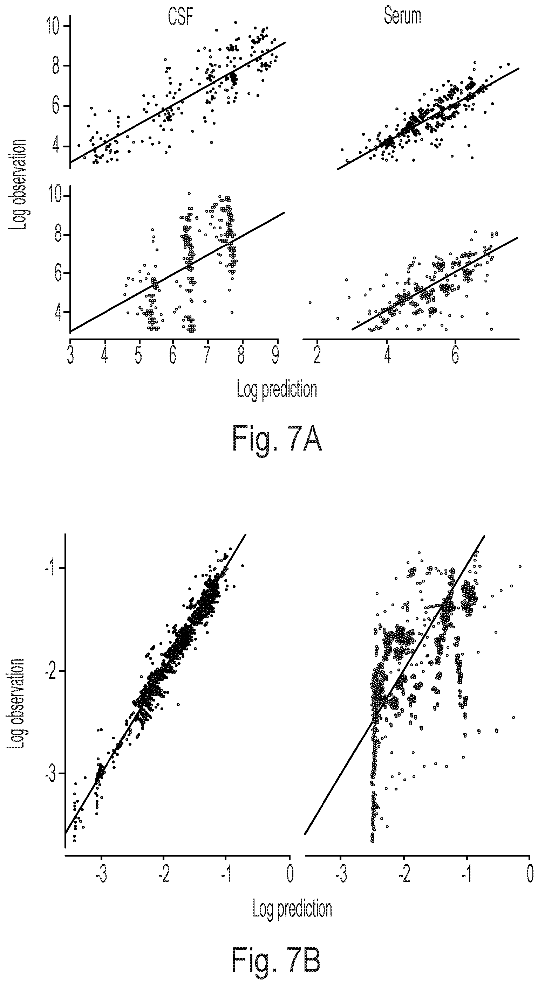

A concentration-dependent reduction in sulfatide in the CSF (model-estimated EC50: 184 ng/mL rhASA with a steep Hill slope of 3.59) and concentration-dependent inhibition in gross motor function loss (model-estimated IC50: 120 ng/mL rhASA, with a shallow Hill slope of 0.554, in brain tissue) were observed. The goodness-of-fit plots of population- and individual-predicted rhASA concentrations in CSF, sulfatide concentration in CSF and GMFM-88 total scores are shown in Figure 7A, B and C, respectively. All three models show reasonable characterization of observed rhASA concentrations; precision of the models varied, which may have been caused by the small number of patients included in the study. The median simulated pre-dose trough rhASA concentrations in the CSF and CNS for the four simulated dosing scenarios are shown in Figure 8.

At 100 mg every other week (scenario 1), it takes approximately 16 weeks to achieve PK steady-state, consistent with the median rhASA elimination half-life of 477 hours. At 100 mg or 150 mg, with weekly dosing for 12 weeks, followed by dosing every other week (scenarios 2 and 3), higher trough concentrations of rhASA were achieved in the CSF and CNS compared with scenario 1. The age-based dosing scenario (scenario 4) showed more consistent steady-state trough concentrations of rhASA over time for all three age groups. However, patients less than 8 months old did not achieve the same high trough concentrations of rhASA during the weekly administration phase as in scenarios 2 and 3.

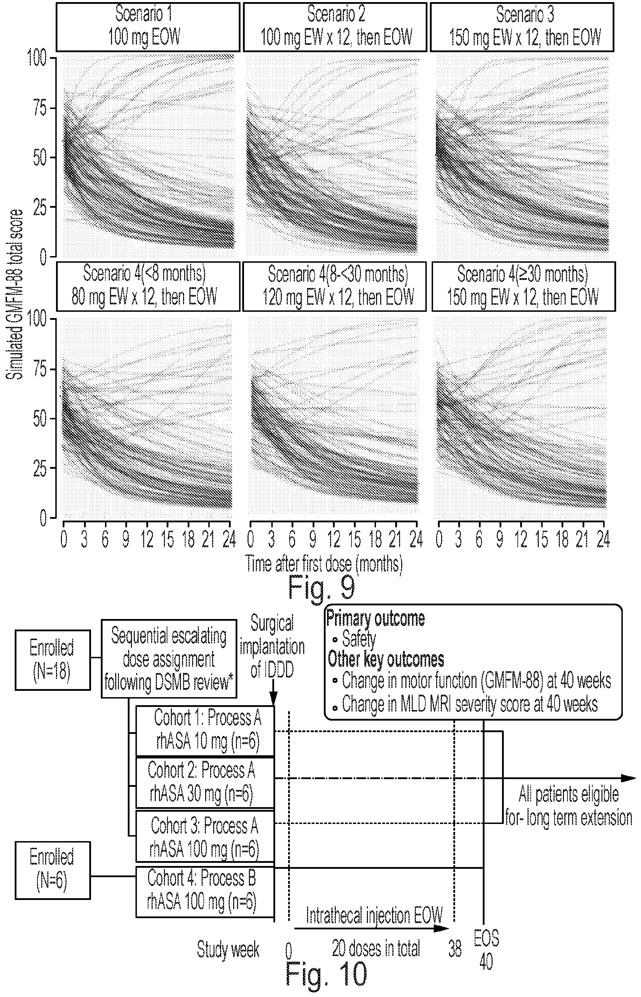

The simulations of the GMFM-88 total score show anticipated stabilization over time in some patients (Figure 9), which is consistent with the observed clinical study results. Scenarios 1-3 predicted that some patients in all groups would show an early response to treatment. Scenario 3 predicted the greatest number of patients with GMFM-88 total scores above 35 or 50 at 12 months and 24 months, respectively. The age-based dosing regimen (scenario 4) predicted that fewer patients in the < 8 months and 8-< 30 months groups would respond to treatment than in the > 30 months group.

Delivery of rhASA via an IDDD may lower the levels of sulfatides in CSF and slow the rate of motor function loss in a dose- and exposure-dependent manner in patients with MLD. Pharmacometric modeling approaches were used to predict the population PK, PK/PD and E-R relationships to GMFM-88 total score. In all cases, the models provided good characterization of the measurements observed in the patients receiving rhASA. Given the small number of patients, the variability observed in rhASA concentrations in CSF and that CSF concentrations were only observed at pre-dose time points, the precision between models varied. However, the model suggested rapid distribution of rhASA into brain tissue and systemic circulation, but a slow terminal elimination half-life from the CNS.

**Example 6: Analysis of responders and non-responders of intrathecal delivery of recombinant human arylsulfatase A in children with late-infantile metachromatic leukodystrophy.**

As shown in Figure 10, patients received rhASA via an intrathecal drug delivery device (IDDD) every other week during weeks 0 to 38. Cohort 1 (n = 6) received 10 mg rhASA, Cohort 2 (n = 6) received 30 mg rhASA, Cohort 3 (n = 6) received 100 mg rhASA and Cohort 4 (n = 6) received 100 mg rhASA that had been produced using a revised manufacturing process.

Eligibility criteria for cohorts 1-4 included a confirmed diagnosis of MLD (based on ASA deficiency, assessed using an assay in leukocytes, and elevated sulfatide concentration in urine); first appearance of MLD symptoms at or before 30 months of age; neurological signs of MLD present at the screening examination; ambulatory (able to walk forward 10 steps with one hand held) at screening. Patients included in cohorts 1-3 were also less than 12 years of age at screening. Additional eligibility criteria for Cohort 4 patients included Gross Motor Function Measure-88 (GMFM-88) total score of at least 40 at screening and at least 35 at baseline, and less than 8 years of age at screening.

Each patient was surgically implanted with an IDDD (PORT-A-CATH® II [Smiths Medical ASD, Inc., St Paul, MN, USA] or SOPH-A-PORT® Mini S [Sophysa, Orsay, France]) before receiving their first dose of rhASA. The location of the IDDD was confirmed postoperatively. Treatment response was assessed *post hoc* in patients from Cohorts 3 and 4 using GMFM-88 total score (0-100%), (Bjornson KF et al. Pediatr Phys Ther 1998;10:43-7) which provides an indication of motor function.

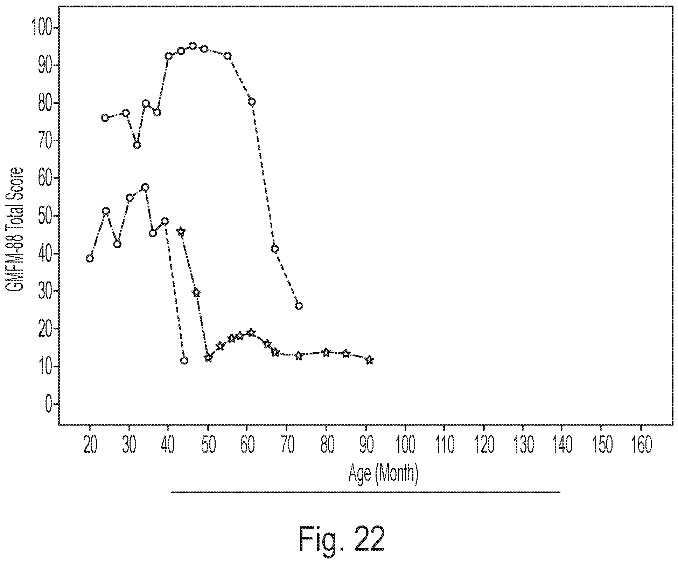

Responders were defined as individuals with a baseline GMFM-88 total score of $\geq 35$, and a maintained (decrease in GMFM-88 total score of $\leq 10$) or increased GMFM-88 total score at the end of the study relative to baseline. Non-responders were defined as individuals who did not meet this criterion. Other key outcomes assessed included change in concentration of sulfatides in the cerebrospinal fluid (CSF), change in MLD severity, measured by magnetic resonance imaging (MRI) of the brain using a similar scoring method to Eichler et al. (Giugliani R et al. JIEMS 2017;5:314:A699) to obtain the MLD-MRI severity score, change in the levels of N-acetylaspartate (NAA) in the white matter of the brain, evaluated by magnetic resonance spectroscopy (MRS). Figure 22 shows individual GMFM-88 total score by age in months of sibling pair patients from a clinical study of rhASA. Biomarkers and motor response appeared to improve together. Additionally, CSF sulfatide and MRS NAA levels appeared to improve together. Motor response was associated with a dose dependent reduction of the accumulated sulfatides in the CSF.

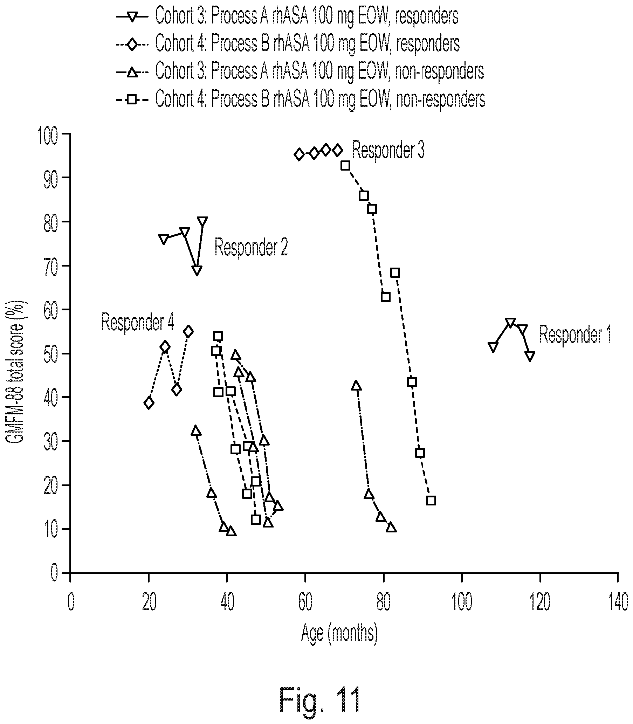

Of the 12 patients enrolled in NCT01510028 who received 100 mg rhASA, four individuals were identified as responders: two from Cohort 3 and two from Cohort 4. The remaining eight patients in Cohorts 3 and 4 were identified as non-responders. Figure 11 shows GMFM-88 total scores over time in the 12 patients. Demographics and baseline characteristics for the four responders are shown in Table 11.

**Table 11. Demographics and baseline characteristics for individuals identified as responders**

| Responder | Cohort | Age at enrollment, months | Age at diagnosis, months | Age at symptom onset, months | Baseline urine sulfatide μg/mL | Baseline GMF-M-88 total score (%) | Baseline CSF sulfatide level μg/mL | Baseline MLD-MRI severity score | Baseline MRS NAA in frontal parietal white matter |
|---|---|---|---|---|---|---|---|---|---|
| 1 | 3 | 107 | 104 | 24 | 4687 | 51 | 0.831 | 23 | 0.74 |
| 2 | 3 | 23 | 22 | 24 | 5368 | 76 | 0.095 | 4 | 1.13 |
| 3 | 4 | 56 | 55 | 30 | 3043 | 96 | 0.186 | 12 | 0.91 |
| 4 | 4 | 19 | 0 | 15 | 4745 | 39 | 0.211 | 1 | 1.14 |
| CSF, cerebrospinal fluid; GMFM-88, Gross Motor Function Measure-88; MLD, metachromatic leukodystrophy, MRS, magnetic resonance spectroscopy, NAA, N- acetylaspartate | | | | | | | | | |

Responders 1, 2 and 3 had a CSF sulfatide concentration below the upper limit of normal (0.113 μg/mL) after 1 month; no follow-up information on CSF sulfatide concentration was available for responder 4. At the end of the study (week 40), CSF sulfatide concentration was reported to be 0.0294 μg/mL, 0.0489 μg/mL and 0.0653 μg/mL in responders 1, 2 and 3, respectively.

Of the eight non-responders, one patient had a CSF sulfatide concentration below the upper limit of normal after 1 month. CSF sulfatide concentrations fell to below the upper limit of normal after 8-36 weeks in the remaining patients. One individual from cohort 1 also met the *post hoc* responder criteria without CSF sulfatide levels normalizing by study end.

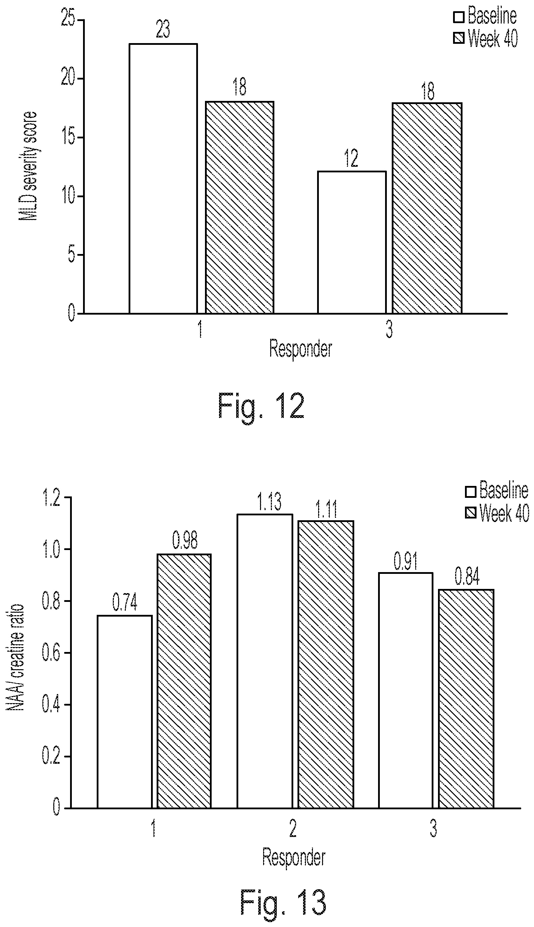

MLD-MRI severity score from baseline to week 40 decreased in two of the responders and increased in one of the responders (Figure 12). Information on MLD-MRI severity score at end of study was not available for responder 4. Two of the four responders had an NAA/creatine ratio in frontal parietal white matter of $\geq 1.0$ at baseline. None of the eight non-responders for whom baseline data were available had an NAA/creatine ratio at baseline of $\geq 1.0$.

The NAA/creatine ratio in frontal parietal white matter increased at week 40 compared with baseline in one of the responders and remained stable in the other two responders who had data available at baseline and at week 40 (Figure 13).

Four of the twelve patients who received 100 mg rhASA were identified as having responded to treatment; age and baseline characteristics varied between the responders. These findings indicate a trend towards disease stabilization in a subset of patients who received the highest dose and support the development of intrathecal therapy with rhASA for patients with LI-MLD. Factors that influence treatment response may include CSF rhASA concentration, baseline NAA/creatine ratio, stabilization of NAA/creatine ratio, disease duration, age and the time taken for CSF sulfatide levels to normalize.

**Example 7: Intrathecally administered recombinant human arylsulfatase A in patients with late-infantile metachromatic leukodystrophy**

Deficiency of the lysosomal enzyme arylsulfatase A in MLD leads to the accumulation of sulfated glycosphingolipids, known collectively as sulfatides. These accumulate in, and are toxic to, the cells which maintain the myelin insulation sheath of axons both centrally and peripherally. IT administration of recombinant human arylsulfatase A may be sufficient to hydrolyze accumulated sulfatides in cells of the nervous system and could slow or prevent further accumulation, which should translate into motor system benefits. Direct CNS administration through, e.g., IT delivery can be used to effectively treat MLD patients. This example illustrates a multicenter open-label, matched historical control clinical study designed to assess the efficacy of once-weekly intrathecal dosing with 150 mg of rhASA in patients with late-infantile MLD. This study will investigate the potential of 150 mg IT administered rhASA to stabilize or slow progression of motor dysfunction in pediatric subjects with late infantile MLD.

Approximately 35 patients will be enrolled to assess the safety and tolerability of rhASA and its delivery via the SOPH-A-PORT Mini S intrathecal drug delivery device. The rate and severity of disease progression is well documented in late infantile MLD. A distinguishing feature of the definition of late infantile MLD is the early age at disease symptom onset with a majority of patients with late infantile MLD showing first motor dysfunction before the age of 18 months. Four treatment groups will be included based on age and degree of motor dysfunction at enrollment:

•    Group A - early symptomatic (18-48 months; Gross Motor Function Classification in MLD [GMFC-MLD] level 1-2;

n=16),

- Group B - intermediate symptomatic (18-72 months; GMFC-MLD level 3; n≤8),

- Group C - advanced symptomatic (18-72 months; GMFC MLD level 4; n≤8) and

- Group D - presymptomatic or minimally symptomatic (<18 months; pre-symptomatic siblings of participants, with the same ASA allelic constitution; n=3).

Symptom onset must be before 30 months in Groups A-C. All subjects will receive a once weekly dose of IT rhASA at 150 mg for 105 weeks. The study will evaluate safety and efficacy of the treatment regimen on gross motor function using the GMFC-MLD and GMFM-88 total score to measure disease progression. Group D subjects will be assessed with the Alberta Infant Motor Scale (Piper and Darrah, 1994). The primary endpoint will be the proportion of children in Group A with an increase in their GMFC-MLD level (indicating motor function decline) of no more than 2 from baseline to 2 years. A secondary endpoint will be the proportion of children in Group A with a Gross Motor Function Measure-88 total score of >40 at 2 years.

Other secondary endpoints will include evaluating the effects of IT administration of rhASA on the time course of declining gross motor function using GMFC-MLD; evaluating the effects of IT administration of rhASA on the time course of declining gross motor function using GMFM-88; evaluating the effects of IT administration of rhASA on expressive language using the Expressive Language Function Classification (ELFC-MLD); evaluating the effects of IT administration of rhASA on cerebrospinal fluid (CSF) biomarkers (i.e., sulfatide levels); and evaluating the effects of IT administration of rhASA on proton magnetic resonance spectroscopy (MRS) of the brain, specifically N-acetylaspartate/Creatine (NAA/Cr) in white matter. Outcomes will be compared with propensity-score-matched, historical control data in children with MLD.

Safety and tolerability of IT rhASA will be based on the occurrence of treatment-emergent adverse events (TEAEs), clinical laboratory testing (serum chemistry, hematology, and urinalysis) and vital signs, physical examination including documentation of signs and symptoms of MLD, 12-lead electrocardiogram (ECG), CSF laboratory parameters (chemistries and cell counts), the presence and activity of anti-rhASA antibodies in CSF and serum and the SOPH-A-PORT® Mini S device in subjects with MLD.

Additional evaluations of the effects of administration of IT rhASA may be based on serum and urine biomarkers (i.e., sulfatide levels), severity score as measured by magnetic resonance imaging (MRI) of the brain, volumetric analysis based on MRI of the brain, ability to swallow as assessed by the Fiberoptic Endoscopic Evaluation of Swallowing (FEES), nerve conduction by electroneurography (ENG), and communication and cognition using the Battelle Developmental Inventory (BDI-2NU). Caregiver burden and subject's health-related quality of life impact in children with MLD will be explored by evaluating caregiver burden as assessed by the Caregiver Impact Questionnaire (CIQ) and Health Related Quality of Life (HRQOL) as assessed by the Infant Toddler Quality of Life Questionnaire- 97 items (ITQOL-97).

The primary efficacy endpoint is response in Group A defined as maintenance of gross motor function at 2 years (Week 106), evaluated as a change from baseline of no greater than 2 levels of GMFC-MLD. The key secondary efficacy endpoint in Group A is the maintenance of gross motor function at 2 years (Week 106), evaluated as a GMFM-88 total score >40. The efficacy of rhASA will be evaluated by comparison of enrolled subjects in Group A with matched historical control subjects. The data from these untreated MLD subjects (i.e., subjects who have received no investigational product or therapy) will come from the ongoing Global Leukodystrophy Initiative (GLIA-MLD) natural history study. Additional matched historical control data from MLD subjects with exposure to rhASA in prior investigational studies may also be used. Matched historical controls from GLIA-MLD and prior Shire studies must have data for at least baseline and 1 post-baseline gross motor function evaluation. These controls will have had very similar inclusion/exclusion criteria as enrolled subjects of the present study, and comparable efficacy assessment at similar time points.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of examples only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.
The invention further provides the following numbered embodiments:

1. A method of treating metachromatic leukodystrophy (MLD), the method comprising administering intrathecally to a subject in need thereof a therapeutically effective dose of a formulation comprising a purified recombinant human arylsulfatase A (rhASA) protein,

wherein the purified rhASA protein is characterized by a proteoglycan map comprising one or more peaks corresponding to neutral recombinant ASA protein (neutral ASA protein, sialylated recombinant ASA protein (sialic acid ASA protein), mannose-6-phosphated recombinant ASA protein (M6P ASA protein), N-acetyl-glucosamine mannose-6-phosphated recombinant ASA protein (capped M6P ASA protein), and hybrid recombinant ASA protein (hybrid ASA protein), and
wherein administering the formulation results in stabilizing or decreasing the progression of at least one symptom of MLD.

2. The method of embodiment 1, wherein the therapeutically effective dose is 100 mg.

3. The method of embodiment 1, wherein the therapeutically effective dose is 150 mg.

4. The method of embodiment 1, wherein the formulation is administered once every week.

5. The method of embodiment 1, wherein the subject has late infantile MLD.

6. The method of any one of the preceding embodiments, wherein stabilizing or decreasing the progression of MLD is measured using a change in Gross Motor Function Classification (GMFC-MLD).

7. The method of any one of the preceding embodiments, wherein administering the formulation results in a change in GMFC-MLD level by $\leq 4$, $\leq 3$ or $\leq 2$ levels from baseline at 2 years of treatment in the subject.

8. The method of any one of the preceding embodiments, wherein administering the formulation results in maintenance of GMFC-MLD score at two years of treatment.

9. The method of embodiment 7 or 8, wherein the maintenance of GMFC-MLD score is a change in GMFC-MLD of no greater than 2 levels from baseline at two years of treatment.

10. The method of any one of the embodiments 7-9, wherein the baseline is an assessment score of GMFC-MLD prior to the first administration of the formulation.

11. The method of any one of the preceding embodiments wherein stabilizing or decreasing the progression of MLD is measured using a change in Gross Motor Function Measure (GMFM-MLD).

12. The method of embodiment 11, wherein administering the formulation results in maintenance of GMFM-MLD score at two years of treatment.

13. The method of embodiment 11 or 12, wherein the GMFM-MLD score is maintained at > 40.

14. The method of any one of the preceding embodiments, wherein stabilizing or decreasing the progression of MLD is measured by the sulfatide levels in the cerebrospinal fluid.

15. The method of any one of the preceding embodiments, wherein stabilizing or decreasing the progression of MLD is measured by the brain N-acetylaspartate/creatine ratio (NAA/cr).

16. The method of any one of the preceding embodiments, wherein stabilizing or decreasing the progression of MLD is measured using Expressive Language Function Classification-MLD level (ELFC-MLD).

17. The method of any one of the embodiments 5-16, wherein the subject has baseline GMFC-MLD level 1-2.

18. The method of any one of the embodiments 5-16, wherein the subject has baseline GMFC-MLD level 3.

19. The method of any one of the embodiments 5-16, wherein the subject has baseline GMFC-MLD level 4.

20. The method of any one of the embodiments 5-16, wherein the subject is pre-symptomatic.

21. The method of embodiment 20, wherein the subject is less than 18 months old.

22. The method of embodiment 21, wherein the subject is assessed with Alberta Infant Motor Scale.

23. The method of any one of the preceding embodiments, wherein the rhASA protein has an amino acid sequence at least 70% identical to SEQ ID NO:1.

24. A method of treating late infantile MLD, the method comprising:

administering to the subject a formulation comprising a purified recombinant human arylsulfatase A (rhASA) protein having an amino acid sequence at least 70% identical to SEQ ID NO:1, wherein
the purified rhASA protein is characterized by a proteoglycan map comprising one or more peaks corresponding to neutral recombinant ASA protein (neutral ASA protein, sialylated recombinant ASA protein (sialic acid ASA protein), mannose-6-phosphated recombinant ASA protein (M6P ASA protein), N-acetyl-glucosamine mannose-6-phosphated recombinant ASA protein (capped M6P ASA protein), and hybrid recombinant ASA protein (hybrid ASA protein), and wherein
the formulation is administered intrathecally at a dose of 150 mg at an interval of once every week.

25. The method of any one of the preceding embodiments, wherein the formulation contains less than about 150 ng/mg Host Cell Protein (HCP).

26. The method of any one of the preceding embodiments, the purified recombinant ASA protein comprises at least about 23% of the total purified recombinant ASA protein corresponds to mannose-6-phosphated recombinant ASA protein (M6P ASA protein).

27. The method of any one of the preceding embodiments, wherein the proteoglycan map of the purified recombinant ASA protein comprises:

about 15% to about 25% neutral recombinant ASA protein (neutral ASA protein),
about 35% to about 45% sialylated recombinant ASA protein (sialic acid ASA protein),
about 23% to about 33% mannose-6-phosphated recombinant ASA protein (M6P ASA protein),
about 1% to about 10% N-acetyl-glucosamine mannose-6-phosphated recombinant ASA protein (capped M6P ASA protein), and
about 5% to about 15% hybrid recombinant ASA protein (hybrid ASA protein).

28. The method of embodiment 27, wherein the proteoglycan map of the purified recombinant ASA protein comprises:

about 18% to about 22% neutral ASA protein,
about 37% to about 41% sialic acid ASA protein,
about 26% to about 29% M6P ASA protein,
about 4% to about 6% capped M6P ASA protein, and
about 7% to about 9% hybrid ASA protein.

29. A composition comprising purified recombinant human arylsulfatase A (rhASA) protein having an amino acid sequence at least 70% identical to SEQ ID NO:1, wherein the purified rhASA protein is characterized by one or more proteoglycan species selected from neutral recombinant ASA protein (neutral ASA protein, sialylated recombinant ASA protein (sialic acid ASA protein), mannose-6-phosphated recombinant ASA protein (M6P ASA protein), N-acetyl-glucosamine mannose-6-phosphated recombinant ASA protein (capped M6P ASA protein), or hybrid recombinant ASA protein (hybrid ASA protein).

30. The composition of embodiment 29, wherein at least about 23% of the total purified recombinant ASA protein corresponds to mannose-6-phosphated recombinant ASA protein (M6P ASA protein).

31. A composition comprising purified recombinant arylsulfatase A (rhASA) protein having an amino acid sequence at least 70% identical to SEQ ID NO:1, wherein

at least about 23% of the total purified recombinant ASA protein corresponds to mannose-6-phosphated recombinant ASA protein (M6P ASA protein) characterized by a proteoglycan map; and
the composition contains less than about 150 ng/mg Host Cell Protein (HCP).

32. The composition of embodiment 30 or 31, wherein the M6P ASA protein is present in an amount that is at least about 26% of the total purified rhASA protein content.

33. The composition of embodiment 30 or 31, wherein the M6P ASA protein is present in an amount that is at least about 28% of the total purified rhASA protein content.

34. The composition of any one of the preceding embodiments, wherein the M6P ASA protein is present in an amount that is about 20% to about 33% of the total purified rhASA protein content.

35. The composition of any one of embodiments 29-34, wherein the total purified rhASA protein comprises neutral recombinant ASA protein (neutral ASA protein), sialylated recombinant ASA protein (sialic acid ASA protein), N-acetyl-glucosamine mannose-6-phosphated recombinant ASA protein (capped M6P ASA protein), or hybrid recombinant ASA protein (hybrid ASA protein), or any combination thereof.

36. The composition of embodiment 30 or 31, wherein the total purified rhASA protein comprises:

about 23% to about 33% mannose-6-phosphated recombinant ASA protein (M6P ASA protein),
about 15% to about 25% neutral recombinant ASA protein (neutral ASA protein),
about 35% to about 45% sialylated recombinant ASA protein (sialic acid ASA protein),
about 1% to about 10% N-acetyl-glucosamine mannose-6-phosphated recombinant ASA protein (capped M6P ASA protein), and
about 5% to about 15% hybrid recombinant ASA protein (hybrid ASA protein).

37. The composition of any one of embodiments 29-36, wherein neutral ASA protein is present in an amount that is about 16% to about 22% of the total purified rhASA protein.

38. The composition of any one of embodiments 29-37, wherein neutral ASA protein is present in an amount that is about 20% to about 25% of the total purified rhASA protein.

39. The composition of any one of embodiments 29-38, wherein sialic acid ASA protein is present in an amount that is about 35% to about 40% of the total purified rhASA protein.

40. The composition of any one of embodiments 29-39, wherein sialic acid ASA protein is present in an amount that is about 37% to about 42% of the total purified rhASA protein.

41. The composition of any one of embodiments 29-40, wherein capped M6P ASA protein is present in an amount that is about 3% to about 5% of the total purified rhASA protein.

42. The composition of any one of embodiments 29-41, wherein capped M6P ASA protein is present in an amount that is about 4% to about 6% of the total purified rhASA protein.

43. The composition of any one of embodiments 29-42, wherein capped M6P ASA protein is present in an amount that is about 4.5% to about 5.5% of the total purified rhASA protein.

44. The composition of any one of embodiments 29-43, wherein hybrid ASA protein is present in an amount that is about 7% to about 10% of the total purified rhASA protein.

45. The composition of any one of embodiments 29-44, wherein hybrid ASA protein is present in an amount that is about 7.5% to about 8.5% of the total purified rhASA protein.

46. The composition of embodiment 30 or 31, wherein the total purified rhASA protein comprises:

about 16% to about 23% neutral ASA protein,
about 37% to about 42% sialic acid ASA protein,
about 23% to about 27% M6P ASA protein,
about 4% to about 8% capped M6P ASA protein, and
about 7% to about 10% hybrid ASA protein.

47. The composition of embodiment 30 or 31, wherein the total purified rhASA protein comprises:

about 20% to about 23% neutral ASA protein,
about 35% to about 39% sialic acid ASA protein,
about 26% to about 32% M6P ASA protein,
about 3% to about 5% capped M6P ASA protein, and
about 7% to about 9% hybrid ASA protein.

48. The composition of embodiment 30 or 31, wherein the total purified rhASA protein comprises:

about 18% to about 22% neutral ASA protein,
about 37% to about 41% sialic acid ASA protein,
about 26% to about 29% M6P ASA protein,
about 4% to about 6% capped M6P ASA protein, and
about 7% to about 9% hybrid ASA protein.

49. The composition of any one of embodiments 29-48, wherein the total purified rhASA protein is present in a concentration of about 20 mg/mL to about 45 mg/mL.

50. The composition of embodiment 49, wherein the total purified rhASA protein is present in a concentration of about 25 mg/mL to about 34 mg/mL or about 28 mg/mL to about 32 mg/mL.

51. The composition of embodiment 49, wherein the total purified rhASA protein is present in a concentration of about 25 mg/mL, about 26 mg/mL, about 27 mg/mL, about 28 mg/mL, about 29 mg/mL, about 30 mg/mL, about 31 mg/mL, about 32 mg/mL, about 33 mg/mL, or about 34 mg/mL.

52. The composition of any one of embodiments 29-51, wherein the purified rhASA protein has a specific activity of about 50 to about 130 U/mL.

53. The composition of embodiment 52, wherein the purified rhASA protein has a specific activity of about 70 to about 100 U/mg.

54. The composition of embodiment 52, wherein the purified rhASA protein has a specific activity of about 80 to about 90 U/mg.

55. The composition of embodiment 52, wherein the purified rhASA protein has a specific activity of about 75 to about 95 U/mg.

56. The composition of any one of embodiments 29-55, wherein the purified rhASA protein contains less than about 140 ng/mg Host Cell Protein (HCP).

57. The composition of embodiment 56, wherein the purified rhASA protein contains less than about 100 ng/mg HCP.

58. The composition of embodiment 56, wherein the purified rhASA protein contains less than about 80 ng/mg HCP.

59. The composition of embodiment 56, wherein the purified rhASA protein contains less than about 60 ng/mg HCP.

60. The composition of any one of embodiments 29-59, wherein the purified rhASA protein contains less than about 100 pg/mg Host Cell DNA (HCD).

61. The composition of embodiment 60, wherein the purified rhASA protein contains less than about 50 pg/mg HCD.

62. The composition of embodiment 60, wherein the purified rhASA protein contains less than about 10 pg/mg HCD.

63. The composition of embodiment 60, wherein the purified rhASA protein contains less than about 5 pg/mg HCD, less than about 4 pg/mg HCD, less than about 3 pg/mg HCD, less than about 2 pg/mg HCD, or less than about 1 pg/mg HCD.

64. The composition of any one of embodiments 29-63, wherein the purified rhASA protein has an amino acid sequence at least 80% identical to SEQ ID NO:1.

65. The composition of embodiment 64, wherein the purified rhASA protein has an amino acid sequence at least 90% identical to SEQ ID NO:1.

66. The composition of embodiment 64, wherein the purified rhASA protein has an amino acid sequence at least 95% identical to SEQ ID NO:1.

67. The composition of embodiment 64, wherein the purified rhASA protein has an amino acid sequence that is identical to SEQ ID NO:1.

68. The composition of any one of embodiments 29-67, comprising about 0.001% to about 0.01% polysorbate-20 (P20).

69. A formulation comprising the composition of any one of embodiments 29-68 and a physiologically acceptable carrier.

70. The formulation of embodiment 69, wherein the formulation is suitable for intravenous administration.

71. The formulation of embodiment 69, wherein the formulation is suitable for intrathecal administration.

72. The formulation of embodiment 69, wherein the formulation is suitable for subcutaneous administration.

73. A method of purifying recombinant arylsulfatase A (ASA) protein, the method comprising:

purifying recombinant arylsulfatase A (ASA) protein from an impure preparation by conducting one or more chromatography steps;
pooling eluate from the one or more chromatography steps;
optionally adjusting the pH of the pooled eluate to pH that is about 6.0 to about 8.0;
optionally subjecting the pooled eluate or the pH-adjusted eluate to ultrafiltration and/or diafiltration;
obtaining an eluate comprising purified recombinant arylsulfatase A (ASA) protein; and
adding of a surfactant to the eluate comprising purified recombinant arylsulfatase A (ASA) protein.

74. The method of embodiment 73, comprising the step of adjusting the pH of the pooled eluate to pH that is about 6.0 to about 8.0.

75. The method of embodiment 74, comprising the step of subjecting the pH-adjusted eluate to ultrafiltration and/or diafiltration.

76. The method of any one of embodiments 73-75, where said adding of a surfactant occurs prior to cold storage of the eluate comprising purified recombinant ASA protein.

77. The method of any one of embodiments 73-75, wherein said surfactant is present in a concentration that is about 0.0001 % (v/v) to about 0.01 % (v/v).

78. The method of embodiment 77, wherein said surfactant is present in a concentration that is about 0.001% (v/v) to about 0.01% (v/v).

79. The method of any one of embodiments 73-78, wherein said surfactant is polysorbate-20 (P20).

80. The method of any one of embodiments 73-79, wherein the step of conducting one or more chromatography steps comprises conducting anion-exchange chromatography, mixed-mode chromatography, hydrophobic interaction chromatography that is phenyl chromatography, and cation-exchange chromatography, in that order.

81. The method of embodiment 80, wherein the anion-exchange chromatography uses a column with TMAE resin.

**Claims**

1. A formulation, comprising a purified recombinant human arylsulfatase A (rhASA) protein for use in a method of treating metachromatic leukodystrophy (MLD), wherein the purified rhASA protein has an amino acid sequence at least 70% identical to SEQ ID NO: 1, and is **characterized by** a proteoglycan map comprising:

   i. 15% to 25% neutral recombinant ASA protein (neutral ASA protein),
   ii. 35% to 45% sialylated recombinant ASA protein (sialic acid ASA protein),
   iii. 23% to 33% mannose-6-phosphated recombinant ASA protein (M6P ASA protein),
   iv. 1% to 10% N-acetyl-glucosamine mannose-6-phosphated recombinant ASA protein (capped M6P ASA protein), and
   v. 5% to 15% hybrid recombinant ASA protein (hybrid ASA protein),

   wherein the recombinant ASA is formulated in a formulation comprising 0.001-0.01% polysorbate 20.

2. A formulation, comprising a purified recombinant human arylsulfatase A (rhASA) protein for use in a method of treating metachromatic leukodystrophy (MLD), wherein the purified rhASA protein has an amino acid sequence at least 70% identical to SEQ ID NO: 1, and is **characterized by** a proteoglycan map comprising:

   i. 15% to 25% neutral recombinant ASA protein (neutral ASA protein),
   ii. 35% to 45% sialylated recombinant ASA protein (sialic acid ASA protein),
   iii. 23% to 33% mannose-6-phosphated recombinant ASA protein (M6P ASA protein),
   iv. 1% to 10% N-acetyl-glucosamine mannose-6-phosphated recombinant ASA protein (capped M6P ASA protein), and
   v. 5% to 15% hybrid recombinant ASA protein (hybrid ASA protein),
   wherein the recombinant ASA contains less than about 150 ng/mg Host Cell Protein (HCP), optionally less than about 140 ng/mg HCP.

3. The formulation for use according to claim 1 or 2, wherein at least 23% of the total purified recombinant ASA protein corresponds to mannose-6-phosphated recombinant ASA protein (M6P ASA protein).

4. The formulation for use according to claim 3, wherein 26% to 28% of the total purified recombinant ASA protein corresponds to mannose-6-phosphated recombinant ASA protein (M6P ASA protein).

5. The formulation for use according to claim 3 or 4, wherein 35% to 45% of the total purified recombinant ASA protein corresponds to sialylated recombinant ASA protein (sialic acid ASA protein).

6. The formulation for use according to claim 1-5, wherein the subject has late infantile MLD.

7. The formulation for use according to claim 6, wherein:

   a. the subject has baseline GMFC-MLD level 1-2; or
   b. the subject has baseline GMFC-MLD level 3; or
   c. the subject has baseline GMFC-MLD level 4; or
   d. the subject is pre-symptomatic, optionally wherein the subject is less than 18 months old, further optionally wherein the subject is assessed with Alberta Infant Motor Scale.

8. The formulation for use according to any one of the preceding claims, wherein the proteoglycan map of the purified recombinant ASA protein comprises:

   i. about 18% to about 22% neutral ASA protein,
   ii. about 37% to about 41% sialic acid ASA protein,

iii. about 26% to about 29% M6P ASA protein,
iv. about 4% to about 6% capped M6P ASA protein, and
v. about 7% to about 9% hybrid ASA protein.

9. The formulation for use according to any one of the preceding claims, wherein the total purified rhASA protein is present in a concentration of 20 mg/mL to 45 mg/mL.

10. The formulation for use according to claim 9, wherein the total purified rhASA protein is present in a concentration of 25 mg/mL to 34 mg/mL or 28 mg/mL to 32 mg/mL.

11. The formulation for use according to any one of the preceding claims, wherein the purified rhASA protein has a specific activity of about 50 to about 130 U/mL.

12. The formulation for use according to claim 11, wherein the purified rhASA protein has a specific activity of about 70 to about 100 U/mg.

13. The formulation for use according to any one of the preceding claims, wherein the purified rhASA protein contains less than about 100 pg/mg Host Cell DNA (HCD).

14. The formulation for use according to claim 1, wherein 0.005% polysorbate 20 is added prior to cold storage.

15. The formulation for use according to any one of the preceding claims, wherein the formulation is suitable for

i. intravenous administration;
ii. intrathecal administration; or
iii. subcutaneous administration.

Fig. 1A

Fig. 1B

Fig. 1C

d

Cerebral peduncle

p=NS
p=NS
p<0.0001
p<0.0001
p<0.0001
p=0.0005

Relative positivity (%)

200

150

100

50

WT  Control  Process  Process  Process  Process
A        A        B        B
0.04 mg  0.21 mg  0.04 mg  0.21 mg

## Fig. 1D

e

Cerebral cortex

p=0.0087
p=NS
p<0.0001
p<0.0001
p=NS
p=NS

Relative positivity (%)

200

150

100

50

WT  Control  Process  Process  Process  Process
A        A        B        B
0.04 mg  0.21 mg  0.04 mg  0.21 mg

## Fig. 1E

f

Striatum

p=NS
p<0.0034
p=NS
p<0.0005
p=NS
p=NS

Relative positivity (%)

200

150

100

50

WT  Control  Process  Process  Process  Process
A        A        B        B
0.04 mg  0.21 mg  0.04 mg  0.21 mg

## Fig. 1F

Fig. 2A

Fig. 2B

Fig. 4

Fig. 5

Fig. 6

Fig. 7A

Fig. 7B

Fig. 7C

Fig. 8

Fig. 9

Fig. 10

—▽— Cohort 3: Process A rhASA 100 mg EOW, responders
--◇-- Cohort 4: Process B rhASA 100 mg EOW, responders
—△-- Cohort 3: Process A rhASA 100 mg EOW, non-responders
--□-- Cohort 4: Process B rhASA 100 mg EOW, non-responders

Fig. 11

Fig. 12

Fig. 13

Fig. 14

Fig. 15

Fig. 16A

Fig. 16B

Fig. 17A

Fig. 17B

Fig. 18A

Fig. 18B

Fig. 19A

Fig. 19B

Fig. 20A

Fig. 20B

Fig. 21

Fig. 22

## REFERENCES CITED IN THE DESCRIPTION

### Patent documents cited in the description

- US 62607831
- US 62608569 B
- US 62625814 B
- US 62760597 B
- WO 2011163648 A
- WO 2011163650 A
- US 6217552 B
- US 4314997 A, Shanbrom
- US 4315919 A
- US 4764369 A

### Non-patent literature cited in the description

- **PIPER M** ; **DARRAH J**. *Motor Assessment of a Developing Infant*, 09 February 1994, 222
- **LAZORTHES et al.** *Advances in Drug Delivery Systems and Applications in Neurosurgery*, 143-192
- *Lancet*, 1963, vol. 2, 983-84
- **DEKABAN AS**. Changes in brain weights during the span of human life: relation of brain weights to body heights and body weights. *Ann Neurol*, 1978, vol. 4, 345-56
- **JOHANSON CE et al.** Multiplicity of cerebrospinal fluid functions: New challenges in health and disease. *Cerebrospinal Fluid Res.*, 14 May 2008, vol. 5, 10
- **AUSTIN et al.** *Biochem J.*, 1964, vol. 93, 15C-17C
- **LAIDLER PM et al.** *Biochim Biophys Acta*, 1985, vol. 827, 73-83
- **DRAPER RK et al.** *Arch Biochemica Biophys.*, 1976, vol. 177, 525-538
- **WAHEED A et al.** *Hoppe Seylers Z Physiol Chem.*, 1982, vol. 363, 425-430
- **FUJII T et al.** *Biochim Biophys Acta*, 1992, vol. 15 (1122), 93-98
- **WAHEED A et al.** *Biochim Biophys Acta*, 1985, vol. 847, 53-61
- **BRAULKE T et al.** *Biochem Biophys Res Commun.*, 1987, vol. 143, 178-185
- **BRAULKE T et al.** *J Biol Chem.*, 1990, vol. 265, 6650-6655
- **KELLY BM et al.** *Eur J Cell Biol.*, 1989, vol. 48, 71-78
- **LUIJTEN JAFM et al.** *J Mol Med.*, 1978, vol. 3, 213
- **DUBOIS et al.** *Biomedicine.*, 1975, vol. 23, 116-119
- **MANOWITZ P et al.** *Biochem Med Metab Biol.*, 1988, vol. 39, 117-120
- **PORETZ et al.** *Biochem J.*, 1992, vol. 287, 979-983
- **STEVENS RL et al.** *Biochim Biophys Acta*, 1976, vol. 445, 661-671
- **FARRELL DF et al.** *Neurology.*, 1979, vol. 29, 16-20
- **SARAFIAN TA et al.** *Biochem Med.*, 1985, vol. 33, 372-380
- **LEE GD** ; **VAN ETTEN RL**. *Arch Biochem Biophys.*, 1975, vol. 171, 424-434
- **JAMES GT**. *Arch Biochem Biophys.*, 1979, vol. 97, 57-62
- **KREYSING et al.** *Eur J Biochem.*, 1990, vol. 191, 627-631
- **STEIN et al.** *J Biol Chem.*, 1989, vol. 264, 1252-1259
- **GIESELMANN et al.** *Hum Mutat.*, 1994, vol. 4, 233-242
- **BARTH et al.** *Hum Mutat.*, 1995, vol. 6, 170-176
- **DRAGHIA et al.** *Hum Mutat.*, 1997, vol. 9, 234-242
- **GIESELMANN et al.** *Dev Neurosci.*, 1991, vol. 13, 222-227
- **SOMMERLADE et al.** *Biochem. J.*, 1994, vol. 297, 123-130
- **SCHMIDT et al.** *Cell*, 1995, vol. 82, 271-278
- **LUKATELA et al.** *Biochemistry*, 1998, vol. 37, 3654-3664
- **BJORNSON KF et al.** *Pediatr Phys Ther*, 1998, vol. 10, 43-7
- **GIUGLIANI R et al.** *JIEMS*, 2017, vol. 5 (314), A699